# EUROPEAN PATENT APPLICATION

(11) **EP 1 650 208 A1**
(43) Date of publication of application: **26.04.2006**
(21) Application number: 04746340.1
(22) Date of filing: 24.06.2004
(51) Int. Cl.: C07D 471/04, H05B 33/14

(54) **WHITE-EMITTING COMPOUNDS, PROCESS FOR THE PRODUCTION THEREOF, AND WHITE-EMITTING DEVICES**

(30) Priority: 30.06.2003 JP 2003188972; 22.08.2003 JP 2003298589
(71) Applicant: HIROSE ENGINEERING CO., LTD., Tokyo 146-0092 (JP)
(72) Inventor: NAKAYA, Tadao, Hirose Engineering Co., Ltd., Ohta-ku, Tokyo 1460092 (JP); IKEDA, Atsushi, Hirose Engineering Co., Ltd., Ohta-ku, Tokyo 1460092 (JP); SATO, Mitukura, Hirose Engineering Co., Ltd., Ohta-ku, Tokyo 1460092 (JP); SAIKAWA, Tomoyuki, Hirose Engineering Co., Ltd., Ohta-ku, Tokyo 1460092 (JP)
(74) Representative: HOFFMANN EITLE
(86) International application number: PCT/JP2004/008871
(87) International publication number: WO 2005/000847

(57) **Abstract**

The objective of the present invention is to provide a novel white light-emitting compound, which is a single substance, capable of emitting white light by itself, a simple process of preparing the white light-emitting compound, and a white light-emitting element including the white light-emitting compound. The white light-emitting compound according to the present invention is **characterized by** its structure represented by formula (1) : 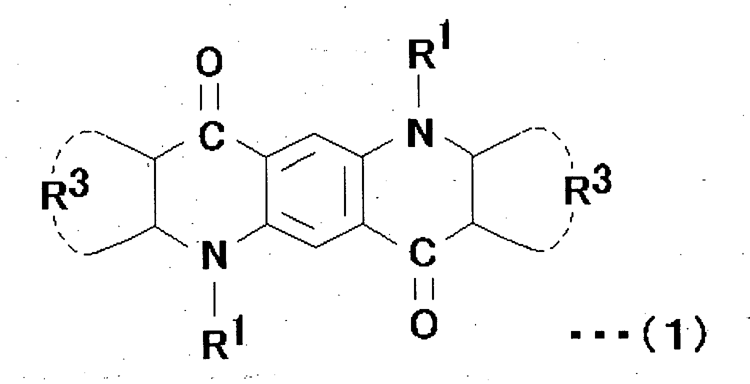 wherein R¹ denotes a hydrogen atom, an alkyl group with 1 to 10 carbon atoms, or a specified aryl group, wherein there are no cases where both R¹ s are hydrogen atoms.

## Description

### TECHNICAL FIELD

The present invention relates to a white light-emitting compound, a process of preparing the compound, and a white light-emitting element including the white light-emitting compound. More particularly, this invention relates to a white light-emitting compound which is a novel compound capable of emitting white light by itself, a process of producing it, and a white light-emitting element utilizing it.

### BACKGROUND ART

Researchers have developed organic EL elements, centering on the development of elements emitting light, the color of which is one of the three primary colors, i.e. red (R), green (G) and blue (B), and that of white light-emitting elements. The emission of white light was realized typically through mixing three compounds that respectively emit red light, blue light and green light, or mixing several light-emitting compounds each having lights of different colors. This technology is disclosed in JP63-19796, A.

However, few compounds that emit white light by themselves are known.

### DISCLOSURE OF THE INVENTION

### [Problems to be Solved by the Invention]

One objective of the present invention is to provide a compound which is capable of emitting white light by itself and applicable, for example, to organic EL elements, a process of producing the compound, and a white light-emitting element utilizing the compound. Another objective of the present invention is to provide an organic compound which is capable of emitting white light and applicable to various kinds of white light-emitting elements including organic EL elements. As a result of intensive studies to achieve the objectives, the inventors succeeded in synthesizing a single fluorescent compound capable of emitting white light at high purity and at high luminance, which led to the invention of a long-life EL element.

### [Means to Solve the Problems]

The first means provided by the present invention to achieve the objectives is a white light-emitting compound represented by formula (1). 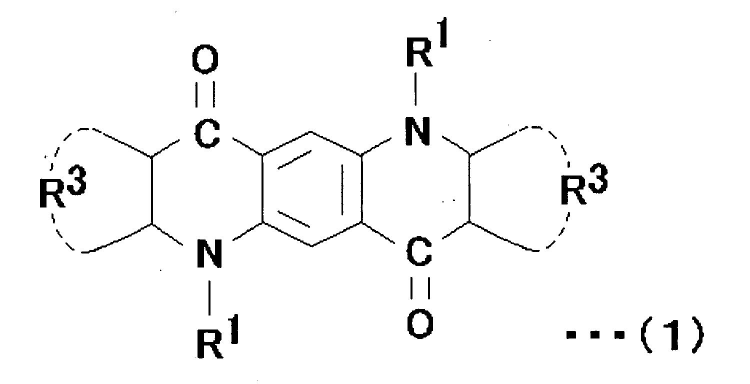 In formula (1), R¹ is a hydrogen atom, an alkyl group with 1 to 10 carbon atoms, an aryl group represented by formula (2), or an aralkyl group represented by formula (3), wherein there are no cases where both R¹ s are hydrogen atoms. R³ in formula (1) denotes one of the substituents respectively represented by formulas (4) - (8) , wherein two R³ s may be the same or different from each other.

Formula (2) is: 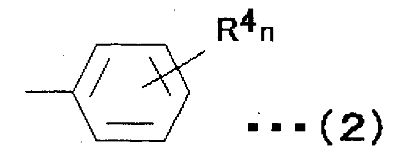 wherein R⁴ is a hydrogen atom, an alkyl group with 1 to 10 carbon atoms, or an alkoxyl group with 1 to 5 carbon atoms; and n denotes an integer from 1 to 5.

Formula (3) is: 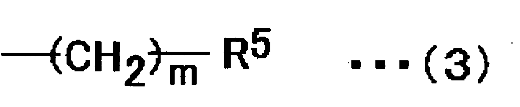 wherein R⁵ is an aryl group represented by formula (2) above; and m denotes an integer from 1 to 10.

Formula (4) is: 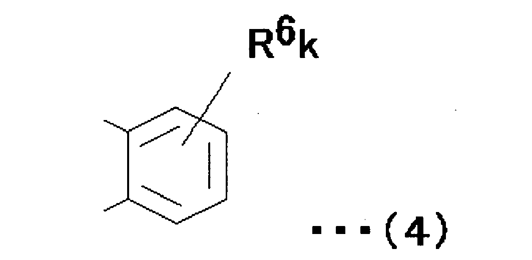 wherein R⁶ is a hydrogen atom, an alkyl group with 1 to 10 carbon atoms, an alkoxyl group with 1 to 5 carbon atoms, or an aryl group represented by formula (2) ; and k denotes an integer from 1 to 4.

Formula 5 is:

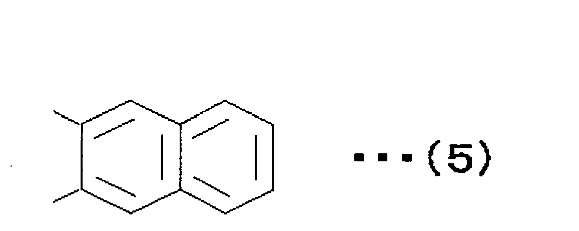

Formula (6) is:

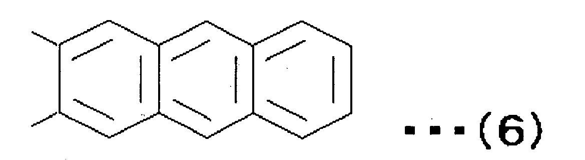

Formula (7) is:

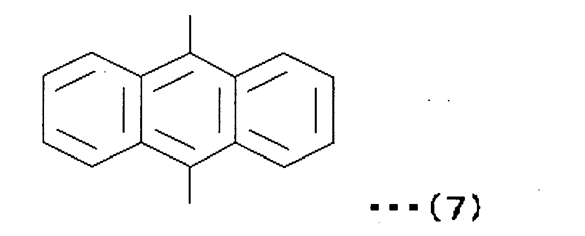

Formula (8) is:

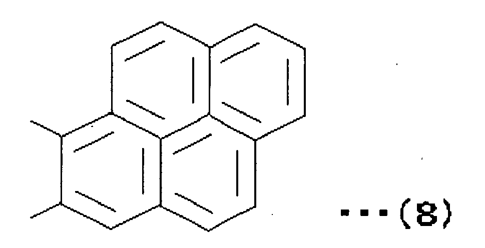

The second means provided by the present invention to achieve the objectives is a process of producing a white light-emitting compound represented by formula (1), comprising dehydrating an aromatic amine represented by formula (9) and a diol represented by formula (10) to produce a first compound represented by formula (11); dehydrogenating the first compound; reacting the dehydrogenated compound with an alkyl halide, the chemical formula of which is R¹-X wherein R¹ denotes the same as that defined in relation to the first means, and X is a halogen atom, to produce a second compound represented by formula (12); and subjecting the second compound to a ring-closing reaction.

Formula (9) is:

R³ ― NH₂ (9)

wherein R³ denotes the same as that defined in relation to the first means.

Formula (10) is: 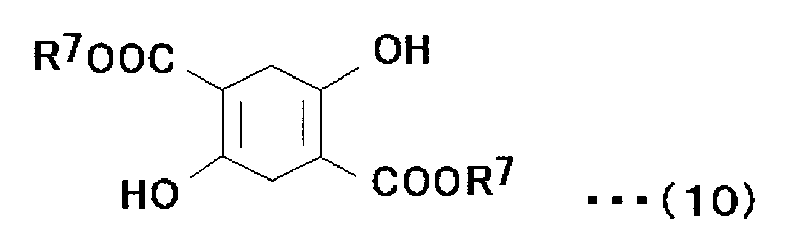 wherein two R⁷s in formula (10) may be the same or different from each other.

Formula (11) is: 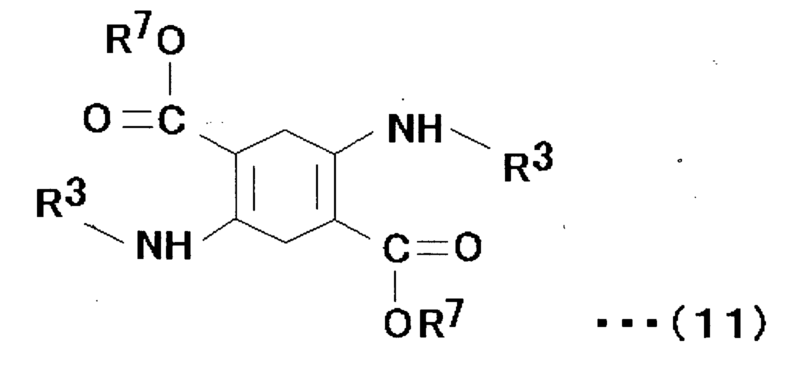 wherein R³ and R⁷ in formula (11) denote the same as those defined above.

Formula (12) is: 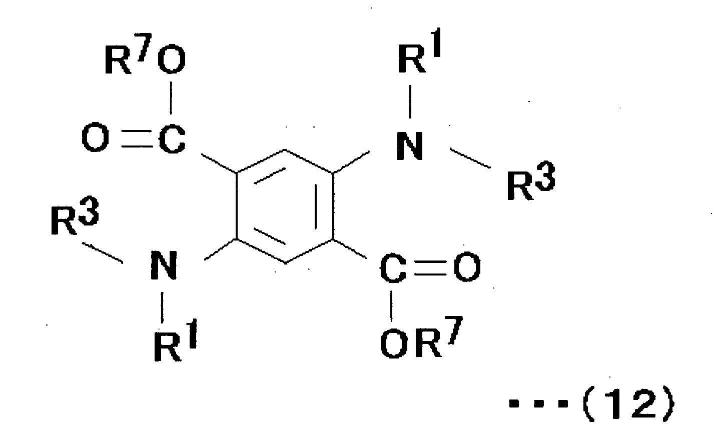 wherein R¹ in formula (12) denotes the same as that defined in relation to the first means and there are no cases where both R¹s are hydrogen atoms, and R³ and R⁷ are the same as those defined above.

The third means to achieve the objectives is a white light-emitting element having a pair of electrodes and a light-emitting layer sandwiched between the electrodes, the light-emitting layer including a white light-emitting compound represented by formula (1).

### [Advantages of the Invention]

The present invention can provide a white light-emitting compound capable of emitting white light, and furthermore a process of producing the compound and a luminescent element including the white light-emitting compound.

### BRIEF DESCRIPTION OF THE DRAWINGS

Figure 1 is an illustration showing an example of the white light-emitting element according to the present invention.
Figure 2 is an illustration showing another example of the white light-emitting element according to the present invention.
Figure 3 is an illustration showing a still another example of the white light-emitting element according to the present invention.
Figure 4 is an illustration showing a further example of the white light-emitting element according to the present invention.
Figure 5 is an NMR spectrum chart of the crystals obtained by the dehydration in Example 1.
Figure 6 is an IR spectrum chart of the crystals obtained by the dehydration in Example 1.
Figure 7 is an NMR spectrum chart of the crystals obtained by the dehydrogenation in Example 1.
Figure 8 is an IR spectrum chart of the crystals obtained by the dehydrogenation in Example 1.
Figure 9 is an IR spectrum chart of the crystals obtained by the alkylation in Example 1.
Figure 10 is an NMR spectrum chart of the crystals obtained by the ring-closure in Example 1.
Figure 11 is an IR spectrum chart of the crystals obtained by the ring-closure in Example 1.
Figure 12 is a fluorescence spectrum chart of the crystals obtained by the ring-closure in Example 1.
Figure 13 is another fluorescence spectrum chart of the crystals obtained by the ring-closure in Example 1.
Figure 14 is an NMR spectrum chart of the crystals obtained by the dehydration in Example 2.
Figure 15 is an IR spectrum chart of the crystals obtained by the dehydration in Example 2.
Figure 16 is an IR spectrum chart of the crystals obtained by the dehydrogenation in Example 2.
Figure 17 is an IR spectrum chart of the crystals obtained by the alkylation in Example 2.
Figure 18 is an NMR spectrum chart of the crystals obtained by the ring-closure in Example 2.
Figure 19 is an IR spectrum chart of the crystals obtained by the ring-closure in Example 2.
Figure 20 is a fluorescence spectrum chart of the crystals obtained by the ring-closure in Example 2.
Figure 21 is an NMR spectrum chart of the crystals obtained by the dehydration in Example 3.
Figure 22 is an IR spectrum chart of the crystals obtained by the dehydration in Example 3.
Figure 23 is an NMR spectrum chart of the crystals obtained by the dehydrogenation in Example 3.
Figure 24 is an IR spectrum chart of the crystals obtained by the dehydrogenation in Example 3.
Figure 25 is an IR spectrum chart of the crystals obtained by the alkylation in Example 3.
Figure 26 is an NMR spectrum chart of the crystals obtained by the ring-closure in Example 3.
Figure 27 is an IR spectrum chart of the crystals obtained by the ring-closure in Example 3.
Figure 28 is a fluorescence spectrum chart of the crystals obtained by the ring-closure in Example 3.
Figure 29 is an NMR spectrum chart of the crystals obtained by the dehydration in Example 4.
Figure 30 is an IR spectrum chart of the crystals obtained by the dehydration in Example 4.
Figure 31 is an NMR spectrum chart of the crystals obtained by the dehydrogenation in Example 4.
Figure 32 is an IR spectrum chart of the crystals obtained by the dehydrogenation in Example 4.
Figure 33 is an IR spectrum chart of the crystals obtained by the alkylation in Example 4.
Figure 34 is an NMR spectrum chart of the crystals obtained by the ring-closure in Example 4.
Figure 35 is an IR spectrum chart of the crystals obtained by the ring-closure in Example 4.
Figure 36 is a fluorescence spectrum chart of the crystals obtained by the ring-closure in Example 4.
Figure 37 is an NMR spectrum chart of the crystals obtained by the dehydration in Example 5.
Figure 38 is an IR spectrum chart of the crystals obtained by the dehydration in Example 5.
Figure 39 is an NMR spectrum chart of the crystals obtained by the dehydrogenation in Example 5.
Figure 40 is an IR spectrum chart of the crystals obtained by the dehydrogenation in Example 5.
Figure 41 is an IR spectrum chart of the crystals obtained by the alkylation in Example 5.
Figure 42 is an NMR spectrum chart of the crystals obtained by the ring-closure in Example 5.
Figure 43 is an IR spectrum chart of the crystals obtained by the ring-closure in Example 5.
Figure 44 is a fluorescence spectrum chart of the crystals obtained by the ring-closure in Example 5.
Figure 45 is an NMR spectrum chart of the crystals obtained by the dehydration in Example 6.
Figure 46 is an IR spectrum chart of the crystals obtained by the dehydration in Example 6.
Figure 47 is an NMR spectrum chart of the crystals obtained by the dehydrogenation in Example 6.
Figure 48 is an IR spectrum chart of the crystals obtained by the dehydrogenation in Example 6.
Figure 49 is an NMR spectrum chart of the crystals obtained by the ring-closure in Example 6.
Figure 50 is an IR spectrum chart of the crystals obtained by the ring-closure in Example 6.
Figure 51 is a fluorescence spectrum chart of the crystals obtained by the ring-closure in Example 6.
Figure 52 is an NMR spectrum chart of the crystals obtained by the dehydration in Example 7.
Figure 53 is an IR spectrum chart of the crystals obtained by the dehydration in Example 7.
Figure 54 is an NMR spectrum chart of the crystals obtained by the dehydrogenation in Example 7.
Figure 55 is an IR spectrum chart of the crystals obtained by the dehydrogenation in Example 7.
Figure 56 is an NMR spectrum chart of the crystals obtained by the ring-closure in Example 7.
Figure 57 is an IR spectrum chart of the crystals obtained by the ring-closure in Example 7.
Figure 58 is a fluorescence spectrum chart of the crystals obtained by the ring-closure in Example 7.
Figure 59 is an NMR spectrum chart of the crystals obtained by the ring-closure in Example 8.
Figure 60 is an IR spectrum chart of the crystals obtained by the ring-closure in Example 8.
Figure 61 is a fluorescence spectrum chart of the crystals obtained by the ring-closure in Example 8.

### [Explanation of Reference Numerals]

A, B, C: white light-emitting element
1: substrate
2: transparent electrode
3: light-emitting layer
4: electrode layer

### BEST MODE TO CARRY OUT THE INVENTION

The white light-emitting compound according to the present invention is represented by formula (1):

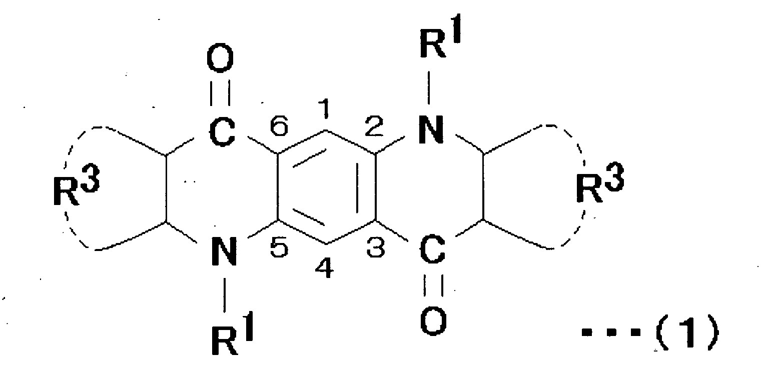

The white light-emitting compound represented by formula (1) is composed of one benzene ring, two carbonyl groups, two alkyl imino groups, the chemical structure of which is -N (R¹) -, and two groups represented by R³ The numerals 1-6 included in formula (1) show the positions for the convenience of explanation.

The benzene ring is bonded with one of the carbonyl groups at the 3-position and with the other at the 6-position, and with one of the alkyl imino groups at the 2-position and with the other at the 5-position.

Each carbonyl group and each alkyl imino group are bonded with a group R³

R¹ may be a hydrogen atom or an alkyl group with 1 to 10 carbon atoms. There are no cases where both R¹ s are hydrogen atoms.

Examples of the alkyl group with 1 to 10 carbon atoms are methyl group, ethyl group, propyl group, isopropyl group, n-butyl group, isobutyl group, sec-butyl group, tert-butyl group, n-pentyl group, sec-pentyl group, tert-pentyl group, n-hexyl group, n-heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group. The most preferable are methyl group, ethyl group, and a propyl group.

The alkyl group with 1 to 10 carbon atoms may be a fluorine atom-including alkyl group where at least one of the hydrogen atoms is replaced with a fluorine atom. Examples of the fluorine atom-including alkyl group with 1 to 10 carbon atoms are fluoromethyl group, difluoromethyl group, trifluoromethyl group, fluoroethyl group, 1,1-difluoroethyl group, 1,2-difluoroethyl group, 1,1,1-trifluoroethyl group, 1,1,2-trifluoroethyl group, 1,2,2-trifluoroethyl group, 1,1,2,2-tetrafluoroethyl group, 1,1,2,2,2-pentafluoroethyl group, 1-fluoropropyl group, 2-fluoropropyl group, 1,1-difluoropropyl group, 1,2-difluoropropyl group, 1,3-difluoropropyl group, 2,2-difluoropropylgroup, 1,1,1-trifluoropropyl group, 1,1,2-trifluoropropyl group, 1,2,3-trifluoropropyl group, 1,2,2-trifluoropropylgroup, and 1,3,3-trifluoropropyl group.

Also, R¹ may be an aryl group represented by formula (2) .

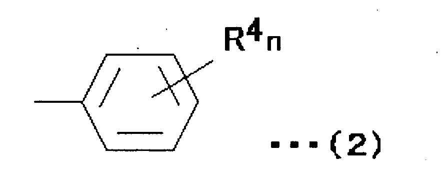

The aryl group represented by formula (2) has phenyl group as its basic skeleton, and the phenyl group is bonded with up to five R⁴s.

R⁴ in formula (2) is a hydrogen atom, an alkyl group with 1 to 10 carbon atoms, or an alkoxyl group with 1 to 5 carbon atoms. "n" denotes an integer from 1 to 5.

The alkyl group with 1 to 10 carbon atoms is the same as that defined in the explanation of formula (1).

Examples of the alkoxyl group with 1 to 5 carbon atoms include methoxyl group, ethoxyl group, propoxyl group, isopropoxyl group, butoxyl group, s-butoxyl group, t-butoxyl group, a pentoxyl group. Among them are preferred an alkoxyl group with 1 to 3 carbon atoms. Particularly preferable are methoxyl group and ethoxyl group.

The aryl group represented by formula (2) preferably has at least one alkoxyl group. Although the alkoxyl group may be bonded to the aryl group at any position, the o- andm-positions are preferable.

R¹ in formula (1) may also be an aralkyl group represented by formula (3):

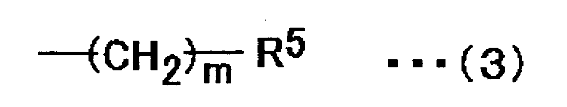

The aralkyl group represented by formula (3) is composed of one or more methylene groups and R⁵ R⁵ is an aryl group represented by formula (2). "m" in formula (3) denotes the number of methylene groups between the nitrogen atom in formula (1) and R⁵, the aryl group. Although the number may be any number, it should be 1 or 2.

Specific examples of the aralkyl group include benzyl group and phenethyl group. Benzyl group is particularly preferable.

R³ in formula (1) may be a substituent represented by formula (4). The numerals 1 to 6 in the formula show the positions for the convenience of explanation.

Formula (4) is:

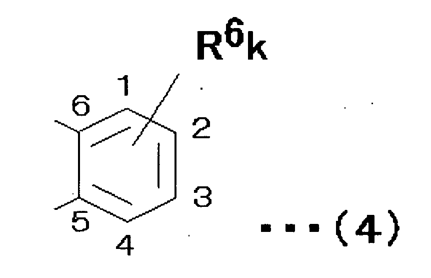

The group represented by formula (4) comprises a benzene ring. Adjacent two carbon atoms of the benzene ring are respectively bonded with the carbon atom of a carbonyl group and the nitrogen atom of the alkyl imino group located on the same side as the carbonyl group in relation to the benzene ring in formula (1).

For example, the 5-positioned carbon atom of the benzene ring in formula (4) is bonded with the carbon atom of a carbonyl group in formula (1) , and the 6-positioned carbon atom thereof in formula (4) with the nitrogen atom of the alkyl imino group located on the same side as the carbonyl group in relation to the benzene ring in formula (1). At least one of the other carbon atoms of the benzene ring in formula (4) is bonded with R⁶

R⁶ denotes a hydrogen atom, an alkyl group with 1 to 10 carbon atoms, an alkoxyl group with 1 to 5 carbon atoms, or an aryl group represented by formula (2) . "k" in formula (4) denotes an integer from 1 to 4.

Examples of the alkyl group with 1 to 10 carbon atoms are methyl group, ethyl group, propyl group, isopropyl group, n-butyl group, isobutyl group, sec-butyl group, tert-butyl group, n-pentyl group, sec-pentyl group, tert-pentyl group, n-hexyl group, n-heptyl group, an octyl group, a nonyl group, a decyl group, etc. Among those are preferred an alkyl group having 1 to 7 carbon atoms, such as methyl group, ethyl group, propyl group, isopropyl group, n-butyl group, isobutyl group, sec-butyl group, tert-butyl group, n-pentyl group, sec-pentyl group, tert-pentyl group, n-hexyl group; or n-heptyl group.

The alkoxyl group with 1 to 5 carbon atoms and the aryl group represented by formula (2) are the same as those explained above.

It is preferable if the group represented by formula ·(4) has at least one aryl group represented by formula (2) as substituent R⁶. The substituent(s) R⁶ may be bonded at any positions.

R³ in formula (1) may also be the group represented by formula (5). The numerals 1 to 8 included in formula (5) show the positions for the convenience, of explanation.

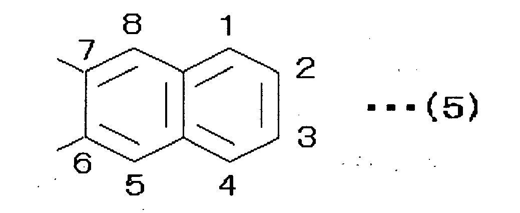

The group represented by formula (5) comprises a naphthalene ring, and the 6-positioned and 7-positioned carbon atoms thereof are respectively bonded with the carbon atom of the carbonyl group and the nitrogen atom of the alkyl imino group in' formula (1). The positions of the naphthalene ring where the naphthalene is bonded with the carbon atom of the carbonyl group and with the nitrogen atom of the alkyl imino group are not limited to the 6- and 7-positions. They may also be the 1- and 2-positions, the 2- and 3-positions, the 3- and 4-positions, the 4- and 5-positions, the 5- and 6-positions, or the 7- and 8-positions.

R³ in formula (1) may further be a group represented by formula (6). The numerals 1-10 in the formula show the positions for the convenience of explanation.

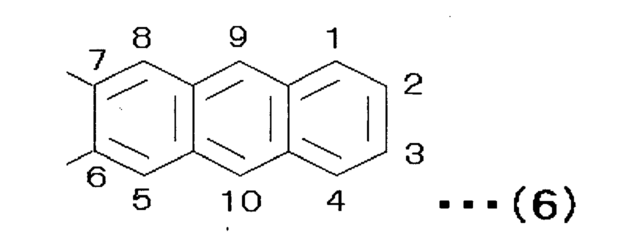

The group represented by formula (6) comprises an anthracene ring, and the 6-positioned and 7-positioned carbon atoms thereof are respectively bonded with the carbon atom of the carbonyl group and the nitrogen atom of the alkyl imino group in formula (1). The positions of the anthracene ring where the anthracene is bonded with the carbon atom of the carbonyl group and with the nitrogen atom of the alkyl imino group are not limited to the 6- and 7-positions. They may also be the 1- and 2-positions, the 2- and 3-positions, the 3- and 4-positions, the 5- and 6-positions, or the 7- and 8-positions.

R³ in formula (1) may still be a group represented by formula (7). The numerals 1-10 in the formula show the positions for the convenience of explanation.

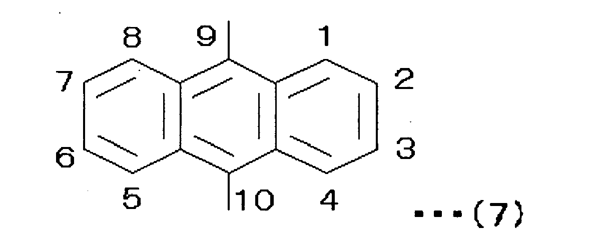

The group represented by formula (7) comprises an anthracene ring, and the 9-positioned and 10-positioned carbon atoms thereof are respectively bonded with the carbon atom of the carbonyl group and the nitrogen atom of the alkyl imino group in formula (1). The positions of the anthracene ring where the anthracene is bonded with the carbon atom of the carbonyl group and with the nitrogen atom of the alkyl imino group are not limited to the 9- and 10-positions. They may also be the 1- and 4-positions, or the 5- and 8-positions.

R³ in formula (1) may still further be a group represented by formula (8). The numerals 1-10 in the formula show the positions for the convenience of explanation.

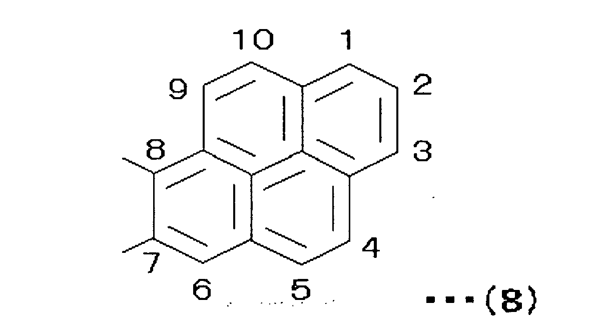

The group represented by formula (8) comprises a pyrene ring, and the 7-positioned and 8-positioned carbon atoms thereof are respectively bonded with the carbon atom of the carbonyl group and the nitrogen atom of the alkyl imino group in formula (1). The positions of the pyrene ring where the pyrene is bonded with the carbon atom of the carbonyl group and with the nitrogen atom of the alkyl imino group are not limited to the 7- and 8-positions. They may also be the 1- and 2-positions, the 2-and 3-positions, the 4- and 5-positions, the 6- and 7-positions, or the 9- and 10-positions.

In the foregoing we have explained the structural features of the white light-emitting compound according to the present invention. Interestingly, as understood from Example 1, a solution of the white light-emitting compound of the present invention prepared by dissolving the compound in a polar solvent, such as benzene and toluene, emits white light, while a solution of the compound prepared by dissolving the compound in a protonic acid, such as sulfuric acid, phosphoric acid, or polyphosphoric acid, emits red light.

The white light-emitting compound represented by formula (1) may be prepared by the steps comprising dehydrating an aromatic amine and a diol to produce a compound; dehydrogenating the compound; alkylating the dehydrogenated compound; and ring-closing the resultant compound.

The aromatic amine includes monocyclic aromatic amines and polycyclic aromatic amines with two or more rings in a molecule, such as amines of biphenyl, naphthalene, anthracene or pyrene.

Specific examples of the monocyclic aromatic amine include 2-alkylanilines such as 2-tert-butylaniline, 4-n-alkylanilines such as 4-n-hexylaniline, 4-n-heptylaniline, or 4-n-octylaniline, or 2-methoxy-5-alkylaniline such as 2,5-dimethoxyaniline.

Specific examples of the polycyclic aromatic amine include biphenylamines such as 2-aminobiphenyl, 3-aminobiphenyl, 2-amino-3-methoxybiphenyl, 2-amino-4-methoxybiphenyl, 2-amino-5-methoxybiphenyl, 2-amino-4-methoxybiphenyl, 2-amino-5-methoxybiphenyl, 2-amino-6-methoxybiphenyl, 3-amino-2-methoxybiphenyl, 3-amino-4-methoxybiphenyl, 3-amino-5-methoxybiphenyl, 3-amino-6-methoxybiphenyl, 4-amino-2-methoxybiphenyl and 4-amino-3-methoxybiphenyl; naphthylamines such as 1-naphthylamine and 2-naphthylamine; anthrylamines such as 1-anthrylamine, 2-anthrylamine and 9-anthrylamine; or aminopyrenes such as 1-aminopyrene and 2-aminopyrene.

In the followings we are describing in detail the process of producing the white light-emitting compound according to the present invention from an aromatic amine represented by formula (9) and a diol represented by formula (10).

Formula (9) is:

R³ ― NH₂ (9)

wherein R³ in formula (9) denotes the same as that defined above.

Formula (10) is: 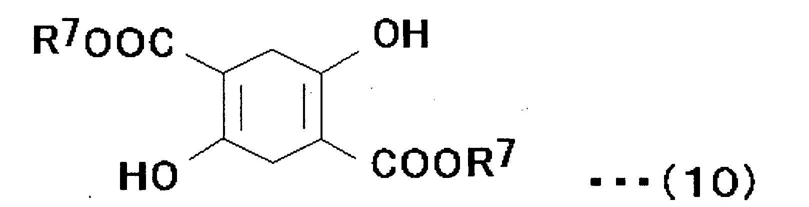 wherein R⁷ is a straight-chain alkyl group with 1 to 3 carbon atoms.

The straight-chain alkyl group with 1 to 3 carbon atoms includes methyl group, ethyl group and n-propyl group.

A dehydrating reaction takes place between the amino group of the aromatic amine and the hydroxyl group of the diol, when the reactants are heated in a solvent.

For the solvent may be used alcoholic solvents such as methanol, ethanol, and isopropanol, or acidic solvents such as acetic acid such as acetic acid, acetic anhydride, phthalic acid and phthalic anhydride.

The reaction temperature should be from 100°C to 130°C.

A dehydrating catalyst may be present in the reaction mixture.

Known catalysts may be used for the dehydrating catalyst. Examples of the dehydrating catalyst include aluminum oxide, calcium oxide, and copper oxide.

The dehydrating reaction provides a first compound represented by formula (11).

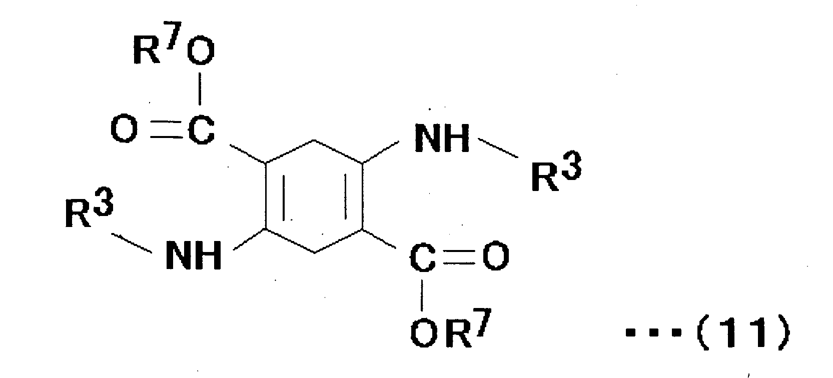

Then, the first compound is subjected to dehydrogenation by heating the first compound in a solvent in the presence of a dehydrogenating catalyst.

For the solvent may be used a non-polar solvent, or a polar solvent such as o-dichlorobenzene, m-dichlorobenzene, pyridine, dioxane, and N,N-dimethylformamide.

The reaction temperature should range between 140°C and 180°C.

For the dehydrogenating catalyst may be used a known dehydrogenating catalyst such as hydrochloric acid, sulfuric acid, nitric acid, iron, zinc, aluminum oxide or aluminum chloride.

The dehydrogenation changes the cyclohexadiene ring located in the center of the first compound represented by formula (11) to a benzene ring, and changes the first compound accordingly.

Then, the compound produced through the dehydrogenation is alkylated with an alkyl halide, the chemical structure of which is R¹-X, by heating a solution including them dissolved in a solvent.

"X" in the chemical structure of the alkyl halide denotes a halogen atom, for which a chlorine atom, a fluorine atom, or a bromine atom may be used.

R¹ in the chemical structure of the alkyl halide is the same as that defined above.

For the solvent may be used a non-polar solvent, or a polar solvent such as o-dichlorobenzene, m-dichlorobenzene, pyridine, dioxane, and N,N-dimethylformamide.

The reaction temperature should be from 140°C to 180°C.

This reaction may be carried out in the presence of a catalyst optionally.

The alkylating reaction produces a second compound represented by formula (12).

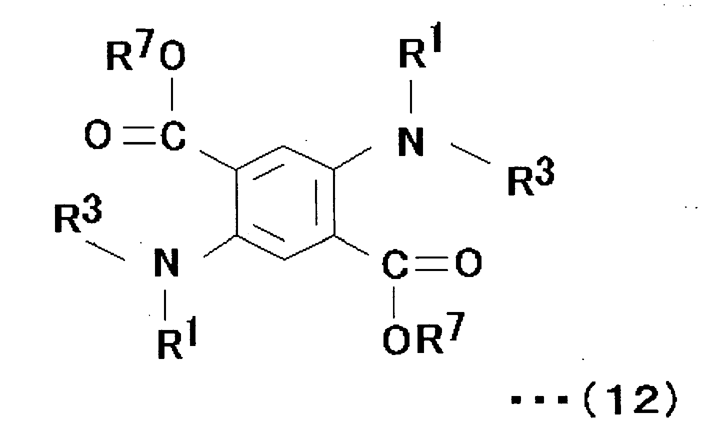

The second compound represented by formula (12) is dissolved in a solvent, and the obtained solution was heated in the presence of a catalyst. Then, a ring-closing reaction takes place with the second compound.

For the solvent may be used a non-polar solvent, or a polar solvent such as o-dichlorobenzene, p-dichlorobenzene, pyridine, dioxane, and N,N-dimethylformamide.

The reaction temperature should be from 140°C to 180°C.

Any known catalyst that is able to expedite the ring-closing reaction may be employed for this reaction. Examples of the catalyst are toluenesulfonic acid and xylenesulfonic acid.

The ring-closing reaction provides a reaction product including the compound represented by formula (1).

After the termination of the reaction, the compound represented by formula (1) is separated and purified by ordinary methods. The compound obtained is easily confirmed by IR spectroscopy, NMR spectroscopy and fluorometry.

The white light-emitting compound according to the present invention is produced from an aromatic amine represented by formula (9) and a diol represented by formula (10) through the dehydration, dehydrogenation, alkylation and ring-closure, which means that the compound of the present invention can easily be produced merely by heating. Therefore this simple method of producing the white light-emitting compound is considered to be an industrial one.

The white light-emitting element utilizing the white light-emitting compound of the present invention will be explained hereinafter.

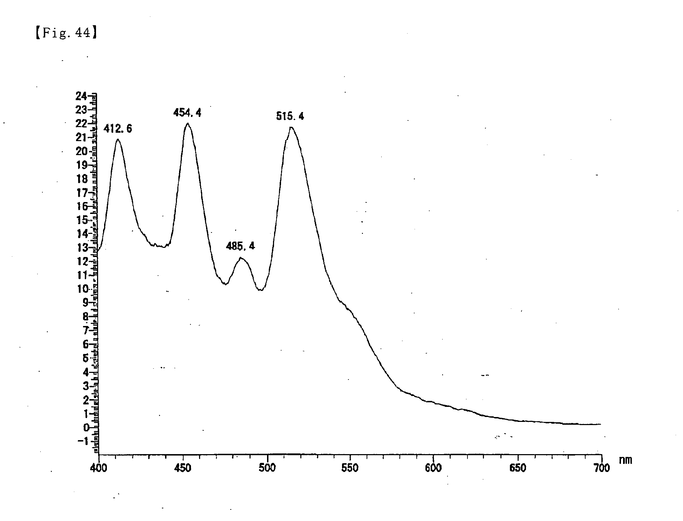

It is observed that the white light-emitting compound according to the present invention emits visible light, the wavelength of which ranges between 400 nm and 620 nm, upon an application of electromagnetic energy. A typical fluorescent spectrum thereof is shown in Figure 44. This compound may be utilized for organic EL elements able to emit white light.

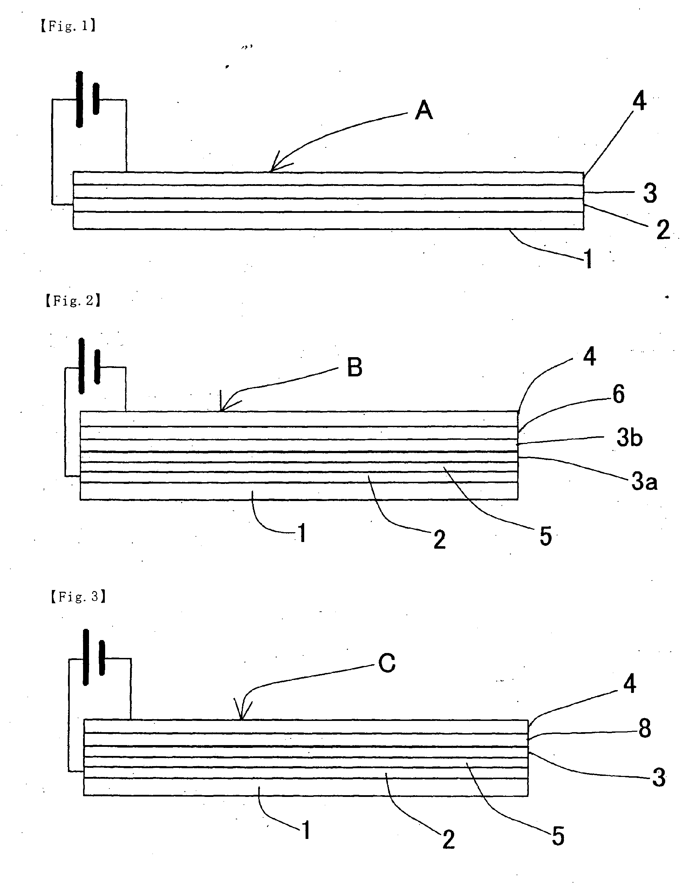

Figure 1 is a schematic illustration that shows the sectional structure of a white light-emitting element, which is a one-layer type organic EL element. As shown in this figure, a white light-emitting element A is prepared by layering a light-emitting layer 3 and an electrode layer 4 in this order on a substrate 1 with which a transparent electrode 2 has been provided. The light-emitting layer 3 includes light-emitting substances.

When electric current is applied to the white light-emitting element A shown in Figure 1 at the transparent electrode 2 and the electrode layer 4, the element emits white light due to the white light-emitting compound. Upon the application of an electric field between the transparent electrode 2 and the electrode layer 4, electrons are injected from the electrode layer 4 and positive holes from the transparent electrode 2. In the light-emitting layer 3, the electrons are recombined with positive holes, which causes the energy level to return to the valence band from the conduction band. This transition of the energy level is accompanied by emission of the energy differential as light.

The white light-emitting element A shown in Figure 1, when it is shaped to a planar form with a large area, may be used as a planar illuminator, for example a large-area wall illuminator-when fixed on a wall, or a large-area ceiling illuminator when fixed on a ceiling. This white light-emitting element may be utilized for a planar light source in place of a point light source, such as a conventional bulb, and a line light source, such as a conventional fluorescent lamp. In particular, this illuminator can suitably be used to light up walls, ceilings and floors in dwelling rooms, offices and passenger trains, or to make them emit light. Moreover, this white light-emitting element Amay be suitable for the backlight used in displays of computers, cellular phones and ATMs. Furthermore, the white light-emitting element A may be used for various light sources, such as the light source of direct illumination and that of indirect illumination. Also, it may be used for the light sources of advertisement apparatuses, road traffic sign apparatuses and light-emitting billboards, which have to emit light at night and provide good visibility. In addition, because this white light-emitting element A includes the white light-emitting compound with the special structure in thelight-emittinglayer,the whitelight-emitting element A may have a long life. Therefore, light sources employing the white light-emitting element A will naturally have a long life.

The white light-emitting element A may also be shaped into a tubular light emitter comprising a tubularly shaped substrate 1, a transparent electrode 2 placed on the inside surface of the substrate 1, a light-emitting layer 3 and an electrode layer 4 placed on the transparent electrode 2 in this order. Because the white light-emitting element A does not include mercury, it is an ecological light source and may be a substitute for conventional fluorescent lamps.

For the substrate 1 may be used any known substrate, as long as the transparent electrode 2 can be formed on the surface of the substrate. Examples of the substrate 1 are a glass substrate, a plastic sheet, a ceramic substrate, and a metal substrate the surface of which is insulated, for example, through the formation of an insulating layer thereon. When the substrate 1 is opaque, the white light-emitting element is a single-faced illuminator that emits white light from the surface layer opposite to the substrate. On the other hand, when the substrate 1 is transparent, the element is a double-faced illuminator that emits white light from both of the substrate and the surface layer opposite to the substrate.

For the transparent electrode 2, various materials may be employed, as long as their work functions are large, they are transparent, and they can function as a cathode and inject holes to the light-emitting layer 3 when voltage is applied thereto. Specifically, the transparent electrode 2 may be made of a transparent inorganic conductive material of ITO, In₂O₃, SnO₂, ZnO, CdO, etc. and derivatives thereof, or an electrically conductive high polymer such as polyaniline.

The transparent electrode 2 may be formed on the substrate 1 by chemical vapor phase deposition, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 is a layer that includes a specific white light-emitting compound according to the present invention. The light-emitting layer 3 may be a high polymer film where a white light-emitting compound according to the present invention is dispersed in a high polymer. The layer may also be a deposited film prepared by depositing a white light-emitting compound according to the present invention on the transparent electrode 2.

Examples of the high polymer for the high polymer film are a polyvinyl carbazole, a poly(3-alkylthiophene), a polyimide including an arylamide, a polyfluorene, a polyphenylene vinylene, a poly-α-methylstyrene, a copolymer of vinyl-carbazole and α-methylstyrene. Among them, a polyvinyl carbazole is preferable.

The amount of the white light-emitting compound of the present invention included in the high polymer film is, typically 0.01-2% by weight, preferably 0.05-0.5% by weight.

The thickness of the light-emitting layer 3 ranges, typically between 30 nm and 500 nm, preferably between 100 nm and 300 nm. When the thickness is too small, the amount of the emitted light may be insufficient. On the other hand, when the thickness is too large, the voltage required to drive the illuminator or element may be too high, which is not desirable. Besides, the large thickness may reduce the flexibility of the film necessary to shape a planar, tubular, curved, or ring article.

The film or sheet including the white light-emitting compound may be formed through the application of a solution of the white light-emitting compound dissolved in a suitable solvent. The application method is one selected from a spin cast method, a coating method, a dipping method, etc.

When the light-emitting layer 3 is a deposited film, the thickness of the film is generally 0.1-100 nm, although a preferable thickness is different depending on the structure of layers and other factors. When the thickness is too large or too small, it might cause the same problems as described above.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode 4 may easily be formed on the surface of light-emitting layer 3, which, in turn, has been formed on substrate 1, by the technique of metal deposition.

When either of the application method or the deposition method is employed, a buffer layer should be inserted between each electrode and the light-emitting layer.

Materials for the buffer layer are, for example, an alkaline metal compound such as lithium fluoride, an alkaline earth metal compound such as magnesium fluoride, an oxide such as an aluminum oxide, and 4,4' -biscarbazole biphenyl (Cz-TPD). Also, materials for forming the buffer layer between the cathode made of a material such as ITO and the organic layer are, for example, m-MTDATA (4,4',4"-tris(3-methylphenyl-phenylamino) triphenylamine), phthalocyanine, polyaniline, and polythiophene derivatives, and inorganic oxides such as molybdenum oxide, ruthenium oxide, vanadium oxide and lithium fluoride. When the materials are suitably selected, these buffer layers can lower the driving voltage of the organic EL element, which is the white light-emitting element, improve the quantum efficiency of luminescence, and achieve an increase in the luminance of the emitted light.

The second example of the white light-emitting element according to the present invention is shown in Figure 2. This figure is an illustration showing the sectional layer structure of an example of the white light-emitting element, which is a multi-layer organic EL element.

As shown in Figure 2, the white light-emitting element B comprises a substrate 1, and a transparent electrode 2, a hole-transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the white light-emitting element A in Figure 1.

The light-emitting layer of the white light-emitting element B comprises light-emitting sublayers 3a and 3b. The light-emitting sublayer 3a is a deposited film formed by depositing the white light-emitting compound on the hole-transporting layer 5. The light-emitting sublayer 3b is a DPVBi layer, which functions as a host material.

Examples of the hole-transporting substance included in the hole-transporting layer 5 are a triphenylamine compound such as N,N'-diphenyl-N,N'-di(m-tolyl)-benzidine (TPD) and α-NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2-(4-tert-butylphenyl)-5-(4-biphenylyl)-1,3,4-oxadiazole and 2,5-bis(1-naphthyl)-1,3,4-oxadiazole (BND), and 2,5-bis (5'-tert-butyl-2'-benzoxazolyl) thiophene. Also, a metal complex material such as quinolinol aluminum complex (Alq3), benzoquinolinol beryllium complex (Bebq2) may be used suitably.

The electron-transporting layer 6 of the white light-emitting element B shown in Figure 2 includes Alq3 as electron-transporting substance.

The thickness of each layer is the same as that in a known multi-layer organic EL element.

The white light-emitting element B in Figure 2 functions and emits light in the same ways as the white light-emitting element A in Figure 1. Therefore, the white light-emitting element B has the same uses as the white light-emitting element A.

The third example of the white light-emitting element of the present invention is shown in Figure 3. This figure is an illustration showing the sectional layer structure of an example of the white light-emitting element, which is a multi-layer organic EL element.

The white light-emitting element C shown in Figure 3 comprises a substrate 1, and a transparent electrode 2, a hole-transporting layer 5, a light-emitting layer 3, an electron- transporting layer 8, and an electrode layer 4, wherein the transparent electrode and the layers are laid on the substrate 1 one by one in this order.

The white light-emitting element C functions in the same way as the white light-emitting element B.

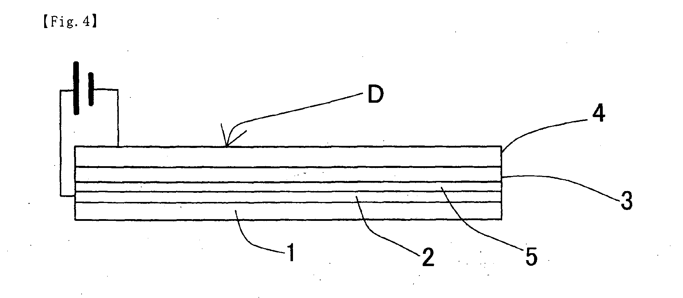

Another example of the white light-emitting element of this invention is shown in Figure 4. The white light-emitting element D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the white light-emitting elements, other than those shown in Figures 1-4, is a two-layer low molecular weight organic white light-emitting element having a hole-transporting layer that includes a hole-transporting substance and an electron-transporting light-emitting layer that includes the organic white-fluorescent compound of the invention laid on the hole-transporting layer, these layers being sandwiched between a cathode, which is the transparent electrode formed on the substrate, and an anode, which is the electrode layer. A specific example of this embodiment is a two-layer pigment-injected white light-emitting element comprising a hole-transporting layer and a light-emitting layer that includes a host pigment and the organic white-fluorescent compound of the present invention as a guest pigment, wherein the light-emitting layer is laid on the hole-transporting layer and these layers are sandwiched between the cathode and the anode. Another example is a two-layer organic white light-emitting element comprising a hole-transporting layer that includes a hole-transporting substance and an electron-transporting light-emitting layer that is made of the organic white-fluorescent compound of the invention and an electron-transporting substance, the latter layer being laid on the former, and these two layers being sandwiched between the cathode and the anode. A specific example of the' second embodiment is a two-layer pigment-injected white light-emitting element comprising a hole-transporting layer and an electron-transporting light-emitting layer that includes a host pigment and the organic white-fluorescent compound of the present invention as a guest pigment, wherein the light-emitting layer is laid on the hole-transporting layer and these layers are sandwiched between the cathode and the anode. A further example is a three-layer organic white light-emitting element comprising a hole-transporting layer, a light-emitting layer including the organic white-fluorescent compound of the present invention that is laid on the hole-transporting layer, and an electron-transporting layer that is laid on the light-emitting layer, these layers being sandwiched between the cathode and the anode.

When the electron-transporting layer of the element according to the present invention typically comprises 50 to 80% by weight of a polyvinylcarbazole (PVK) , 5 to 40% by weight of an electron-transporting luminescent material, and 0.01 to 20% by weight of a white light-emitting compound according to the present invention, the element emits white light at high luminance.

Also, it is preferable, if the light-emitting layer includes, as a sensitizing agent, rubrene, especially both of rubrene and Alq3.

A white light-emitting element utilizing the white light-emitting compound of the present invention may be used as an organic EL element which is driven, generally, by direct current, and also by pulses and alternating current.

### EXAMPLES

### (Example 1) Synthesis of a White Light-emitting Compound

### <Dehydration>

In a 1L three-necked flask were placed 25.0 g of 3-aminobiphenyl, 15 . 5 g of the diol represented by formula (13), 250 ml of acetic acid, and 250 ml of ethanol. The flask containing the mixture was placed in a silicone oil bath and the mixture was heated to 115°C. The mixture was stirred for 4 hours at around the temperature and allowed to react. After the termination of the reaction, the product was cooled naturally. The cooled product was filtered with a glass filter and solids were collected. The solids were washed with methanol, and then with petroleum ether. The washed was vacuum dried. 16.0 g of orange crystals were obtained.

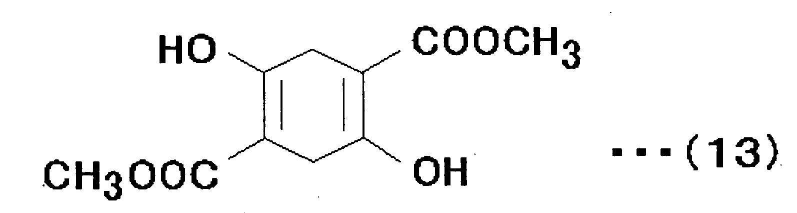

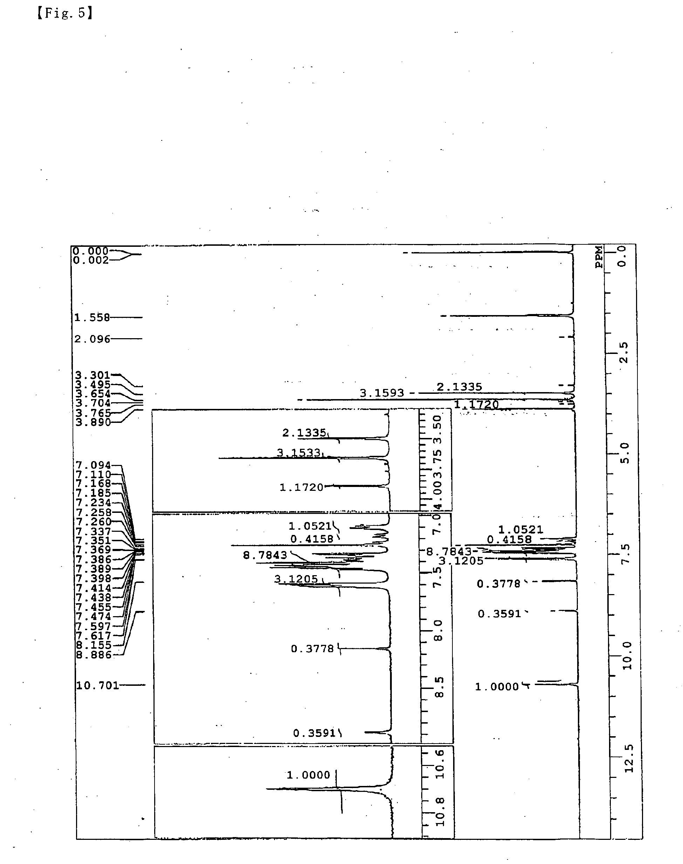

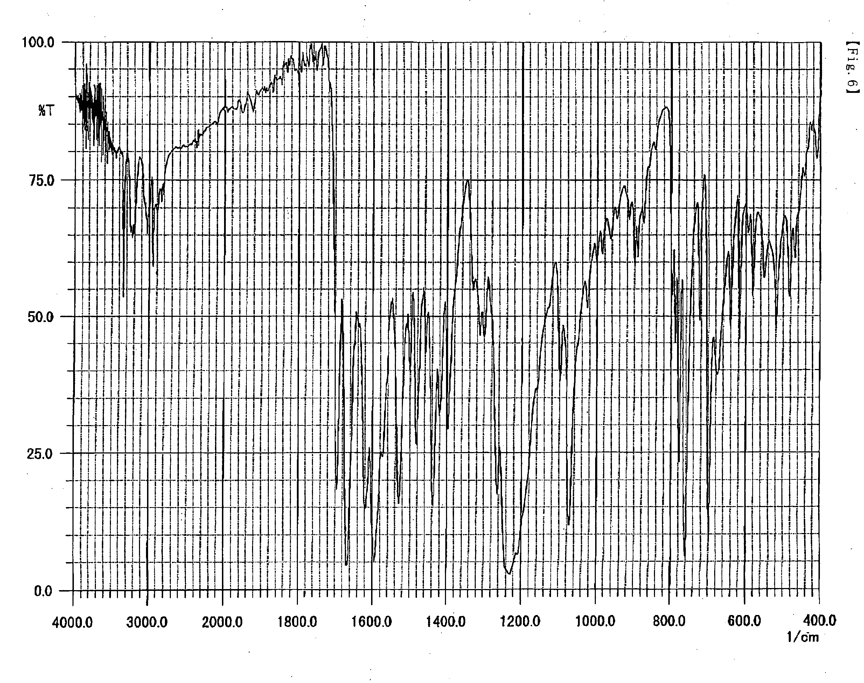

An NMR spectrum chart of the obtained crystals is shown in Figure 5 and an IR spectrum chart thereof in Figure 6.

These orange crystals were identified as the compound having the structure represented by formula (14).

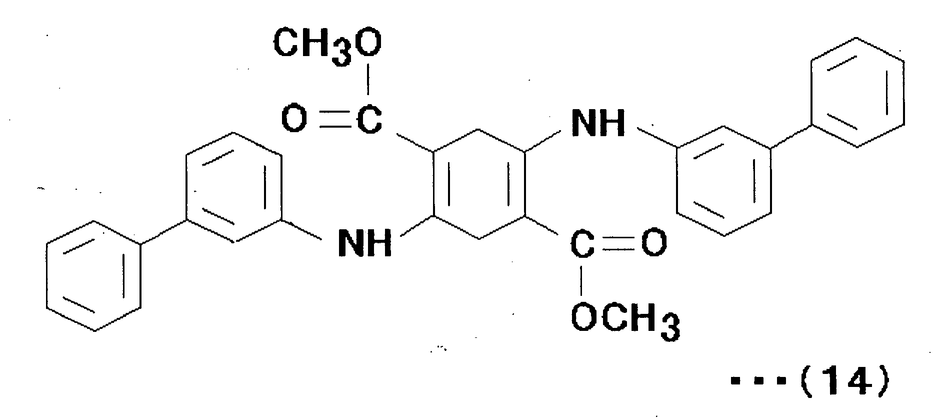

### <Dehydrogenation>

In a 1L three-necked flask were placed 15.0 g of the compound prepared in the step of dehydration and 500 ml of o-dichlorobenzene. A 95% aqueous solution of sulfuric acid was gradually added to the mixture in the flask over 30 minutes with stirring, while the mixture was kept at room temperature. The total amount of the added sulfuric acid solution was 0.2 g. The flask containing the resultant mixture was placed in a silicone oil bath and heated to 160°C. The mixture was stirred for 2 hours at around the temperature and allowed to react. After the termination of the reaction, the product was cooled with ice. The cooled product was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried to 12.5 g of red crystals.

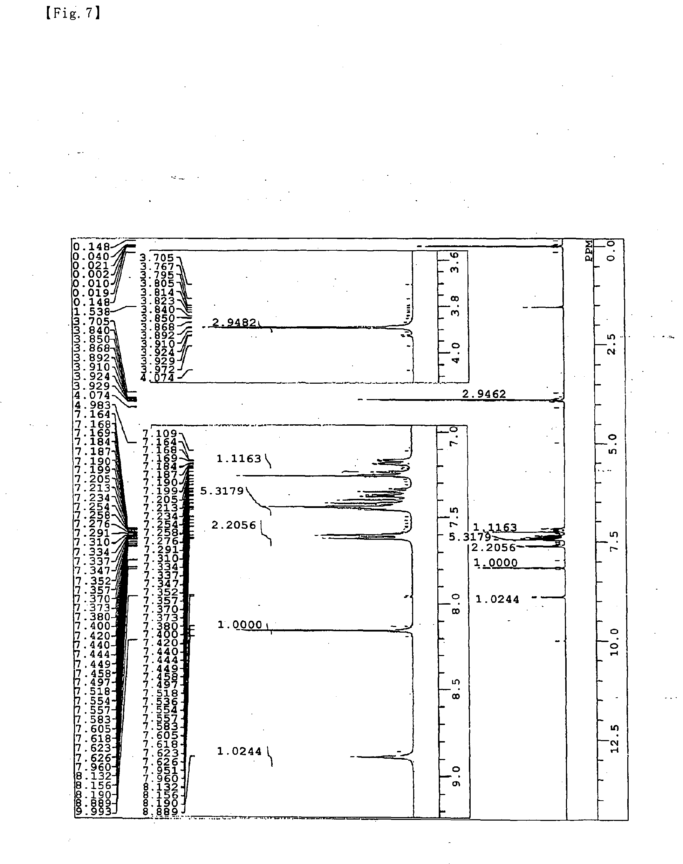

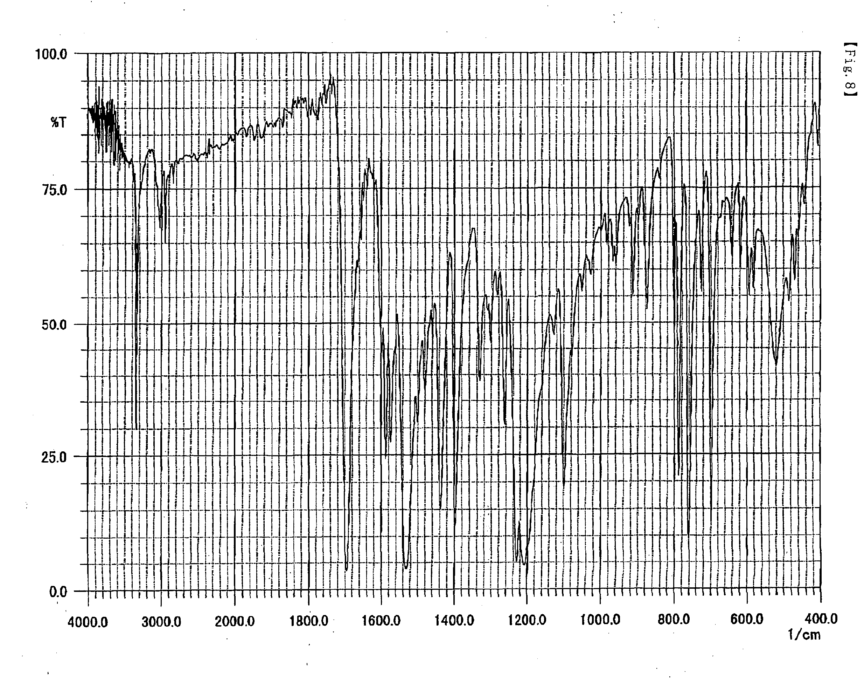

An NMR spectrum chart of the obtained crystals is shown in Figure 7 and an IR spectrum chart thereof in Figure 8.

These red crystals were identified as the compound having the structure represented by formula (15).

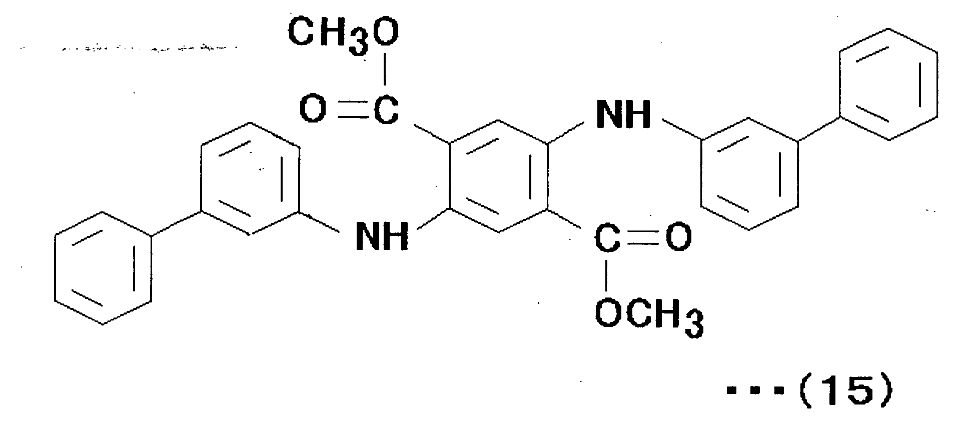

### <Alkylation>

In a 500 ml pressure bottle were placed 5 . 0 g of the compound prepared in the step of dehydrogenation, 8.0 g of α-chloro-p-xylene, and 300 ml of N,N-dimethylformamide. The pressure bottle containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrate was cooled with ice, and then neutralized with sodium hydroxide. The neutralized was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried to 3.2 g of reddish brown crystals.

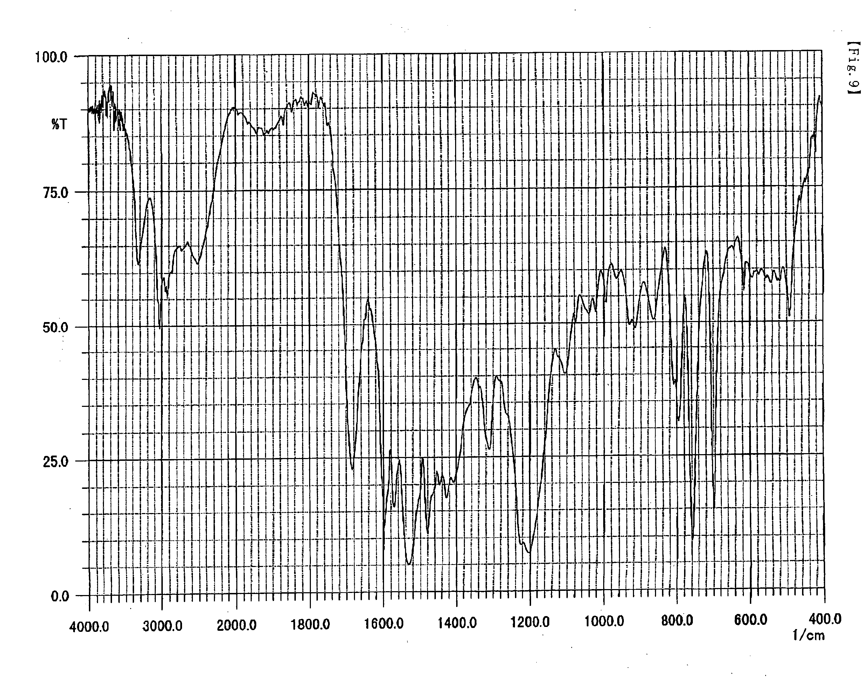

An IR spectrum chart of the obtained crystals is shown in. Figure 9.

These reddish brown crystals were identified based on the chart as the compound having the structure represented by formula (16).

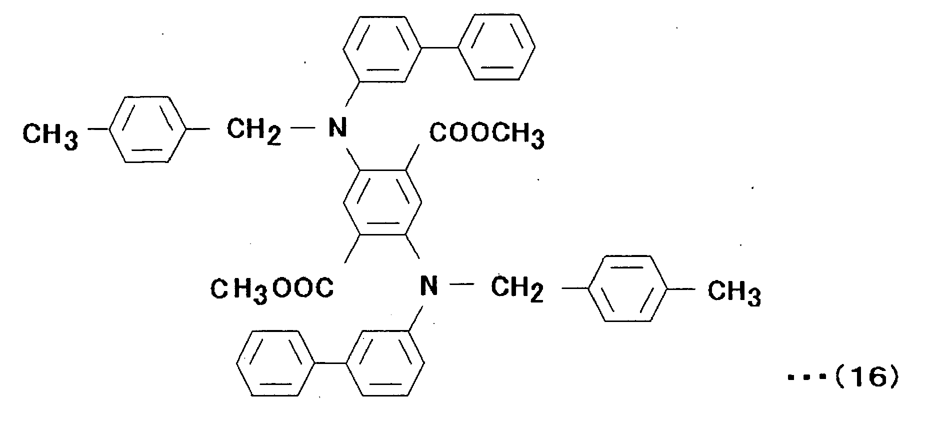

### <Ring Closure>

In a 500 ml three-necked flask were placed 3.0 g of the compound prepared in the step of alkylation, 4.8 g of monohydrated p-toluenesulfonic acid, and 200 ml of o-dichlorobenzene. The flask containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrated was filtered with a glass filter and solids were collected. The solids were washed with methanol, acetone, and petroleum ether in this order. The washed was vacuum dried. 2.1 g of dark violet crystals were obtained.

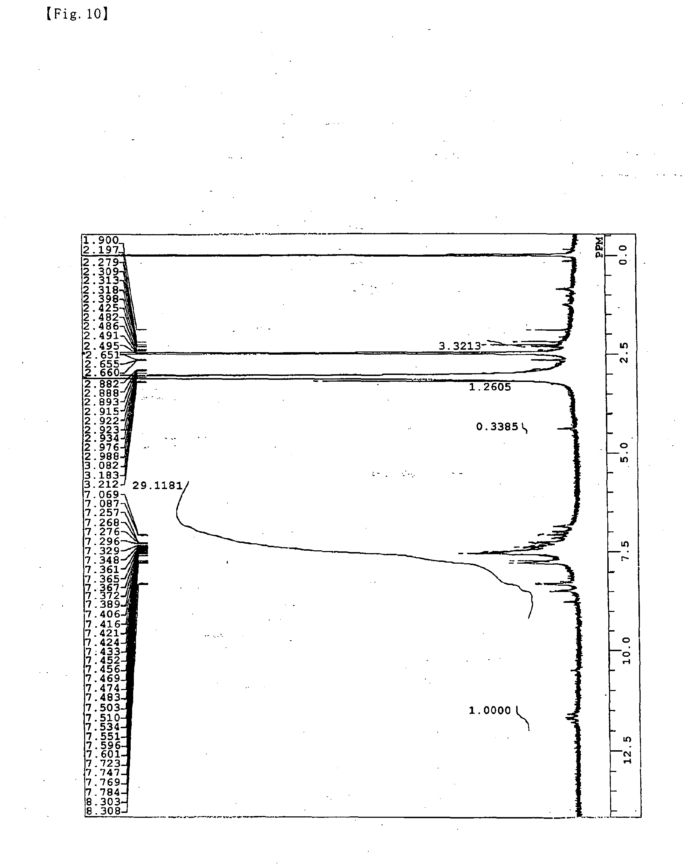

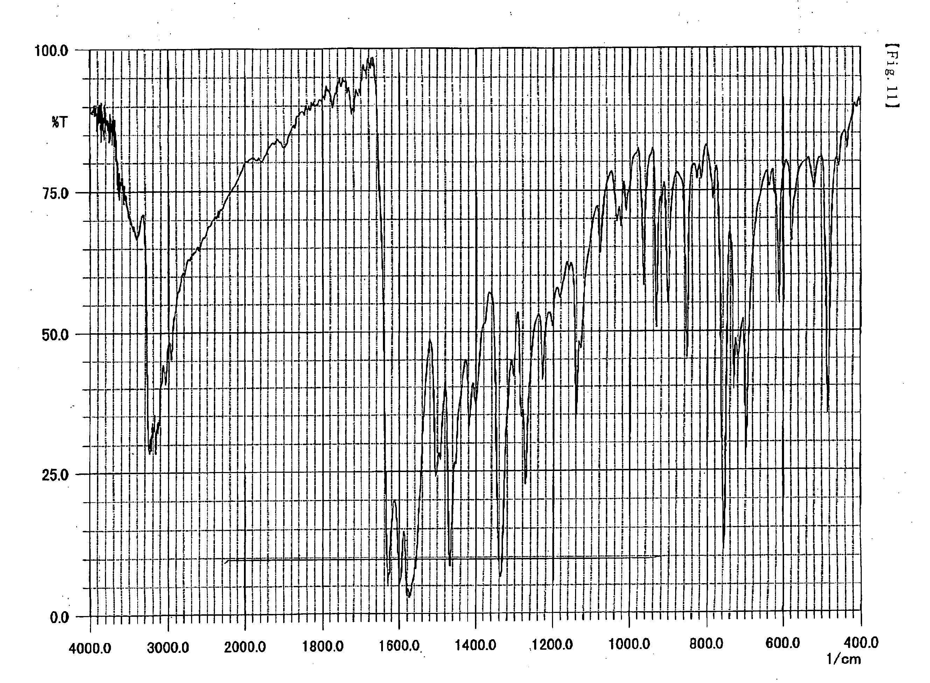

An NMR spectrum chart of the obtained crystals is shown in Figure 10 and an IR spectrum chart thereof in Figure 11.

These dark violet crystals were identified based on these data as the compound having the structure represented by formula (17).

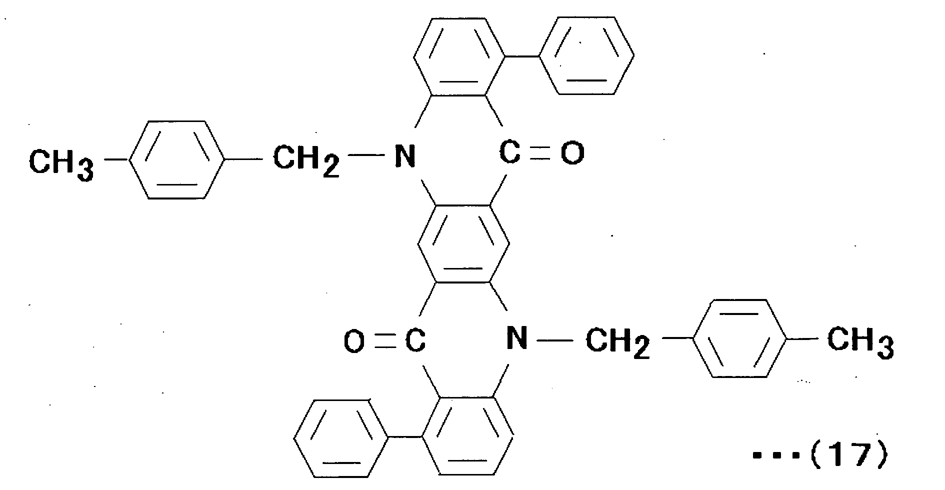

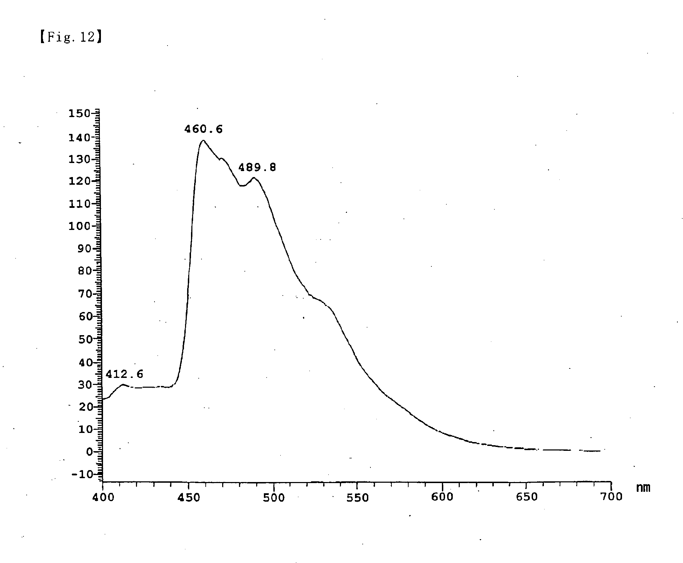

A first sample solution was prepared by dissolving the obtained crystals in toluene so that the concentration of the target compound was 15 mg/L. The first sample solution was loaded in a model F-4500 spectrofluorophotometer, a product by Hitachi, Ltd. , and the fluorescence spectrum of the compound was measured under the following conditions. The measured spectrum is shown in Figure 12.

### Conditions of Measurement

Measuring mode: Wavelength scanning
Exciting wavelength: 365 nm
Wavelength at which the emission of fluorescence started: 400 nm
Wavelength at which the emission of fluorescence ended: 700 nm
Scanning speed: 2400 nm/min.
Slit on the side of excitation: 5.0 nm
Slit on the side of fluorescence emission: 2.5 nm
Photomal voltage: 700 V

As understood from Figure 12, the compound showed fluorescence at wavelengths of 450 nm to 550 nm, which confirmed that the crystals obtained in this example emit white light.

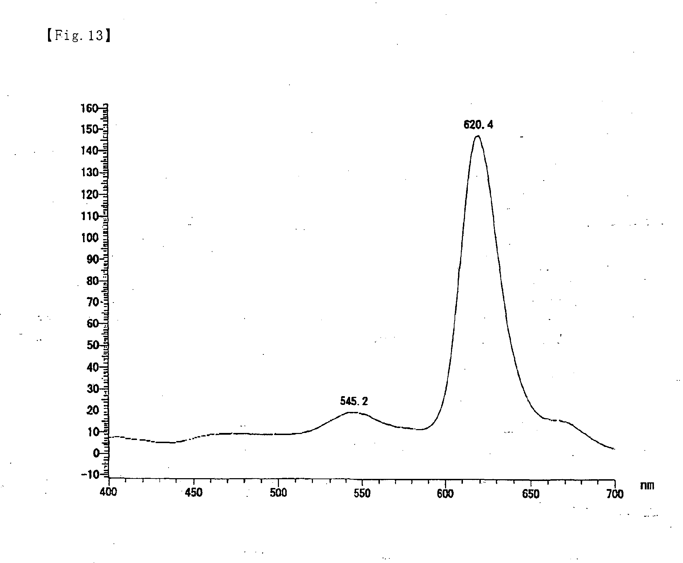

In addition, a second sample solution was prepared by dissolving the obtained crystals in sulfuric acid so that the concentration of the target compound was 100 mg/L: This second sample solution was loaded in a model F-4500 spectrofluorophotometer, a product by Hitachi, Ltd., and the fluorescence spectrum of the compound was measured under the same conditions as that of the first sample was measured. The measured spectrum is shown in Figure 13.

As understood from Figure 13, the compound obtained through the ring-closing reaction in this example showed fluorescence also at wavelengths of 600 nm to 650 nm.

### (Example 2) Synthesis of a White Light-emitting Compound

### <Dehydration>

In a 1L three-necked flask were placed 20.0 g of 1-naphthylamine, 13.0g of the diol represented by formula (13), 250 ml of acetic acid, and 250 ml of ethanol. The flask containing the mixture was placed in a silicone oil bath and the mixture was heated to 115°C. The mixture was stirred for 4 hours at around the temperature and allowed to react. After the termination of the reaction, the product was cooled naturally. The cooled product was filtered with a glass filter and solids were collected. The solids were washed with methanol and petroleum ether in this order. The washed was vacuum dried. 16.0 g of orange crystals were obtained.

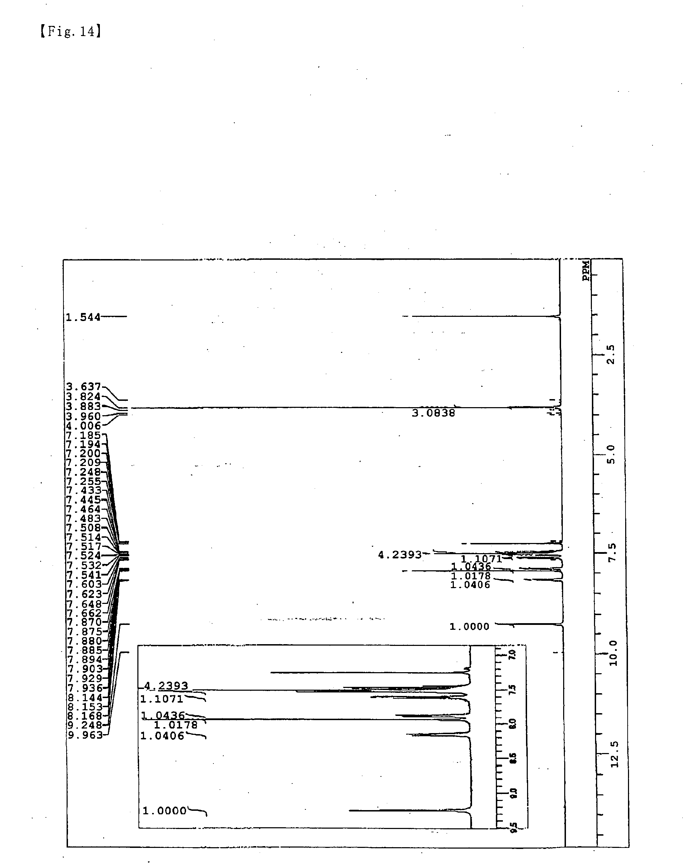

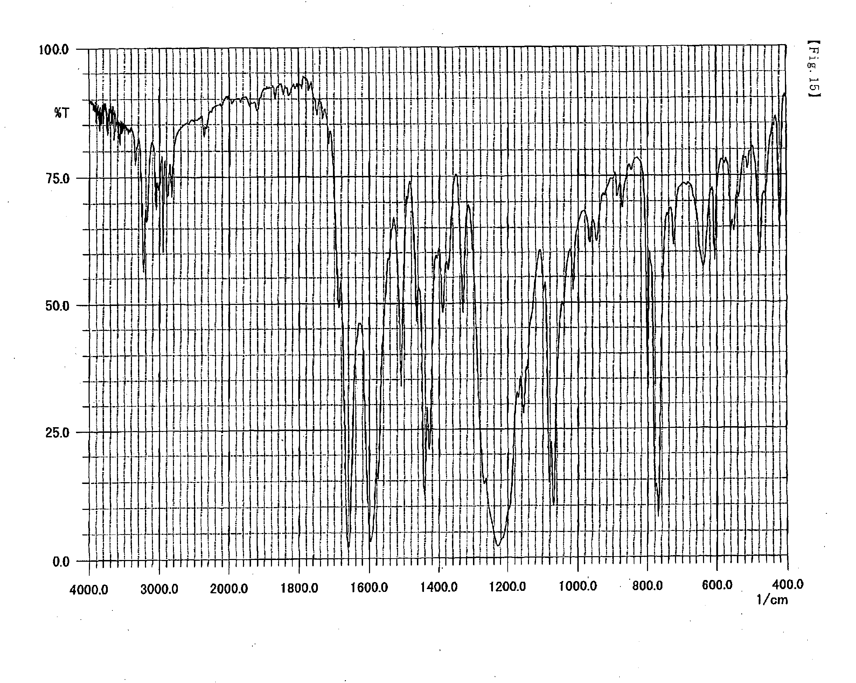

An NMR spectrum chart of the obtained crystals is shown in Figure 14 and an IR spectrum chart thereof in Figure 15.

These orange crystals were identified based on these data as the compound having the structure represented by formula (18).

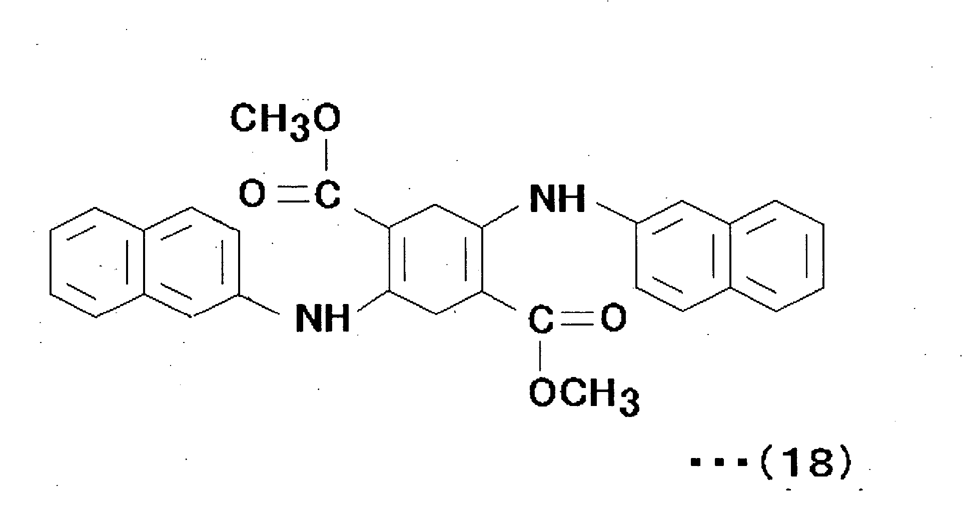

### <Dehydrogenation>

In a 1L three-necked flask were placed 15.0 g of the compound prepared in the step of dehydration and 500 ml of o-dichlorobenzene. A 95% aqueous solution of sulfuric acid was gradually added to the mixture in the flask over 30 minutes with stirring, while the mixture was kept at room temperature. The total amount of the added sulfuric acid solution was 0.5 g. The flask containing the resultant mixture was placed in a silicone oil bath and heated to 160°C. The mixture was stirred for 2 hours at around the temperature and allowed to react. After the termination of the reaction, the product was cooled with ice. The cooled product was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried to 12.1 g of light red crystals.

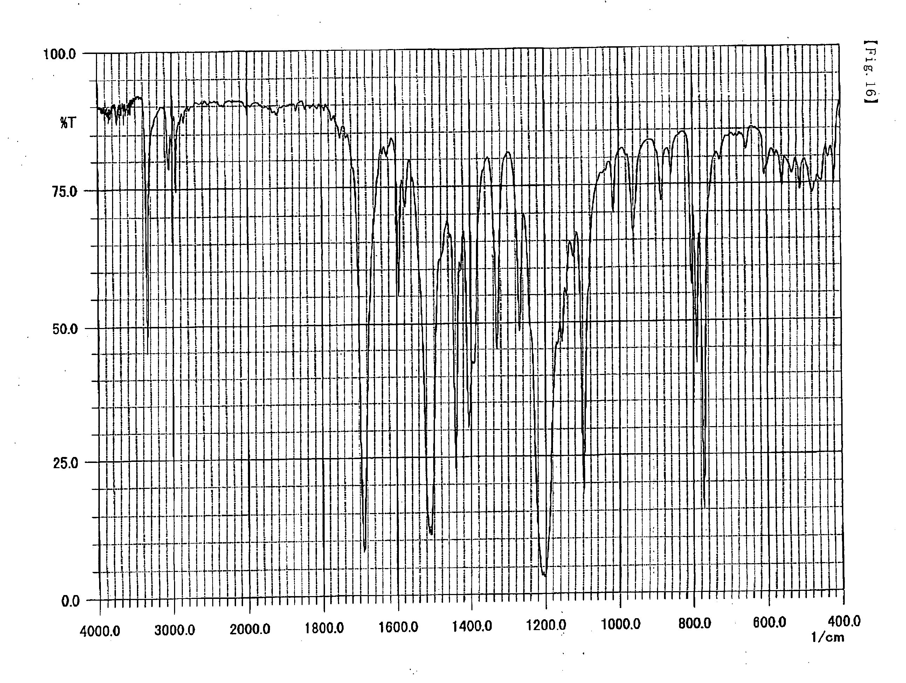

An IR spectrum chart of the obtained crystals is shown in Figure 16.

These light red crystals were identified based on the chart as the compound having the structure represented by formula (19) .

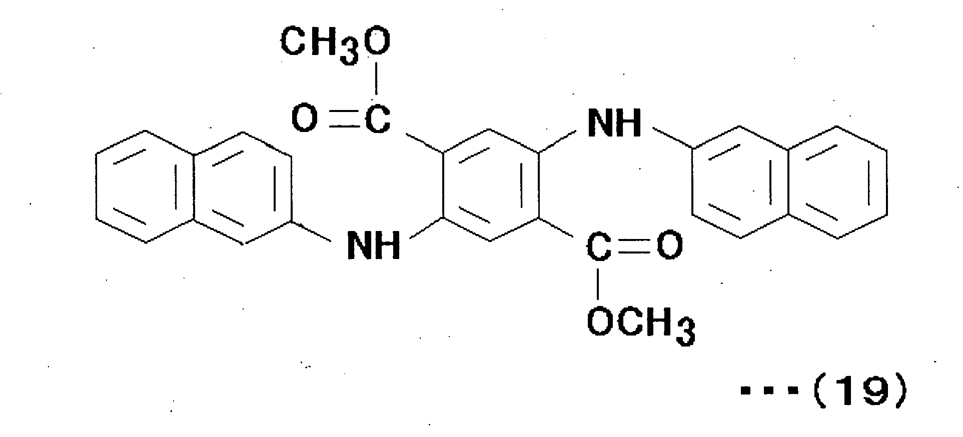

### <Alkylation>

In a 500 ml pressure bottle were placed 10.0 g of the compound prepared in the step of dehydrogenation, 17.7g of α-chloro-p-xylene, and 300 ml of N,N-dimethylformamide. The pressure bottle containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrate was cooled with ice, and then neutralized with sodium hydroxide. The neutralized was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed withmethanol, and then with petroleum ether. The washed solids were vacuum dried to 7.6 g of reddish brown crystals.

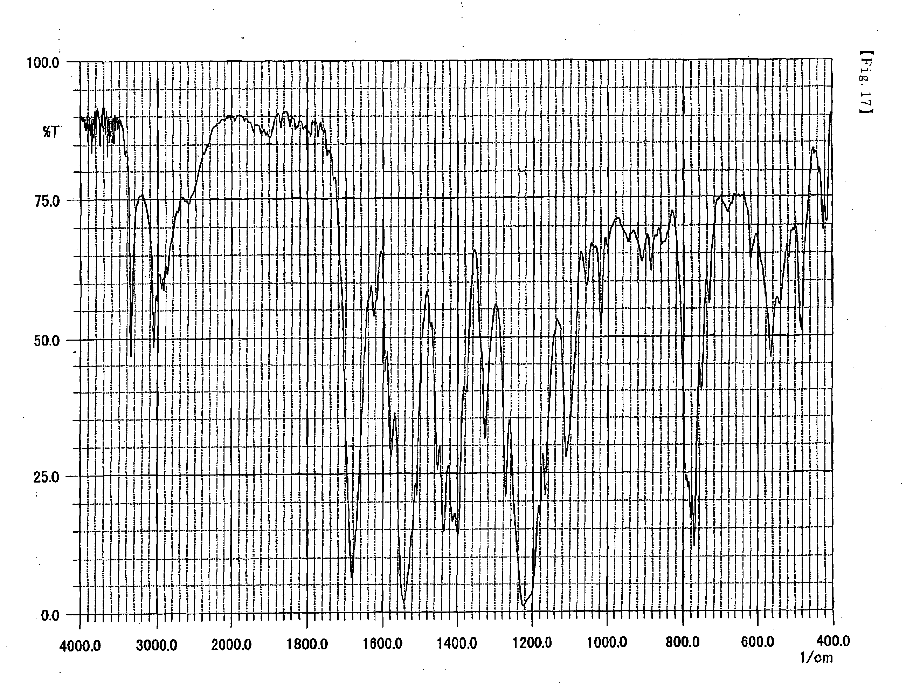

An IR spectrum chart of the obtained crystals is shown in Figure 17.

These reddish brown crystals were identified based on the chart as the compound having the structure represented by formula (20).

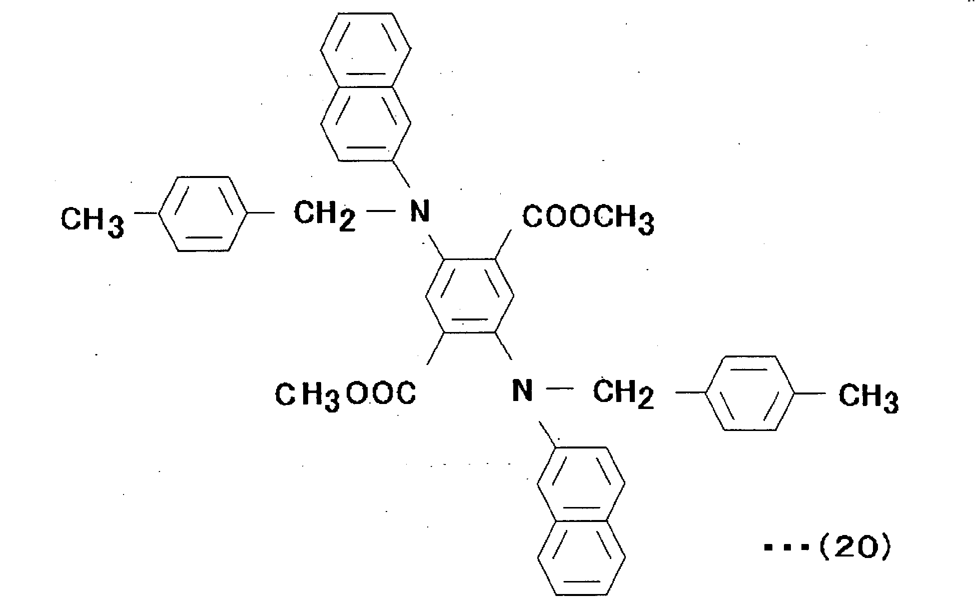

### <Ring Closure>

In a 500 ml three-necked flask were placed 5.0 g of the compound prepared in the step of alkylation, 8.3 g of monohydrated p-toluenesulfonic acid, and 200 ml of o-dichlorobenzene. The flask containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrated was filtered with a glass filter and the solids were collected. The solids were washed with methanol, acetone, and petroleum ether in this order. The washed was vacuum dried. 3.9 g of purplish red crystals were obtained.

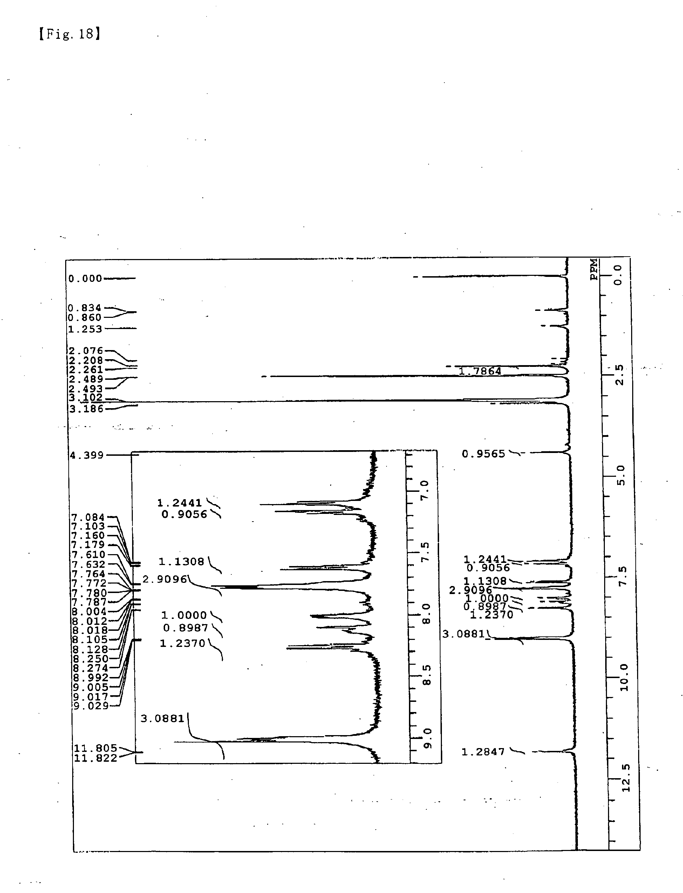

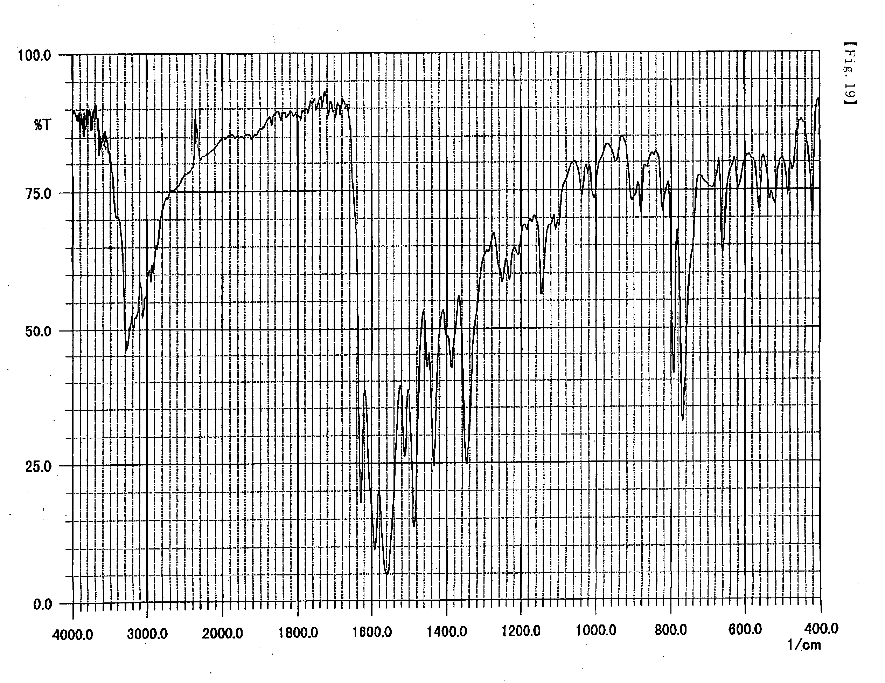

An NMR spectrum chart of, the obtained crystals is shown in Figure 18 and an IR spectrum chart thereof in Figure 19.

These purplish red crystals were identified based on these data as the compound having the structure represented by formula (21).

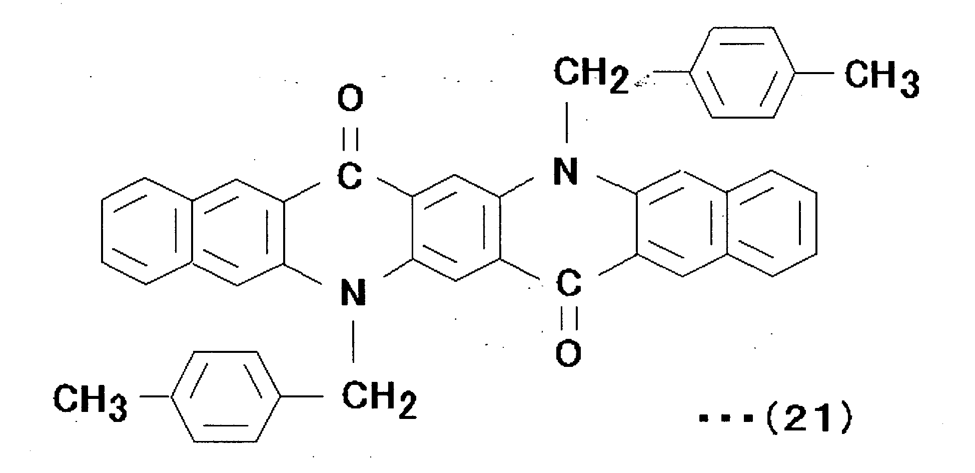

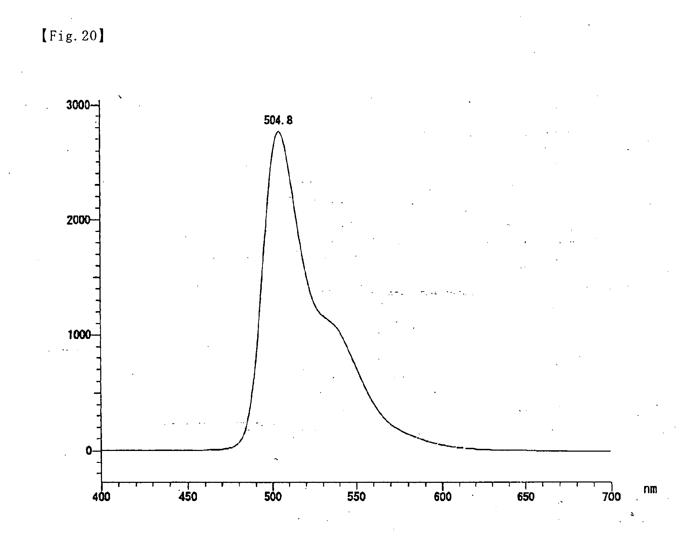

A sample solution was prepared by dissolving the obtained crystals in acetone so that the concentration of the target compound was 15 mg/L. This sample solution was loaded in a model F-4500 spectrofluorophotometer, a product by Hitachi, Ltd. , and the fluorescence spectrum of the compound was measured under the same conditions as those of the samples in Example 1 were measured. The measured spectrum is shown in Figure 20.

As understood from Figure 20, the compound showed fluorescence at wavelengths of 480 nm to 600 nm, which confirmed that the crystals obtained in this example emit white light.

### (Example 3) Synthesis of a White Light-emitting Compound

### <Dehydration>

In a 1L three-necked flask were placed 20. 0 g of 2-aminoanthracene, 10.8 g of the diol represented by formula (13), 250 ml of acetic acid, and 250 ml of ethanol. The flask containing the mixture was placed in a silicone oil bath and the mixture was heated to 115°C. The mixture was stirred for 4 hours at around the temperature and allowed to react. After the termination of the reaction, the product was cooled naturally. The cooled product was filtered with a glass filter and solids were collected. The solids were washed with methanol and petroleum ether in this order. The washed was vacuum dried. 17.3 g of orange crystals were obtained.

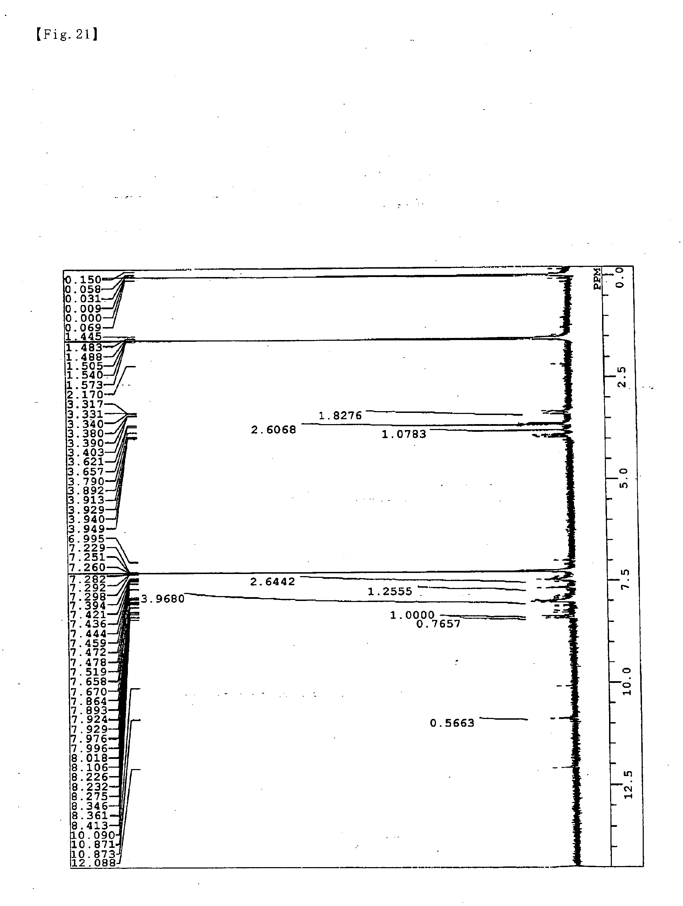

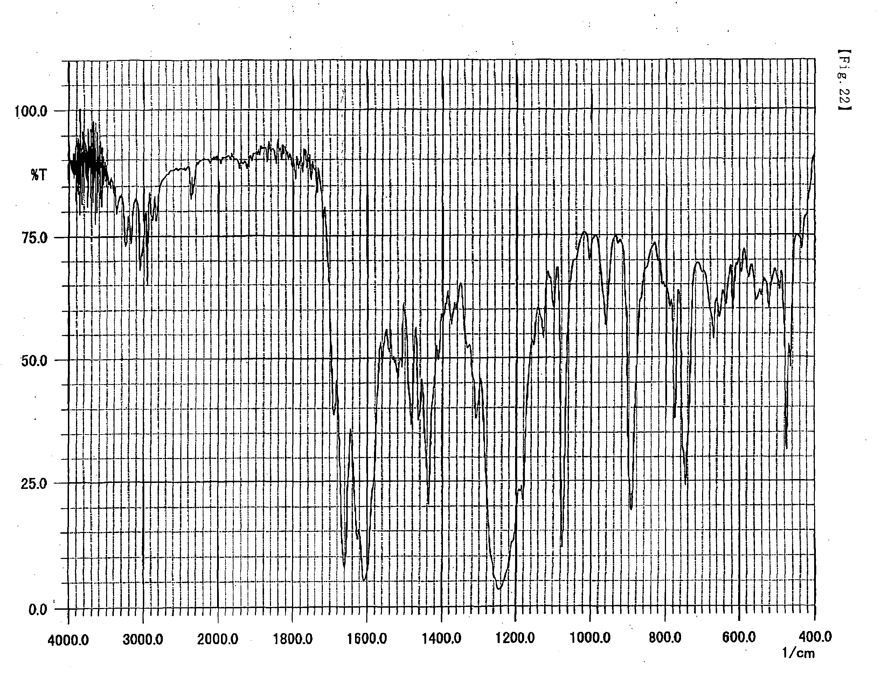

An NMR spectrum chart of the obtained crystals is shown in Figure 21 and an IR spectrum chart thereof in Figure 22.

These orange crystals were identified based on these data as the compound having the structure represented by formula (22) .

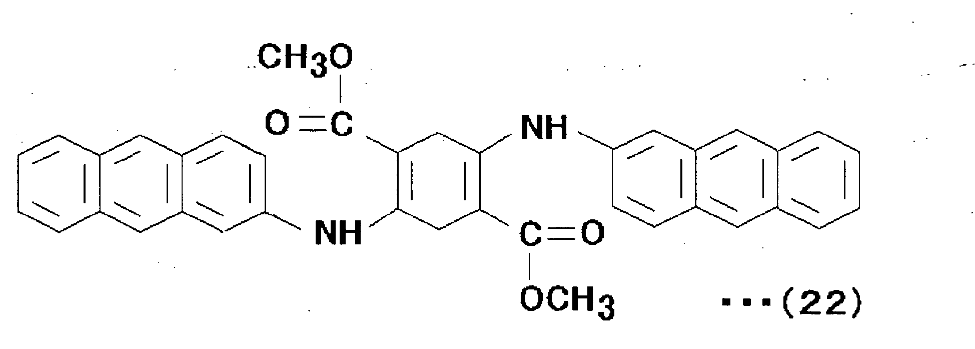

### <Dehydrogenation>

In a 1L three-necked flask were placed 15.0 g of the compound prepared in the step of dehydration and 500 ml of o-dichlorobenzene. A 95% aqueous solution of sulfuric acid was gradually added to the mixture in the flask over 30 minutes with stirring, while the mixture was kept at room temperature. The total amount of the added sulfuric acid solution was 0.3 g. The flask containing the resultant mixture was placed in a silicone oil bath and heated to 160 °C . The mixture was stirred for 2 hours at around the temperature and allowed to react. After the termination of the reaction, the product was cooled with ice. The cooled product was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried. 11.6 g of red crystals were obtained.

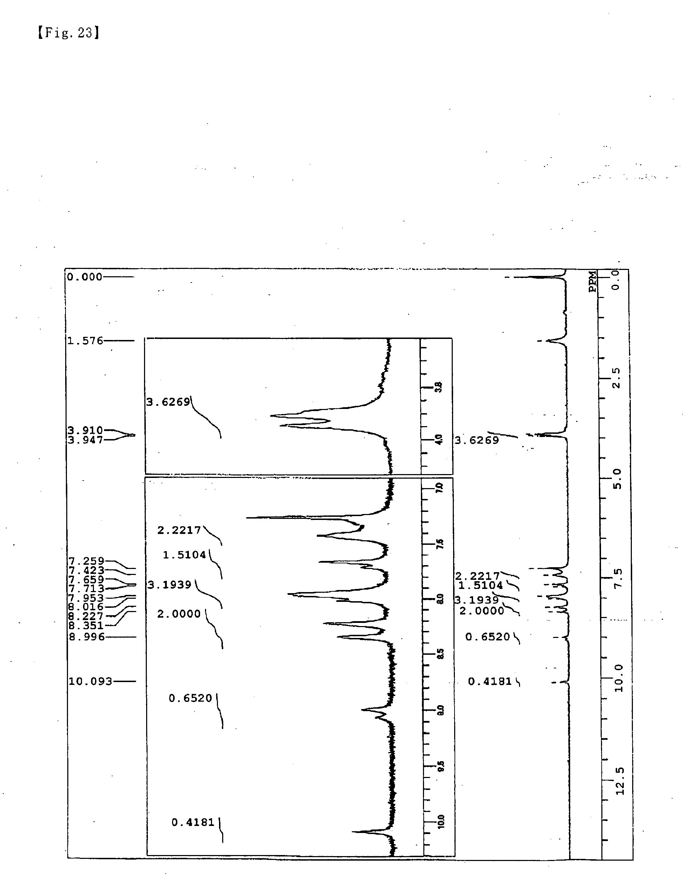

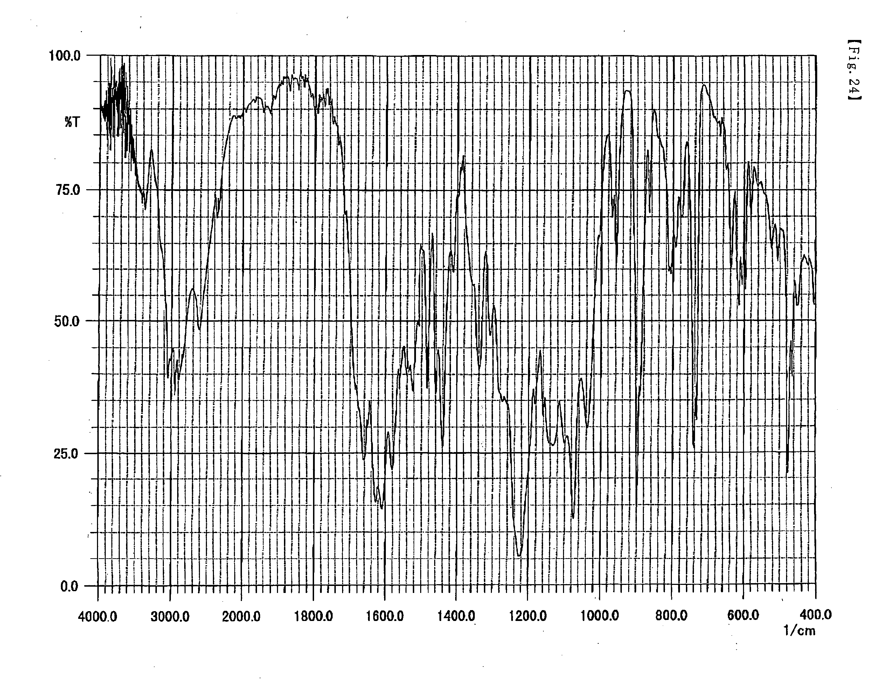

An NMR spectrum chart of the obtained crystals is shown in Figure 23 and an IR spectrum chart thereof in Figure 24.

These red crystals were identified based on the data as the compound having the structure represented by formula (23) .

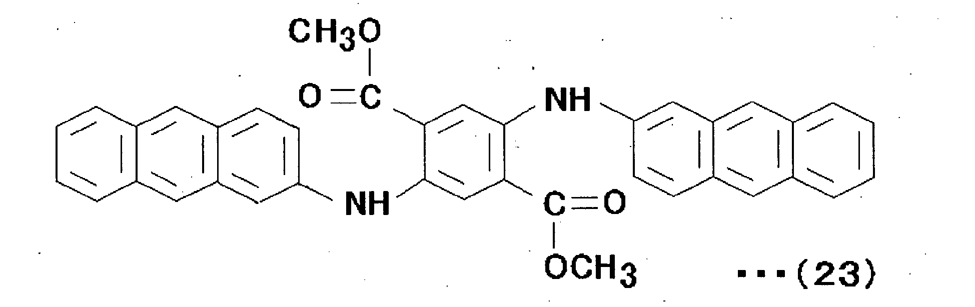

### <Alkylation>

In a 500 ml pressure bottle were placed 5.0 g of the compound prepared in the step of dehydrogenation, 7.3g of α-chloro-p-xylene, and 300 ml of N,N-dimethylformamide. The pressure bottle containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrate was cooled with ice, and then neutralized with sodium hydroxide. The neutralized was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed withmethanol, and then with petroleum ether . The washed solids were vacuum dried. 2.8 g of reddish brown crystals were obtained.

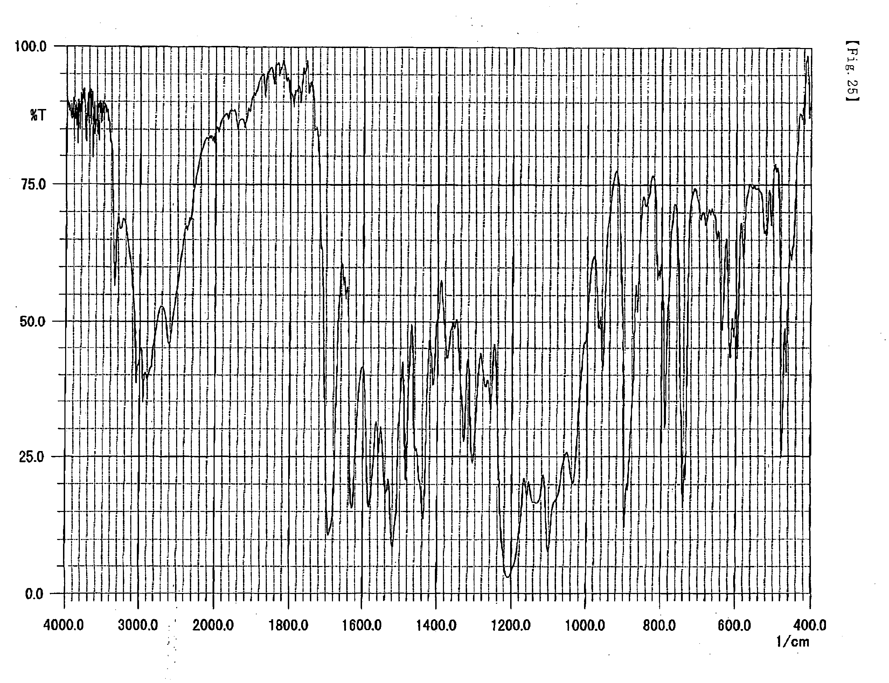

An IR spectrum chart of the obtained crystals is shown in Figure 25.

These reddish brown crystals were identified based on the chart as the compound having the structure represented by formula (24).

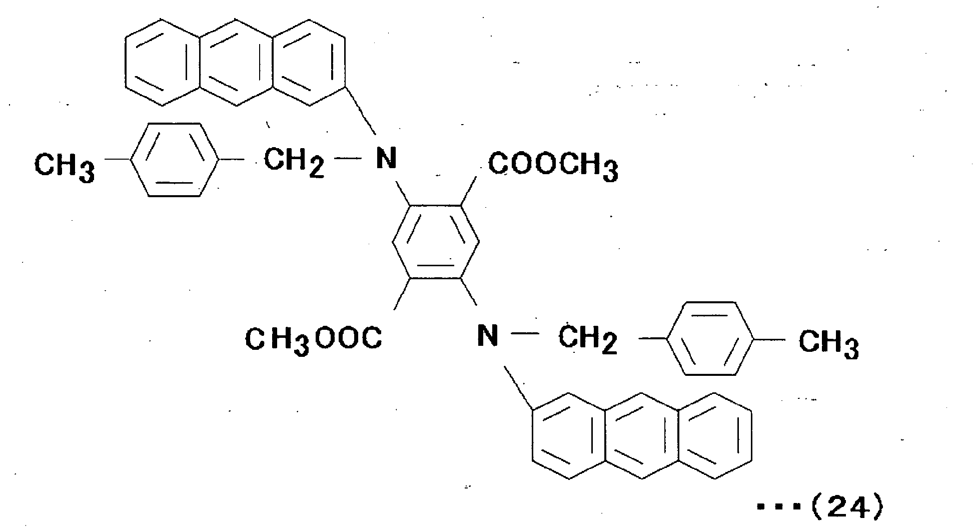

### <Ring Closure>

In a 500 ml three-necked flask were placed 2.5 g of the compound prepared in the step of alkylation, 3.6 g of monohydrated p-toluenesulfonic acid, and 200 ml of o-dichlorobenzene. The flask containing the reaction mixture was placed in a silicone oil bath and the mixture was heated to 160 °C . The mixture was stirred for 20 hours at around the temperature and allowed to react. After the termination of the reaction, the product was cooled naturally. The cooled product was concentrated using an evaporator. The concentrated was filtered with a glass filter and solids were collected. The solids were washed with methanol, acetone, and petroleum ether in this order. The washed was vacuum dried. 2.0 g of dark violet crystals were obtained.

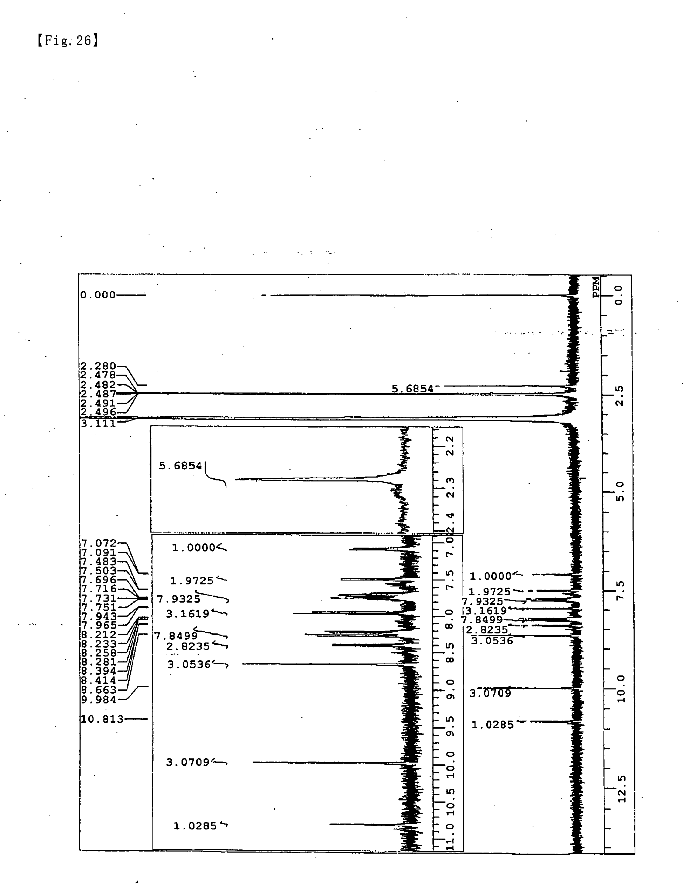

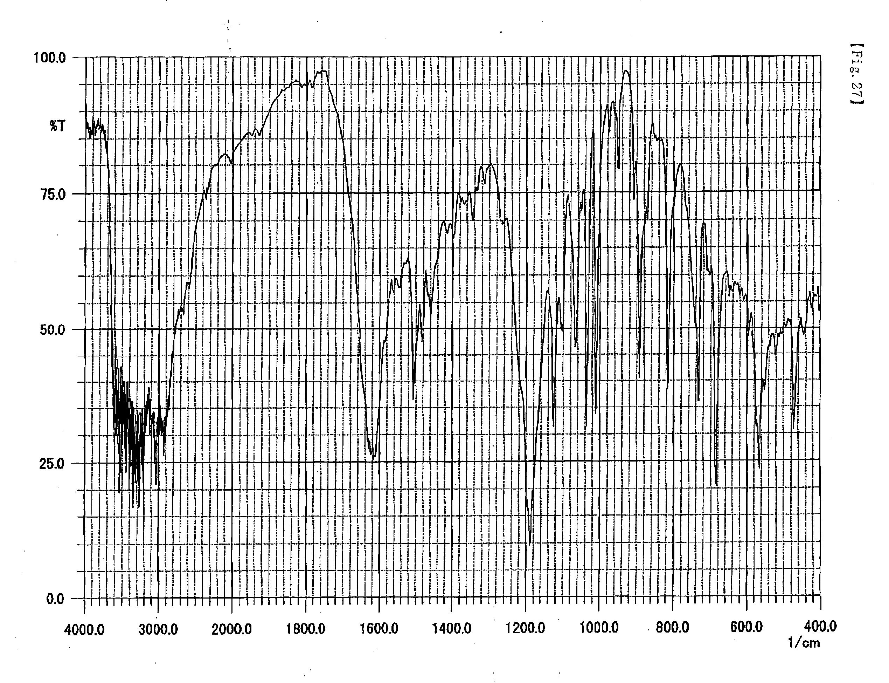

An NMR spectrum chart of the obtained crystals is shown in Figure 26 and an IR spectrum chart thereof in Figure 27.

These dark violet crystals were identified based on these data as the compound having the structure represented by formula (25).

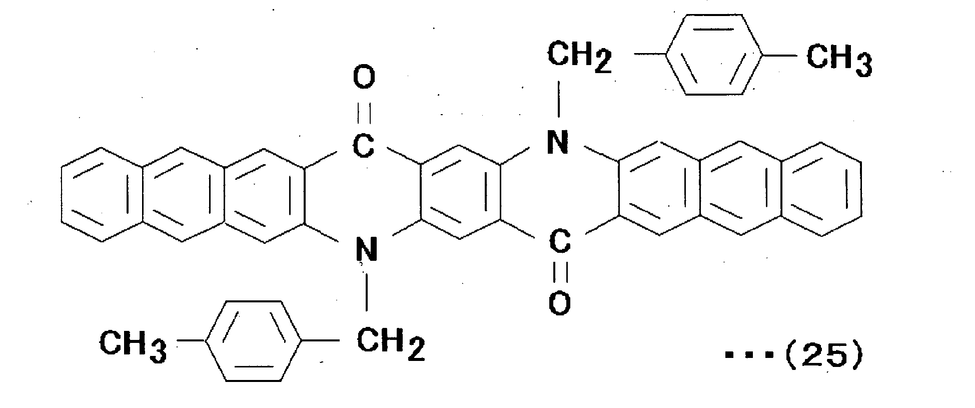

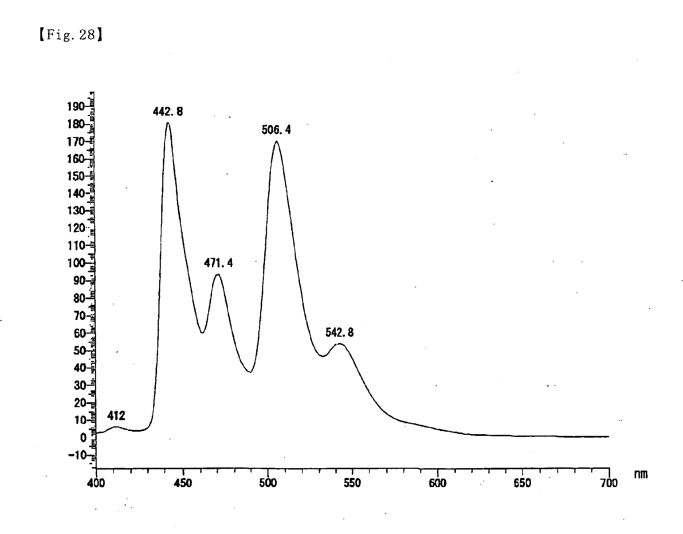

A sample solution was prepared by dissolving the obtained crystals in xylene so that the concentration of the target compound was 15 mg/L. This sample solution was loaded in a model F-4500 spectrofluorophotometer, a product by Hitachi, Ltd., and the fluorescence spectrum of the compound was measured under the same conditions as those of the samples in Example 1 were measured. The measured spectrum is shown in Figure 28.

As understood from Figure 28, the compound showed fluorescence at wavelengths of 430 nm to 600 nm, which confirmed that the crystals obtained in this example emit white light.

### (Example 4) Synthesis of a White Light-emitting Compound

### <Dehydration>

In a 1L three-necked flask were placed 25.0 g of 3-amino-4-methoxybiphenyl, 13.0 g of the diol represented by formula (13), 250 ml of acetic acid, and 250 ml of ethanol. The flask containing the mixture was placed in a silicone oil bath and the mixture was heated to 115°C. The mixture was stirred for 4 hours at around the temperature and allowed to react. After the termination of the reaction, the product was cooled naturally. The cooled product was filtered with a glass filter and solids were collected. The solids were washed with methanol, ethyl acetate, and petroleum ether in this order. The washed was vacuum dried. 23.7 g of reddish pink crystals were obtained.

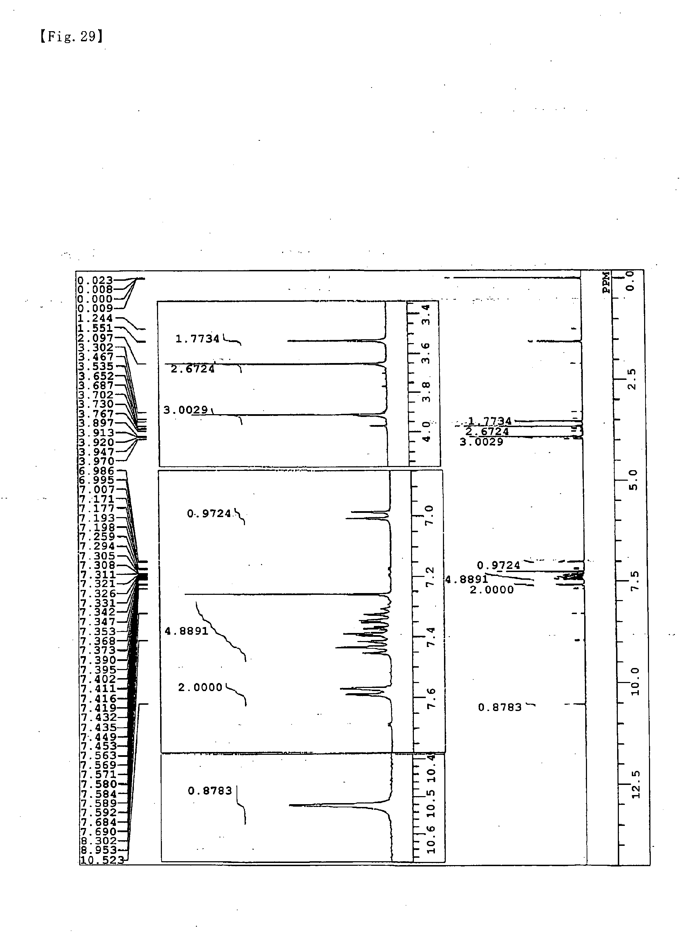

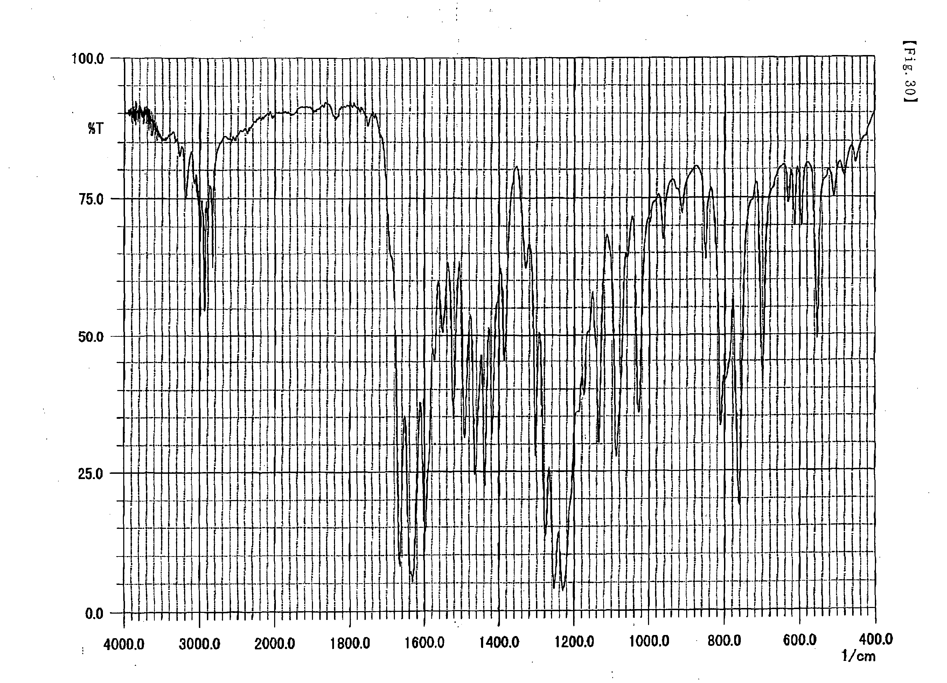

An NMR spectrum chart of the obtained crystals is shown in Figure 29 and an IR spectrum chart thereof in Figure 30.

These reddish pink crystals were identified based on these data as the compound having the structure represented by formula (26).

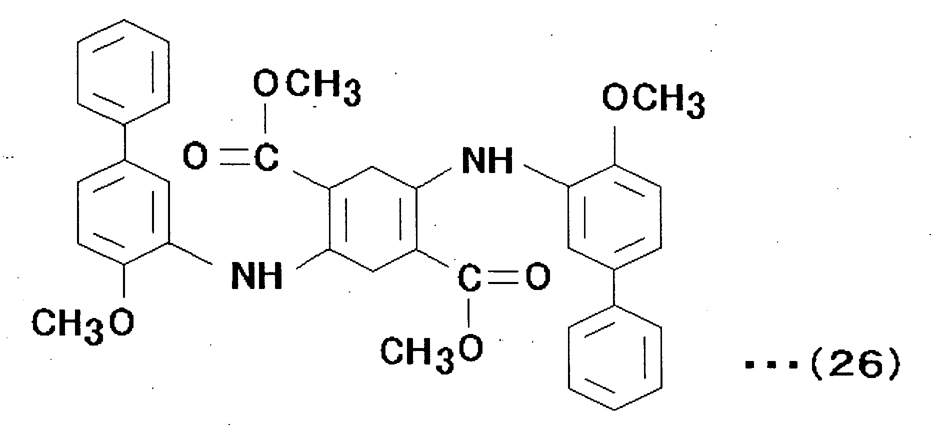

### <Dehydrogenation>

In a 1L three-necked flask were placed 10.0 g of the compound prepared in the step of dehydration and 500 ml of o-dichlorobenzene. A 95% aqueous solution of sulfuric acid was gradually added to the mixture in the flask over 30 minutes with stirring, while the mixture was kept at room temperature. The total amount of the added sulfuric acid solution was 0.3 g. The flask containing the resultant mixture was placed in a silicone oil bath and heated to 160°C. The mixture was stirred for 2 hours at around the temperature and allowed to react. After the termination of the reaction, the product was cooled with ice. The cooled product was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried. 8.5 g of deep red crystals were obtained.

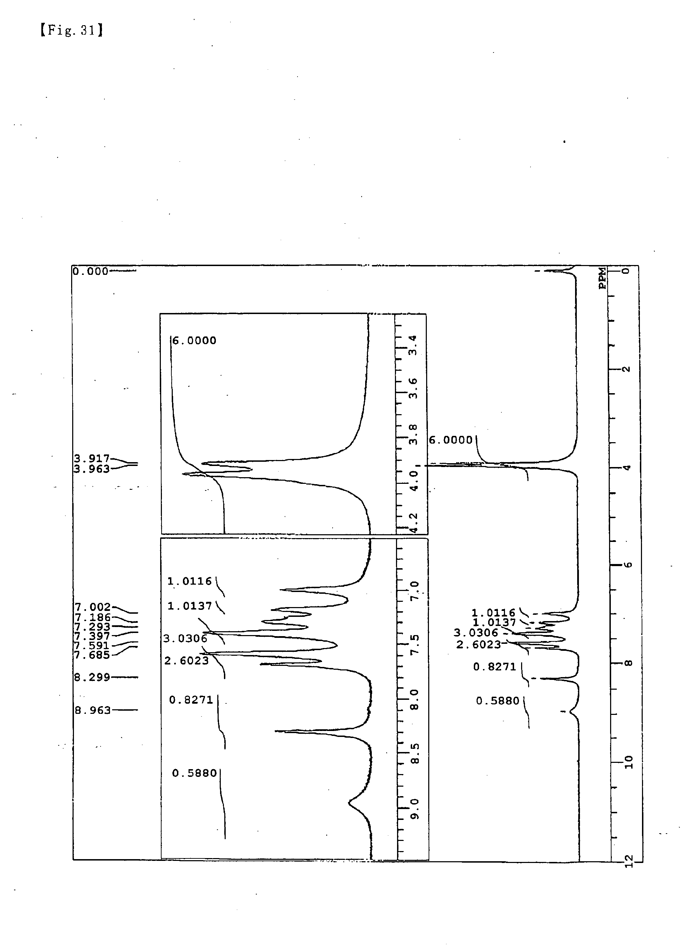

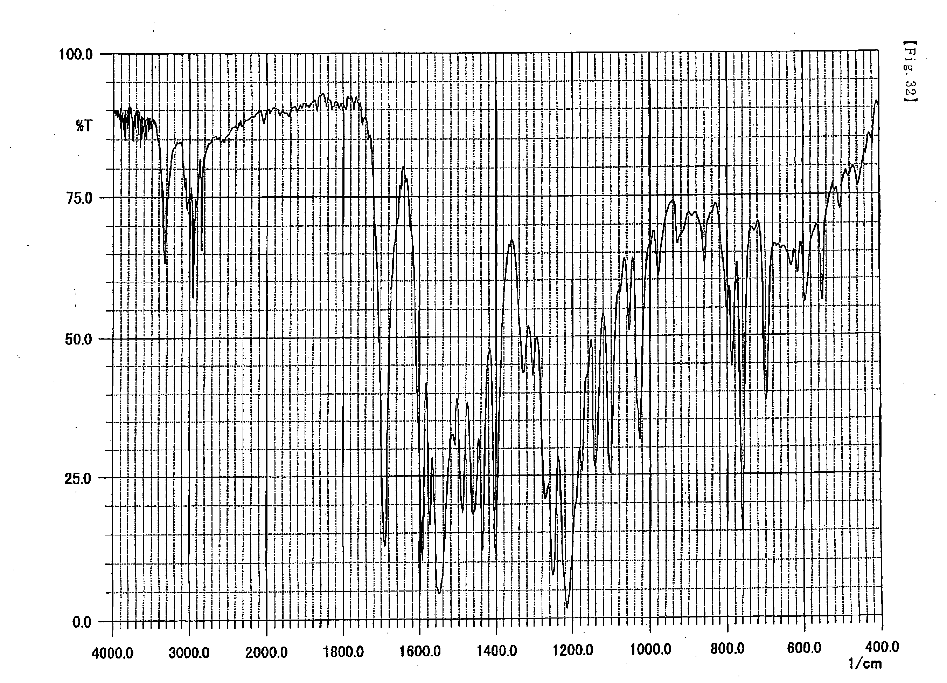

An NMR spectrum chart of the obtained crystals is shown in Figure 31 and an IR spectrum chart thereof in Figure 32.

These deep red crystals were identified based on the data as the compound having the structure represented by formula (27).

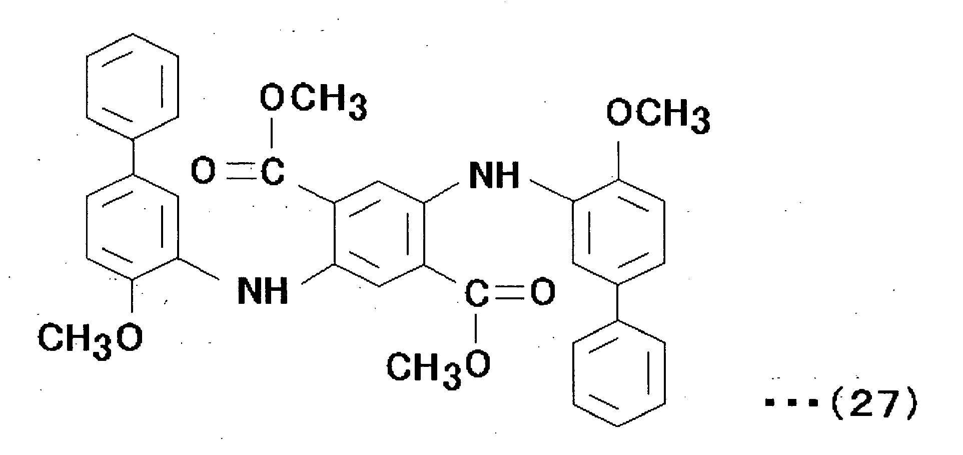

### <Alkylation>

In a 500 ml pressure bottle were placed 8.0 g of the compound prepared in the step of dehydrogenation, 11.5 g of α-chloro-p-xylene, and 300 ml of N,N-dimethylformamide. The pressure bottle containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrate was cooled with ice, and then neutralized with sodium hydroxide. The neutralized was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed withmethanol, and then with petroleum ether . The washed solids were vacuum dried. 6.9 g of purplish brown crystals were obtained.

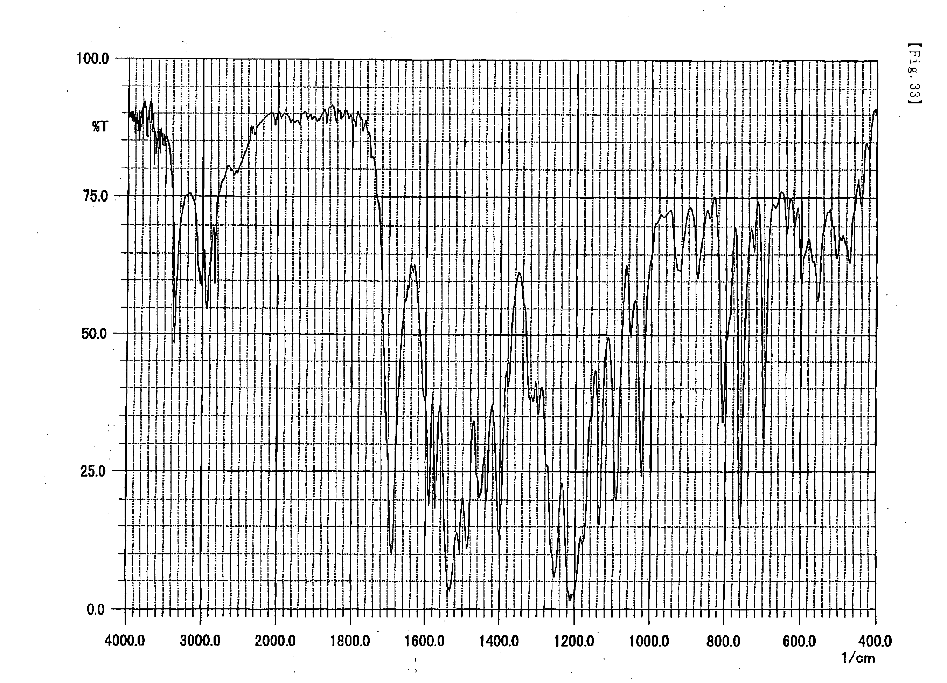

An IR spectrum chart of the obtained crystals is shown in Figure 33.

These purplish brown crystals were identified based on the chart as the compound having the structure represented by formula (28).

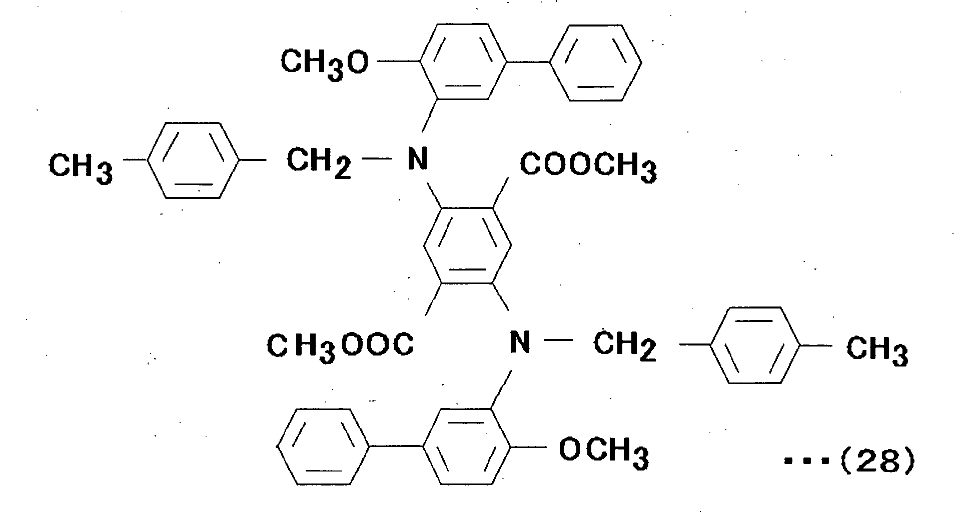

### <Ring Closure>

In a 500 ml three-necked flask were placed 5.0 g of the compound prepared in the step of alkylation, 9.3 g of monohydrated p-toluenesulfonic acid, and 200 ml of o-dichlorobenzene. The flask containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrated was filtered with a glass filter and solids were collected. The solids were washed with methanol, acetone, and petroleum ether in this order. The washed was vacuum dried. 2.0 g of dark violet crystals were obtained.

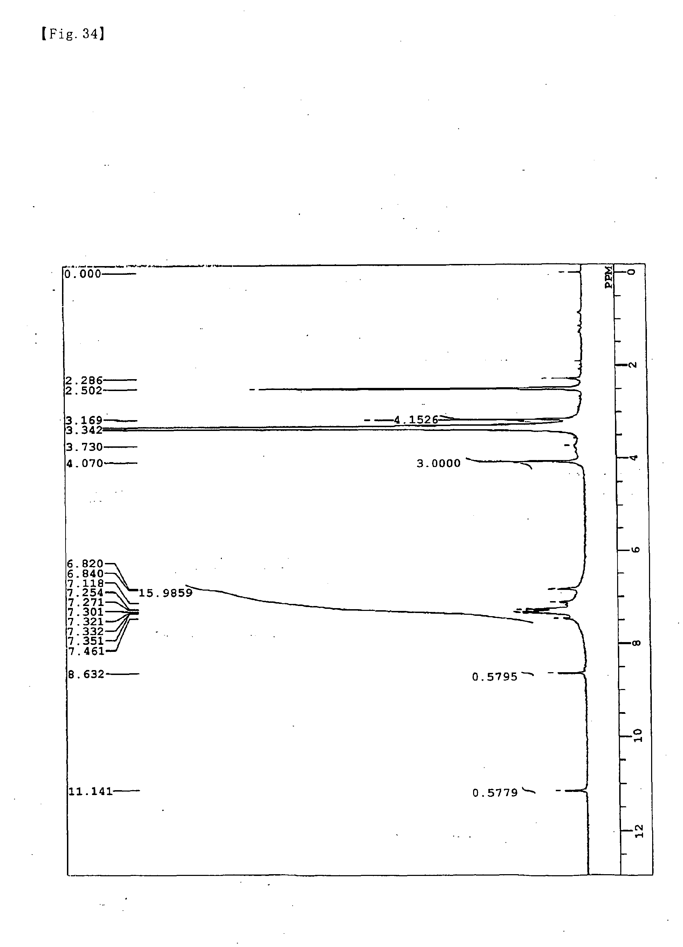

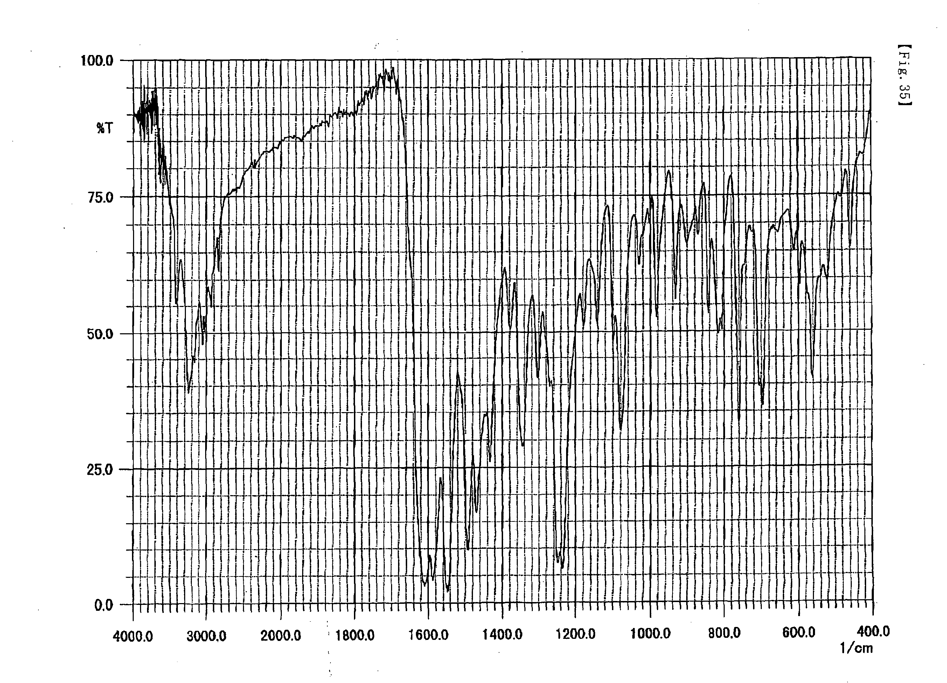

An NMR spectrum chart of the obtained crystals is shown in Figure 34 and an IR spectrum chart thereof in Figure 35.

These dark violet crystals were identified based on these data as the compound having the structure represented by formula (29).

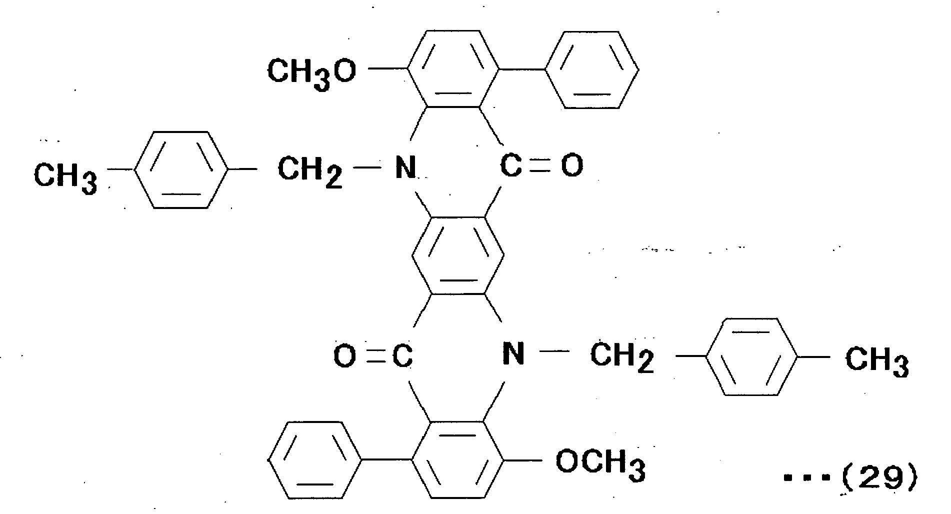

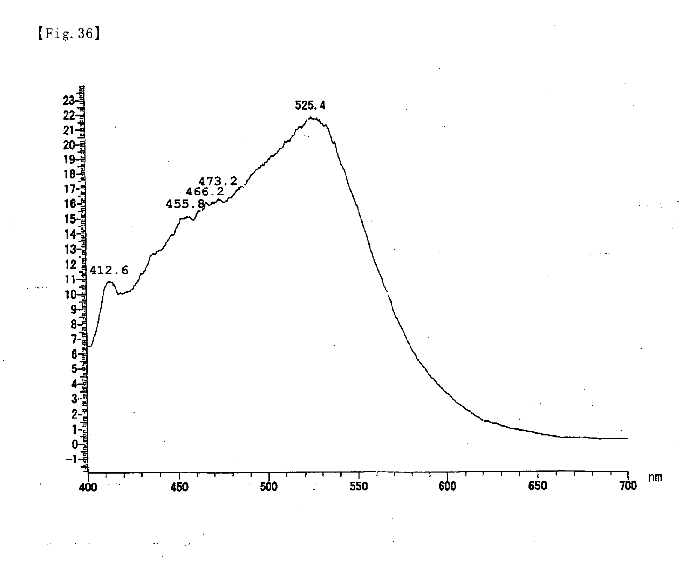

A sample solution was prepared by dissolving the obtained crystals in xylene so that the concentration of the target compound was 15 mg/L. This sample solution was loaded in a model F-4500 spectrofluorophotometer, a product by Hitachi, Ltd., and the fluorescence spectrum of the compound was measured under the same conditions as those of the samples in Example 1 were measured. The measured spectrum is shown in Figure 36.

As understood from Figure 36, the compound showed fluorescence at wavelengths of 430 nm to 600 nm, which confirmed that the crystals obtained in this example emit white light.

### (Example 5) Synthesis of a White Light-emitting Compound

### <Dehydration>

In a 1L three-necked flask were placed 25.0 g of 2-tert-butylaniline, 15.5 g of the diol represented by formula (13), 250 ml of acetic acid, and 250 ml of ethanol. The flask containing the mixture was placed in a silicone oil bath and the mixture was heated to 115°C. The mixture was stirred for 4 hours at around the temperature and allowed to react. After the termination of the reaction, the product was cooled naturally. The cooled product was filtered with a glass filter and solids were collected. The solids were washed with methanol, ethyl acetate, and petroleum ether in this order. The washed was vacuum dried. 28.0 g-of orange crystals were obtained.

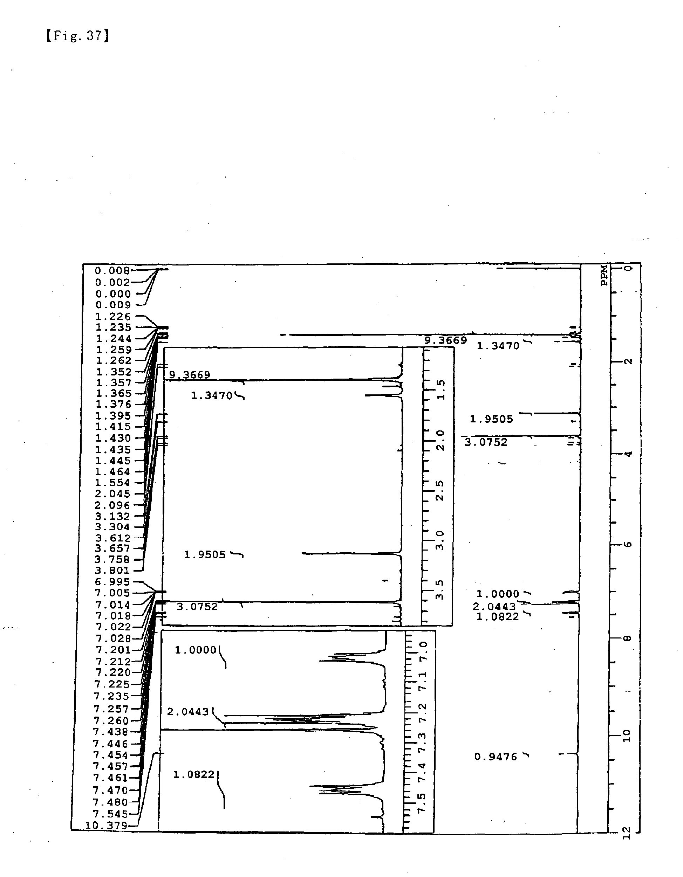

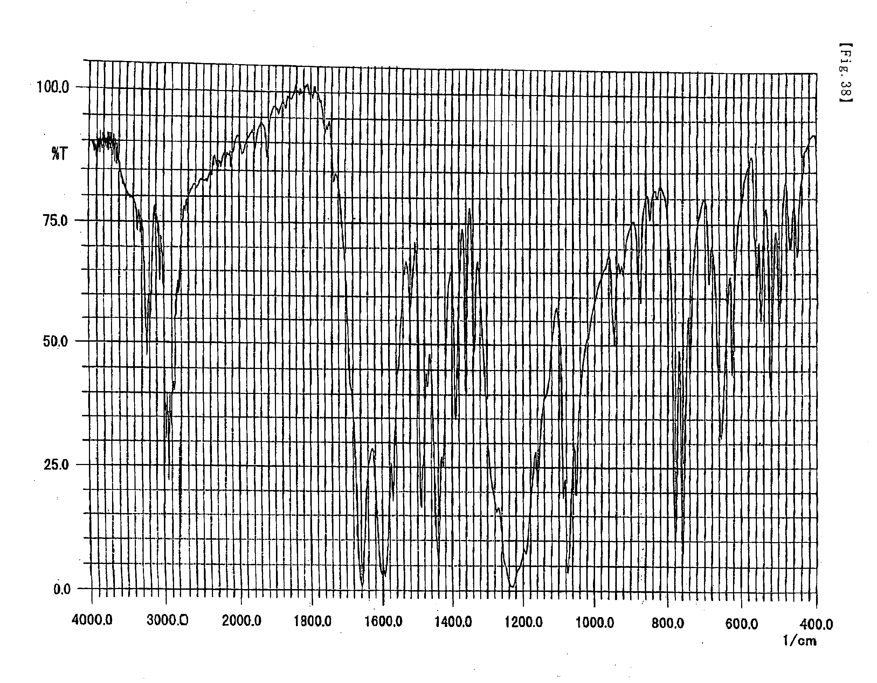

An NMR spectrum chart of the obtained crystals is shown in Figure 37 and an IR spectrum chart thereof in Figure 38.

These orange crystals were identified based on these data as the compound having the structure represented by formula (30) .

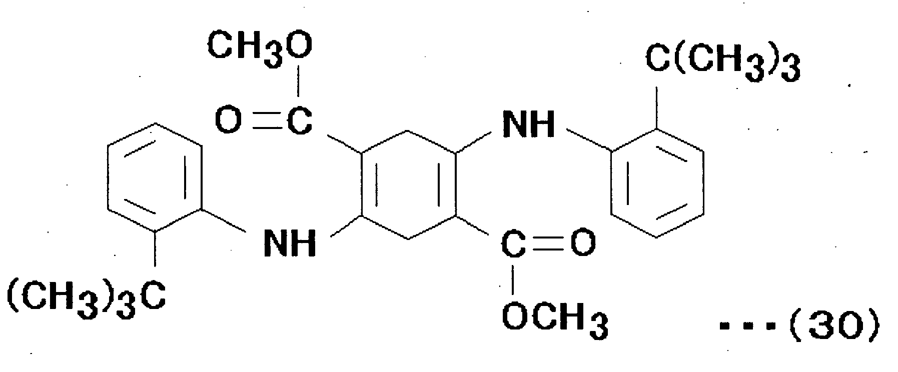

### <Dehydrogenation>

In a 1L three-necked flask were placed 20.0 g of the compound prepared in the step of dehydration and 500 ml of o-dichlorobenzene. A 95% aqueous solution of sulfuric acid was gradually added to the mixture in the flask over 30 minutes with stirring, while the mixture was kept at room temperature. The total amount of the added sulfuric acid solution was 0.4 g. The flask containing the resultant mixture was placed in a silicone oil bath and heated to 160 °C. The mixture was stirred for 2 hours at around the temperature and allowed to react. After the termination of the reaction, the product was cooled with ice. The cooled product was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried. 12.6 g of deep red crystals were obtained.

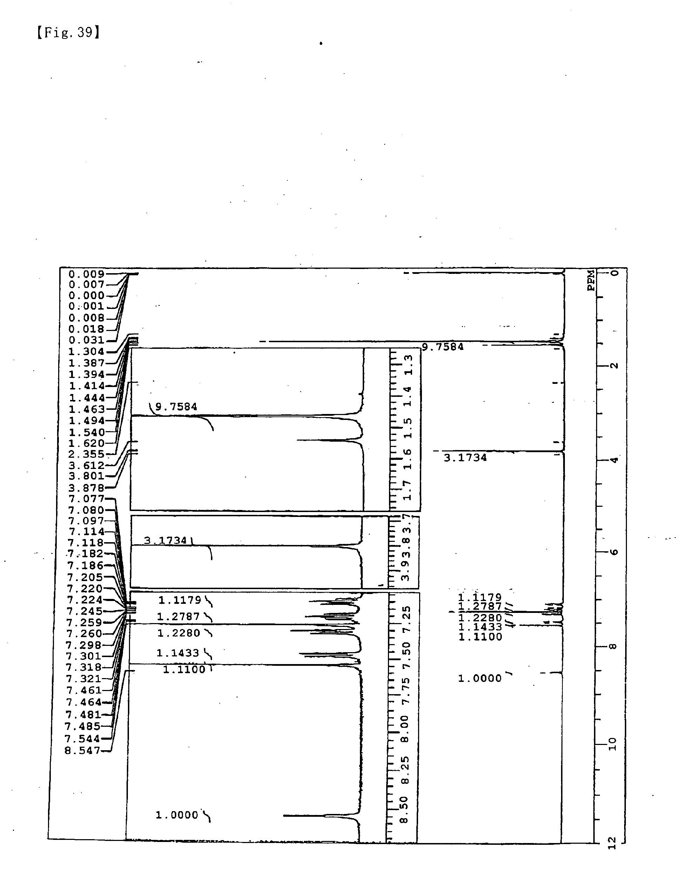

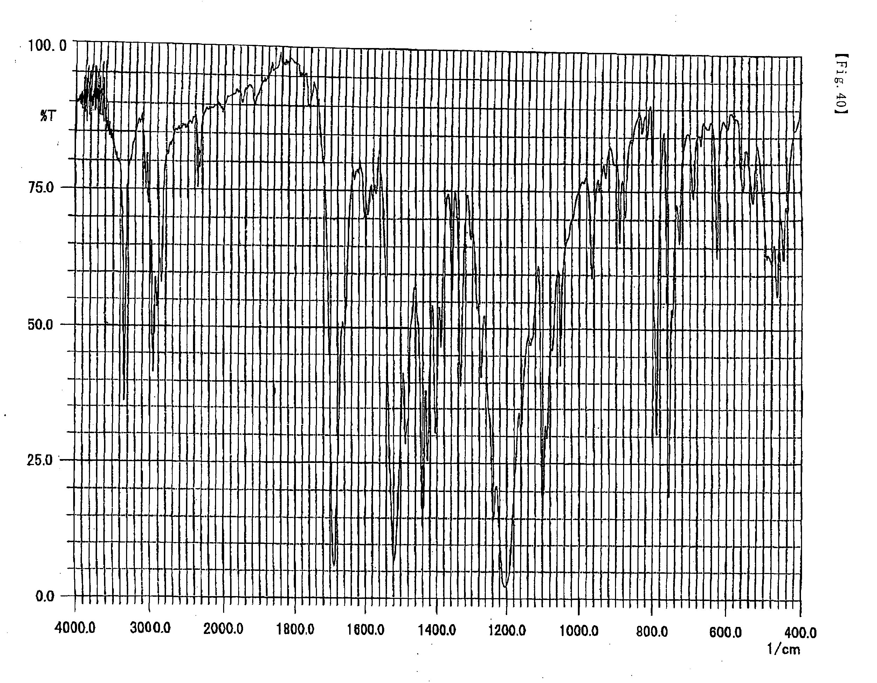

An NMR spectrum chart of the obtained crystals is shown in Figure 39 and an IR spectrum chart thereof in Figure 40.

These deep red crystals were identified based on the data as the compound having the structure represented by formula (31) .

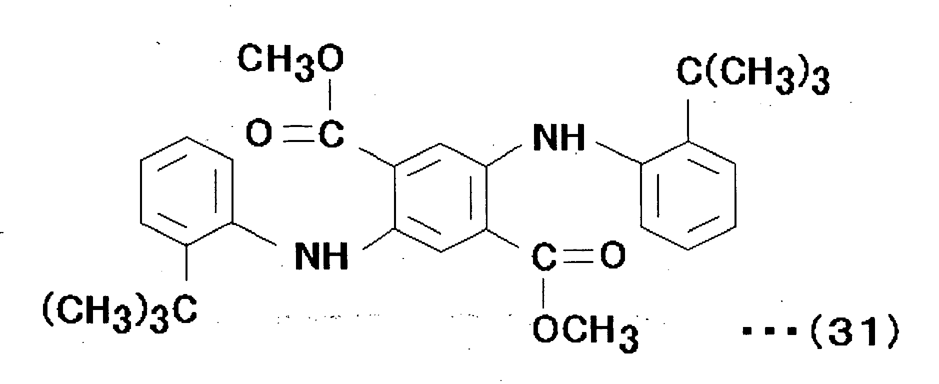

### <Alkylation>

In a 500 ml pressure bottle were placed 5.0 g of the compound prepared in the step of dehydrogenation, 8.6 g of α-chloro-p-xylene, and 300 ml of N,N-dimethylformamide. The pressure bottle containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrate was cooled with ice, and then neutralized with sodium hydroxide. The neutralized was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried. 2.4 g of brown crystals were obtained.

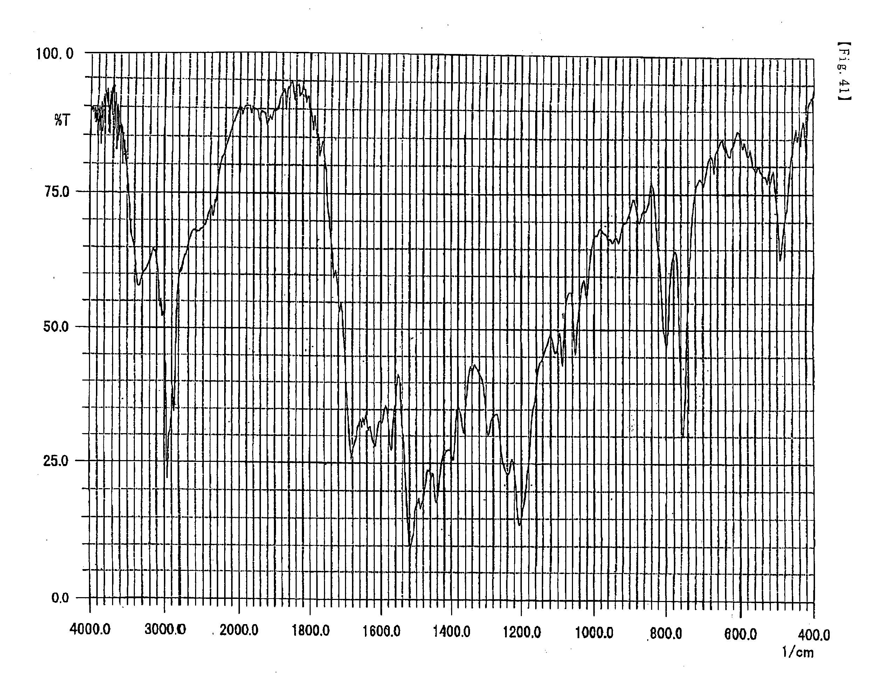

An IR spectrum chart of the obtained crystals is shown in Figure 41.

These brown crystals were identified based on the chart as the compound having the structure represented by formula (32).

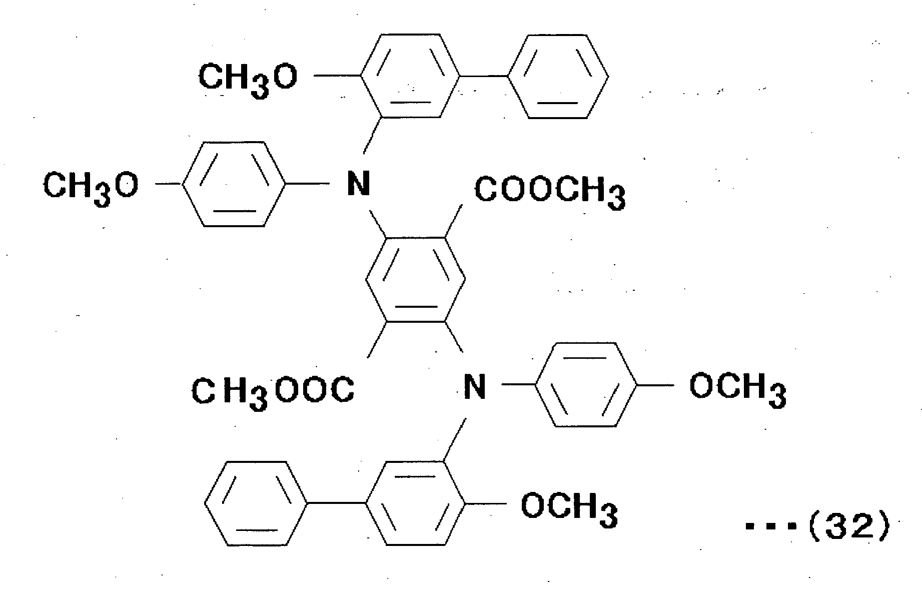

### <Ring-Closure>

In a 500 ml three-necked flask were placed 2.0 g of the compound prepared in the step of alkylation, 3.3 g of monohydrated p-toluenesulfonic acid, and 200 ml of o-dichlorobenzene. The flask containing the reaction mixture was placed in a silicone oil bath and the mixture was heated to 160°C. The mixture was stirred for 20 hours at around the temperature and allowed to react. After the termination of the reaction, the product was cooled naturally. The cooled product was concentrated using an evaporator. The concentrated was filtered with a glass filter and solids were collected. The solids were washed with methanol, acetone, and petroleum ether in this order. The washed was vacuum dried. 1.8 g of dark violet crystals were obtained.

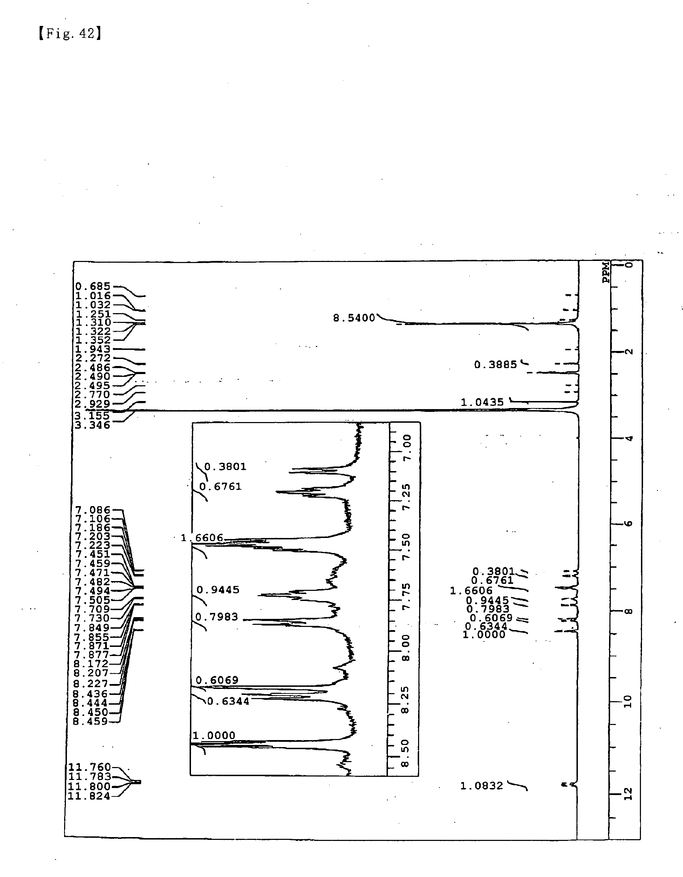

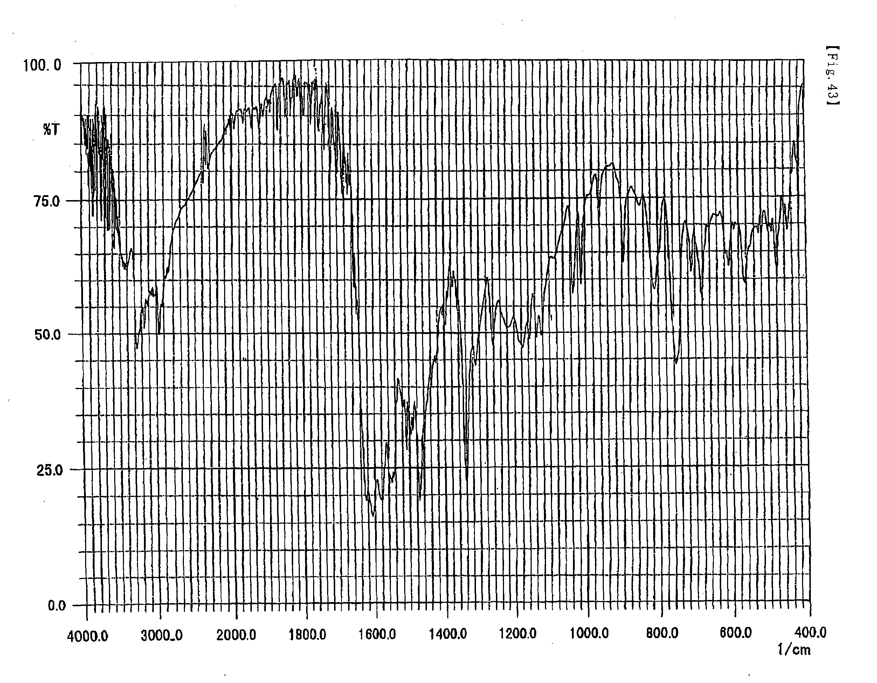

An NMR spectrum chart of the obtained crystals is shown in Figure 42 and an IR spectrum chart thereof in Figure 43.

These dark violet crystals were identified based on these data as the compound having the structure represented by formula (33).

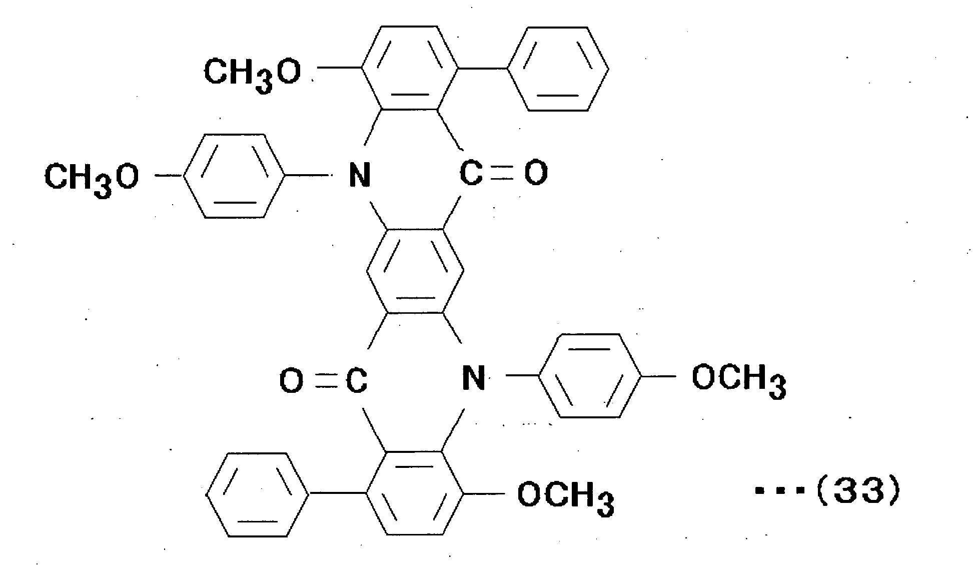

A sample solution was prepared by dissolving the obtained crystals in xylene so that the concentration of the target compound was 15 mg/L. This sample solution was loaded in a model F-4500 spectrofluorophotometer, a product by Hitachi, Ltd., and the fluorescence spectrum of the compound was measured under the same conditions as those of the samples in Example 1 were measured. The measured spectrum is shown in Figure 44.

As understood from Figure 44, the compound showed fluorescence at wavelengths of 400 nm to 600 nm, which confirmed that the crystals obtained in this example emit white light.

### (Example 6) Synthesis of a White Light-emitting Compound

### <Dehydration>

In a 1L three-necked flask were placed 25.0 g of 4-n-heptylaniline, 13.5 g of the diol represented by formula (13), 250 ml of acetic acid, and 250 ml of ethanol. The flask containing the mixture was placed in a silicone oil bath and the mixture was heated to 115°C. The mixture was stirred for 4 hours at around the temperature and allowed to react. After the termination of the reaction, the product was cooled naturally. The cooled product was filtered with a glass filter and solids were collected. The solids were washed with methanol and petroleum ether in this order. The washed was' vacuum dried. 24.0 g of yellow crystals were obtained.

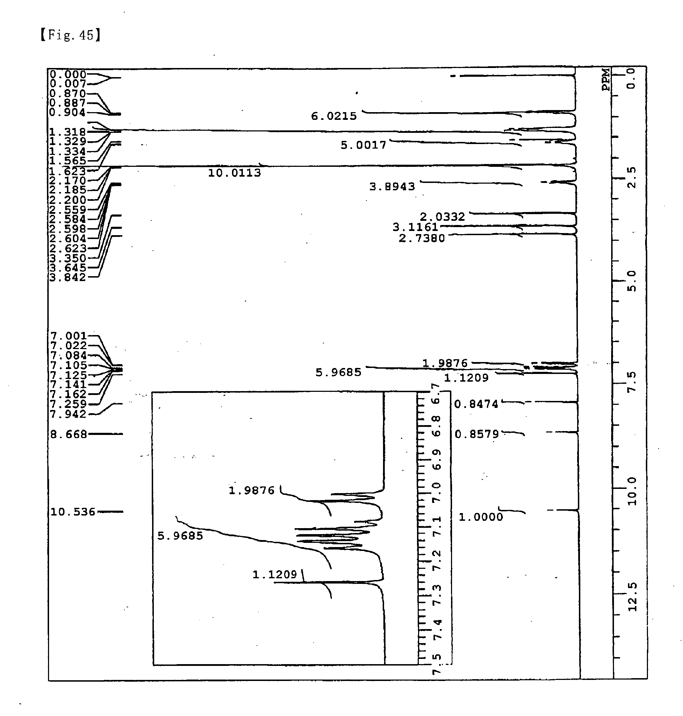

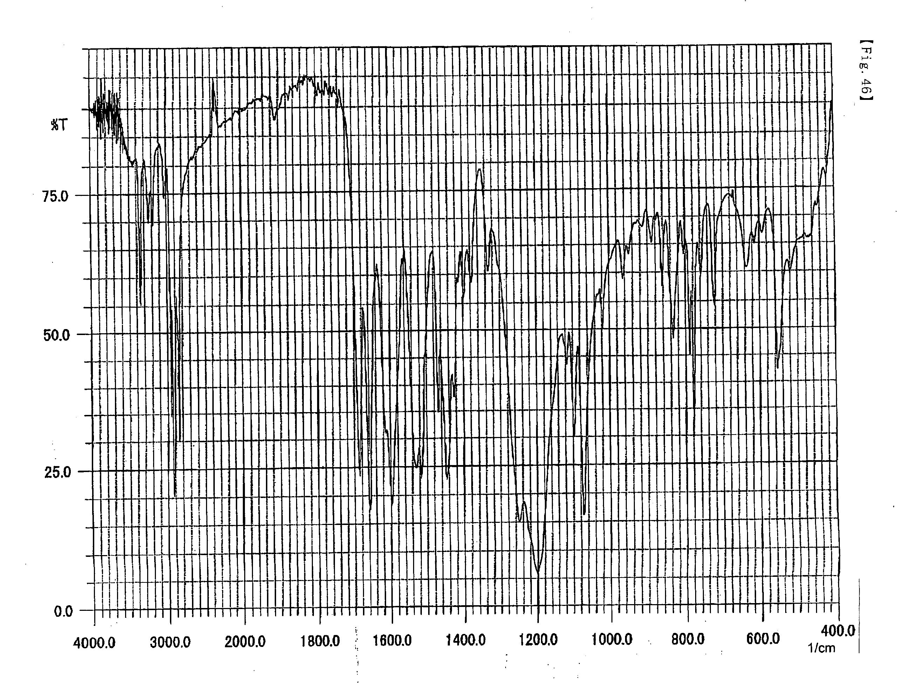

An NMR spectrum chart of the obtained crystals is shown in Figure 45 and an IR spectrum chart thereof in Figure 46.

These yellow crystals were identified based on these data as the compound having the structure represented by formula (34) .

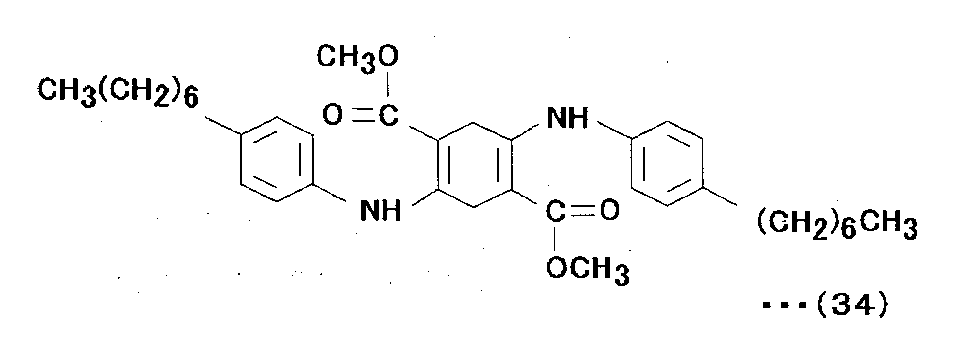

### <Dehydrogenation>

In a 1L three-necked flask were placed 20.0 g of the compound prepared in the step of dehydration and 500 ml of o-dichlorobenzene. A 95% aqueous solution of sulfuric acid was gradually added to the mixture in the flask over 30 minutes with stirring, while the mixture was kept at room temperature. The total amount of the added sulfuric acid solution was 0.3 g. The flask containing the resultant mixture was placed in a silicone oil bath and heated to 160 °C. The mixture was stirred for 2 hours at around the temperature and allowed to react. After the termination of the reaction, the product was cooled with ice. The cooled product was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried. 16.0 g of red crystals were obtained.

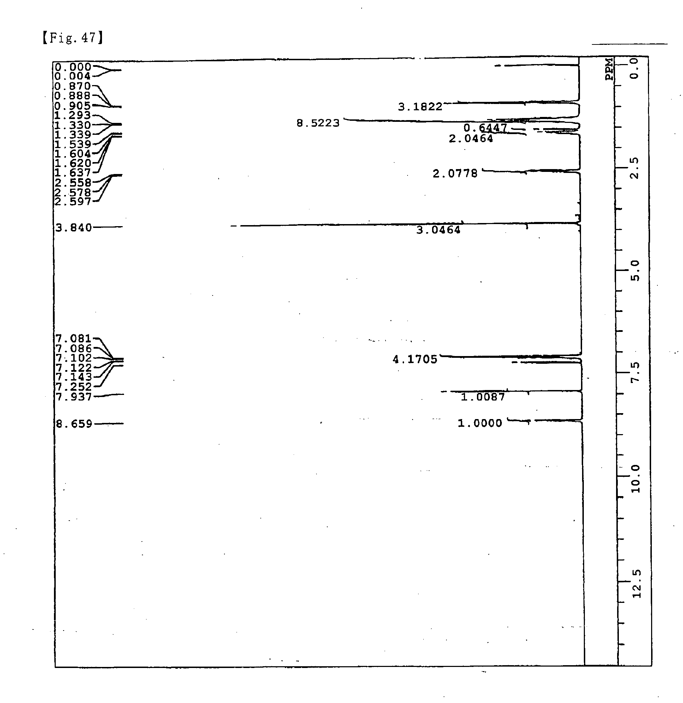

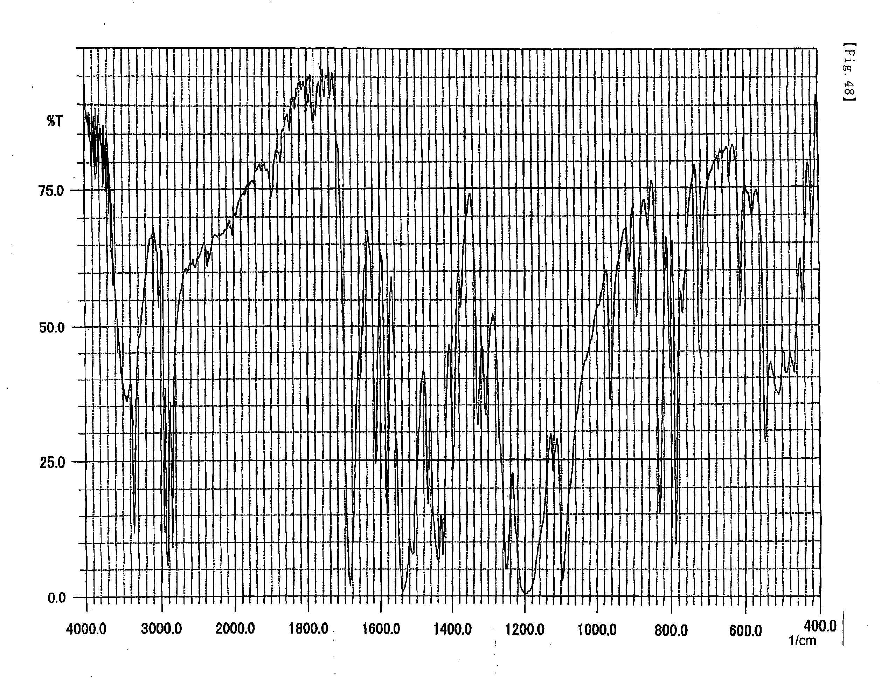

An NMR spectrum chart of the obtained crystals is shown in Figure 47 and an IR spectrum chart thereof in Figure 48.

These red crystals were identified based on the data as the compound having the structure represented by formula (35) .

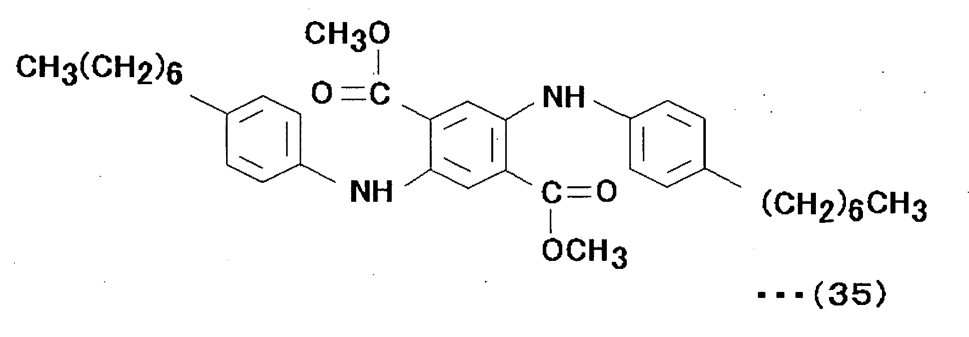

### <Ring Closure>

In a 500 ml three-necked flask were placed 10.0 g of the compound prepared in the step of dehydrogenation, 19.8 g of monohydrated p-toluenesulfonic acid, and 300 ml of o-dichlorobenzene. The flask containing the reaction mixture was placed in a silicone oil bath and the mixture was heated to 160°C. The mixture was stirred for 24 hours at around the temperature and allowed to react. After the termination of the reaction, the product was cooled naturally. The cooled product was concentrated using an evaporator. The concentrated was filtered with a glass filter and solids were collected. The solids were washed with methanol, acetone, and petroleum ether in this order. The washed was vacuum dried. 7.2 g of dark violet crystals were obtained.

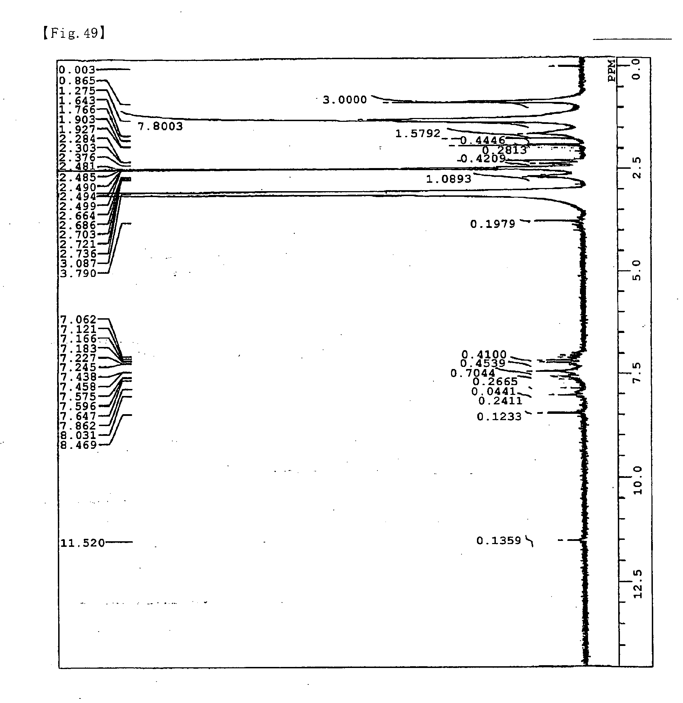

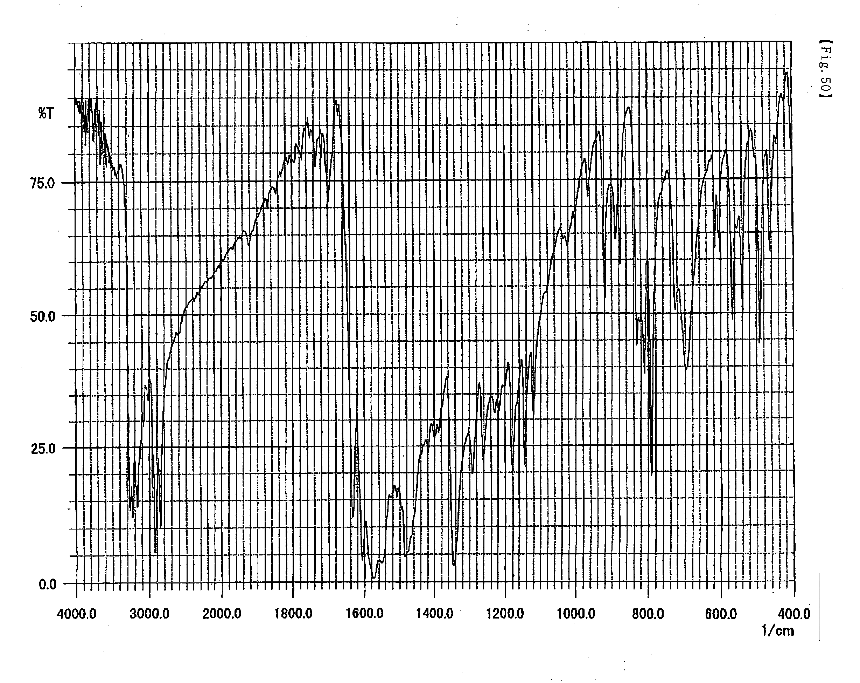

An NMR spectrum chart of the obtained crystals is shown in Figure 49 and an IR spectrum chart thereof in Figure 50.

These dark violet crystals were identified based on these data as the compound having the structure represented by formula (36).

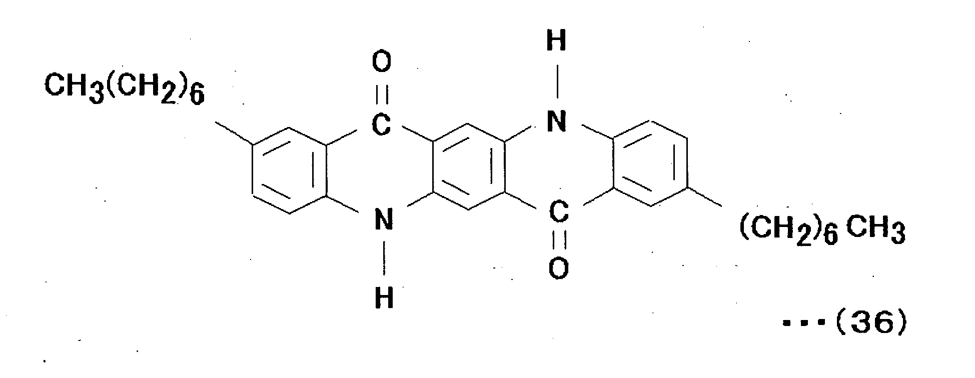

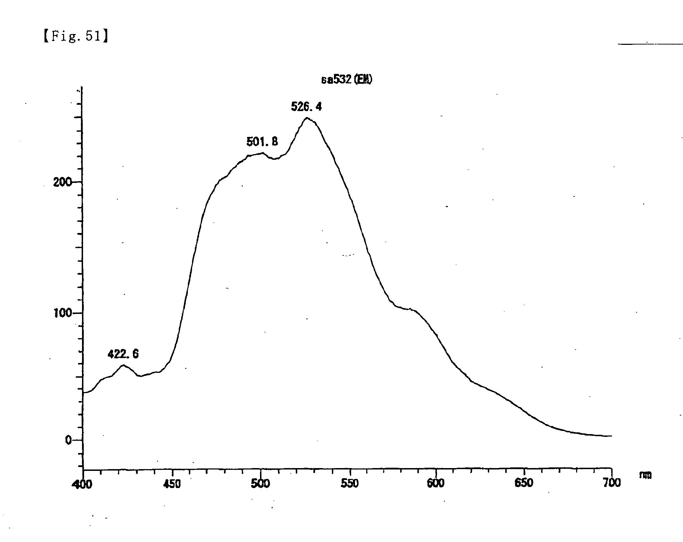

A sample solution was prepared by dissolving the obtained crystals in xylene so that the concentration of the target compound was 15 mg/L. This sample solution was loaded in a model F-4500 spectrofluorophotometer, a product by Hitachi, Ltd., and the fluorescence spectrum of the compound was measured under the same conditions as those of the samples in Example 1 were measured. The measured spectrum is shown in Figure 51.

As understood from Figure 51, the compound showed fluorescence at wavelengths of 450 nm to 570 nm, which confirmed that the crystals obtained in this example emit white light.

### (Example 7) Synthesis of a White Light-emitting Compound

### <Dehydration>

In a 1L three-necked flask were placed 25.0 g of 4-n-pentylaniline, 16. 5 g of the diol represented by formula (13), 200 ml of acetic acid, and 200 ml of.ethanol. The flask containing the mixture was placed in a silicone oil bath and the mixture was heated to 115°C. The mixture was stirred for 4 hours at around the temperature and allowed to react. After the termination of the reaction, the product was cooled naturally. The cooled product was filtered with a glass filter and solids were collected. The solids were washed with methanol and petroleum ether in this order. The washed was vacuum dried. 24.0 g of orange crystals were obtained.

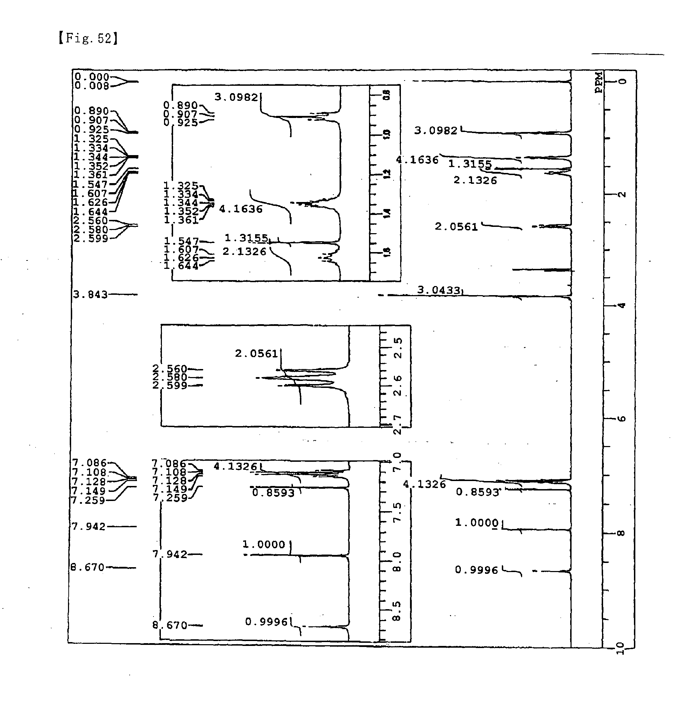

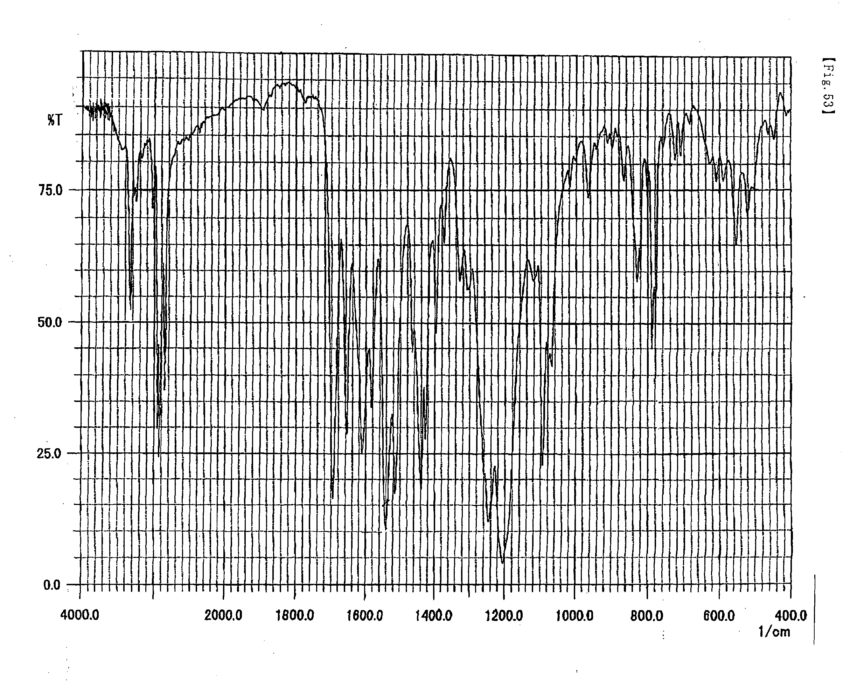

An NMR spectrum chart of the obtained crystals is shown in Figure 52 and an IR spectrum chart thereof in Figure 53.

These orange crystals were identified based on these data as the compound having the structure represented by formula (37).

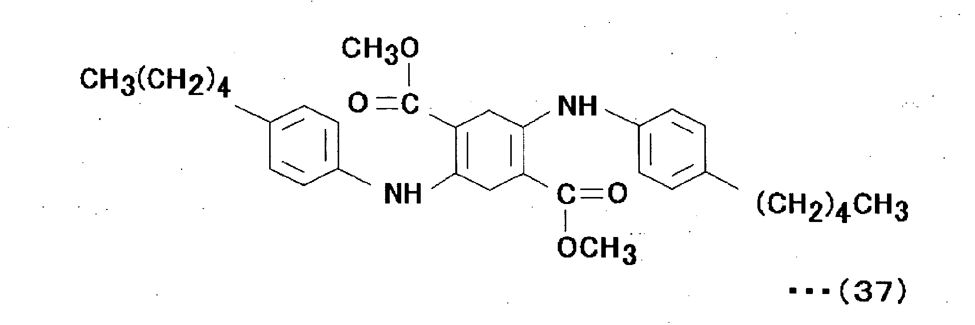

### <Dehydrogenation>

In a 1L three-necked flask were placed 20.0 g of the compound prepared in the step of dehydration and 500 ml of o-dichlorobenzene. A 95% aqueous solution of sulfuric acid was gradually added to the mixture in the flask over 30 minutes with stirring, while the mixture was kept at room temperature. The total amount of the added sulfuric acid solution was 0.2 g. The flask containing the resultant mixture was placed in a silicone oil bath and heated to 160°C. The mixture was stirred for 2 hours at around the temperature and allowed to react. After the termination of the reaction, the product was cooled with ice. The cooled product was subjected to extraction with chloroform. The chloroform solution was washed with water, and completely dried over anhydrous sodium sulfate. The dried solution with the sodium sulfate was filtered. Then, the filtrate was concentrated and dried up to solids. The solids were washed with methanol, and then with petroleum ether. The washed solids were vacuum dried. 14.7 g of red crystals were obtained.

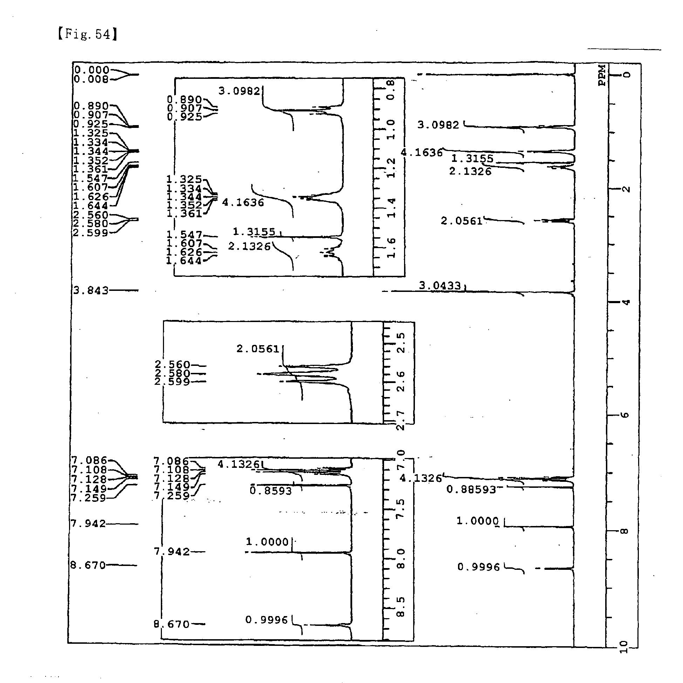

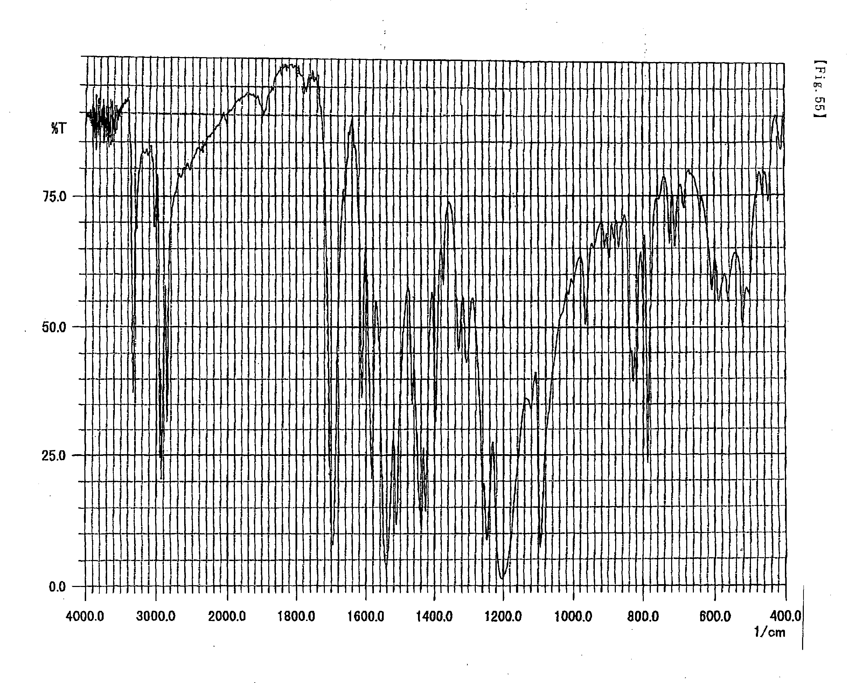

An NMR spectrum chart of the obtained crystals is shown in Figure 54 and an IR spectrum chart thereof in Figure 55.

These red crystals were identified based on the data as the compound having the structure represented by formula (38) .

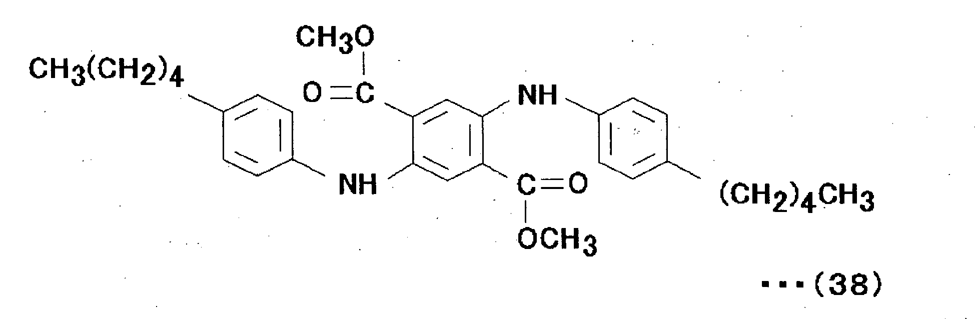

### <Ring Closure>

In a 500 ml three-necked flask were placed 5.0 g of the compound prepared in the step of dehydrogenation, 10.1 g of monohydrated p-toluenesulfonic acid, and 250 ml of o-dichlorobenzene. The flask containing the reaction mixture was placed in a silicone oil bath and the mixture was heated to 160°C. The mixture was stirred for 24 hours at around the temperature and allowed to react. After the termination of the reaction, the product was cooled naturally. The cooled product was concentrated using an evaporator. The concentrated was filtered with a glass filter and solids were collected. The solids were washed with methanol, acetone, And petroleum ether in this order. The washed was vacuum dried. 1.0 g of dark red crystals were obtained.

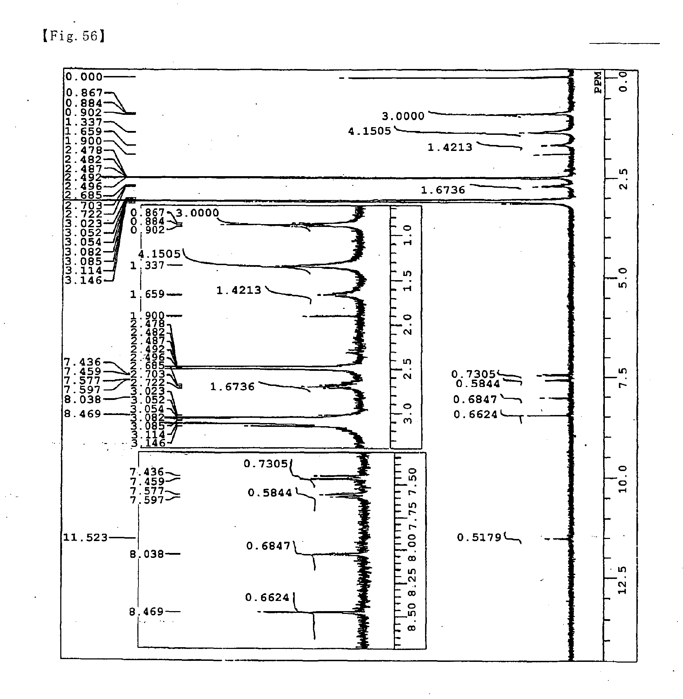

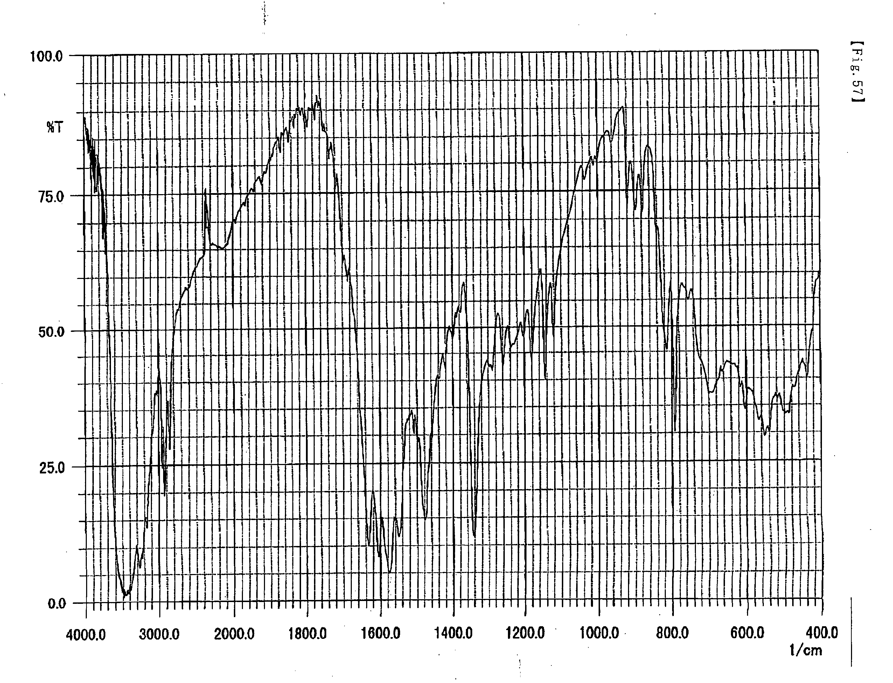

An NMR spectrum chart of the obtained crystals is shown in Figure 56 and an IR spectrum chart thereof in Figure 57.

These dark red crystals were identified based on these data as the compound having the structure represented by formula (39).

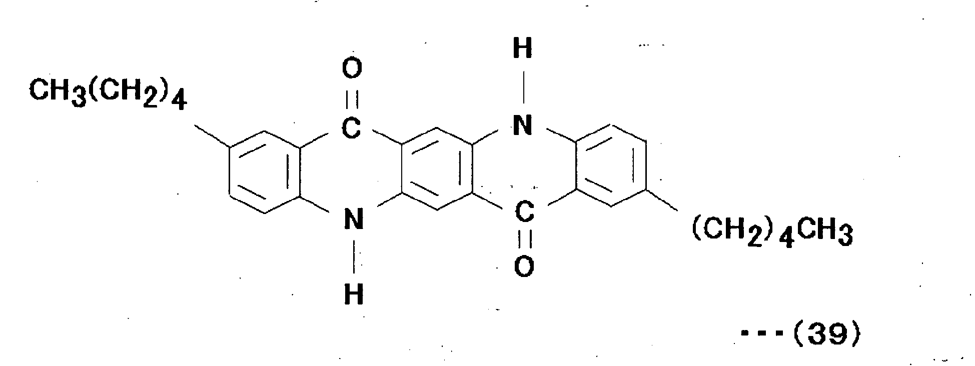

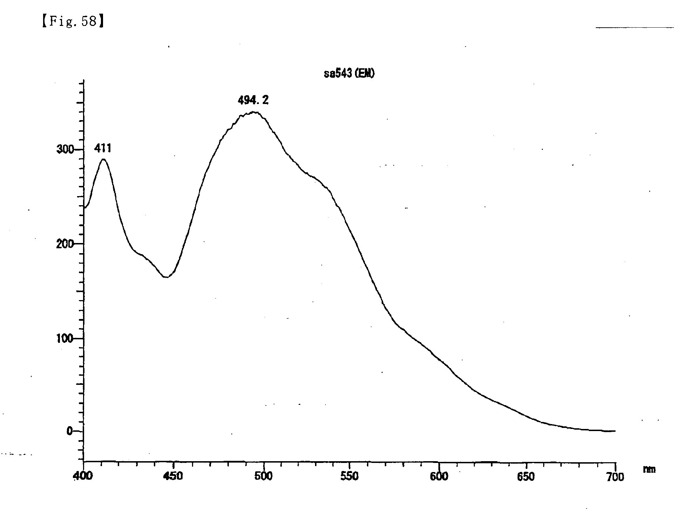

A sample solution was prepared by dissolving the obtained crystals in xylene so that the concentration of the target compound was 15 mg/L. This sample solution was loaded in a model F-4500 spectrofluorophotometer, a product by Hitachi, Ltd. , and the fluorescence spectrum of the compound was measured under the same conditions as those of the samples in Example 1 were measured. The measured spectrum is shown in Figure 58.

As understood from Figure 58, the compound showed fluorescence at wavelengths of 450 nm to 600 nm, which confirmed that the crystals obtained in this example emit white light.

### (Example 8) Synthesis of a White Light-emitting Compound

### <Ring Closure>

The steps of Example 5 were repeated, except that the step of alkylation was omitted. 0.7 g of dark red crystals were obtained.

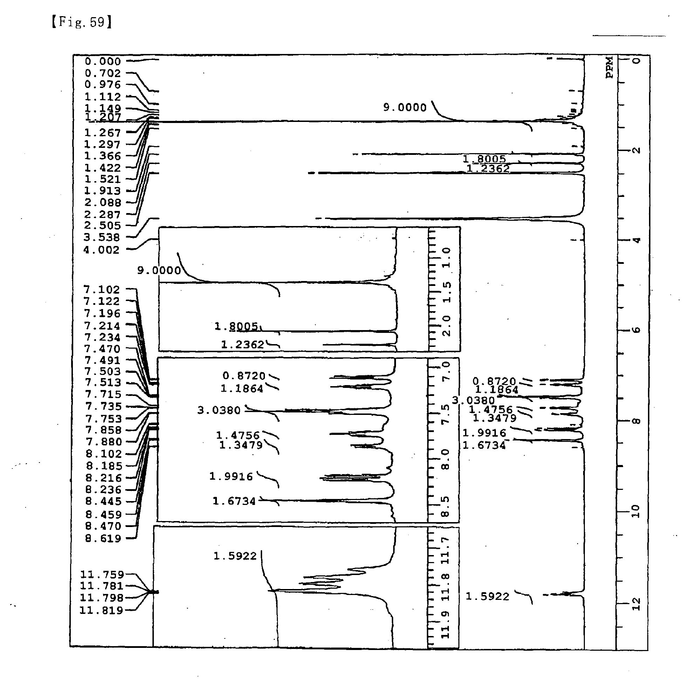

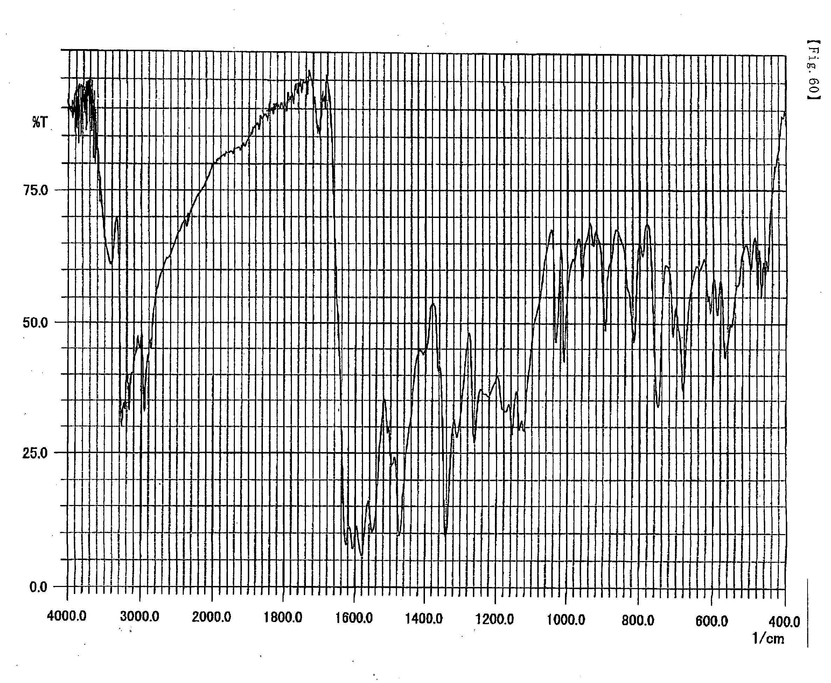

An NMR spectrum chart of the obtained crystals is shown in Figure 59 and an IR spectrum chart thereof in Figure 60.

These dark red crystals were identified based on these data as the compound having the structure represented by formula (40).

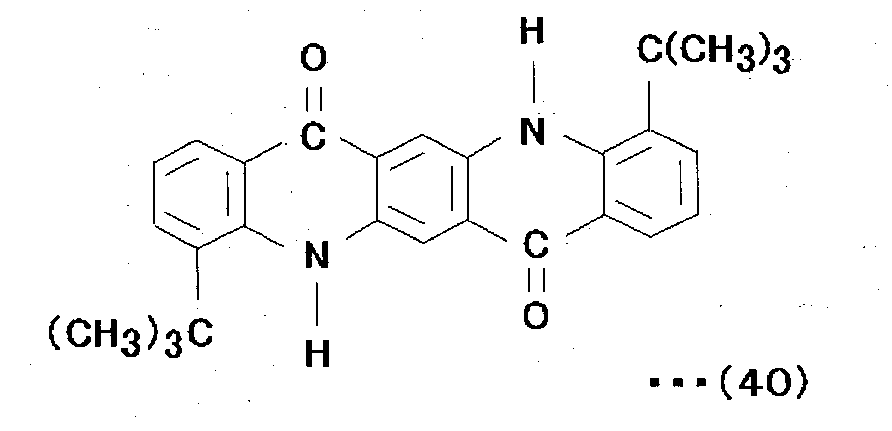

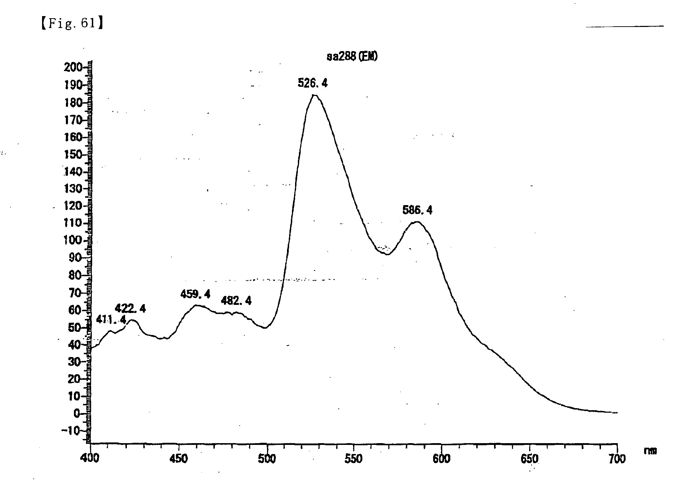

A sample solution was prepared by dissolving the obtained crystals in xylene so that the concentration of the target compound was 15 mg/L. This sample solution was loaded in a model F-4500 spectrofluorophotometer, a product by Hitachi, Ltd. , and the fluorescence spectrum of the compound was measured under the same conditions as those of the samples in Example 1 were measured. The measured spectrum is shown in Figure 61.

As understood from Figure 61, the compound showed fluorescence at wavelengths of 500 nm to 600 nm, which confirmed that the crystals obtained in this example emit white light.

### INDUSTRIAL APPLICABILITY

The present invention provides a white light-emitting compound capable of emitting white light by itself, a process of producing the compound, and an element capable of emitting white light.

## Claims

1. A white light-emitting compound represented by formula (1) : 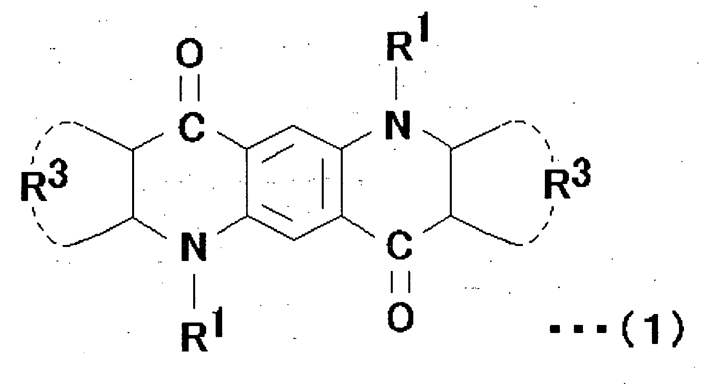 wherein R¹ is a hydrogen atom, an alkyl group with 1 to 10 carbon atoms, an aryl group represented by formula (2), or an aralkyl group represented by formula (3), wherein there are no cases where both R¹ s are hydrogen atoms; R³ denotes one of the substituents respectively represented by formulas (4)-(8), wherein two R³s may be the same or different from each other;
the formula (2) is: 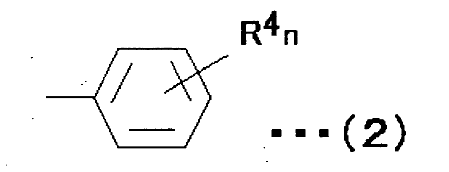 wherein R⁴ is a hydrogen atom, an alkyl group with 1 to 10 carbon atoms, or an alkoxyl group with 1 to 5 carbon atoms; and n denotes an integer from 1 to 5,
the formula (3) is: 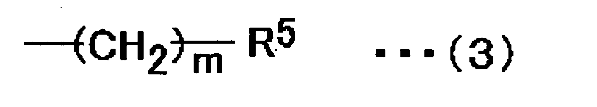 wherein R⁵ is an aryl group represented by the formula (2); and m denotes an integer from 1 to 10,
the formula (4) is: 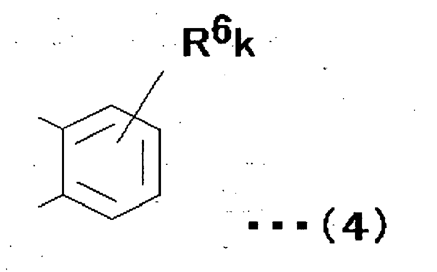 wherein R⁶ is a hydrogen atom, an alkyl group with 1 to 10 carbon atoms, an alkoxyl group with 1 to 5 carbon atoms, or an aryl group represented by the formula (2) ; and k denotes an integer from 1 to 4,
the formula 5 is: 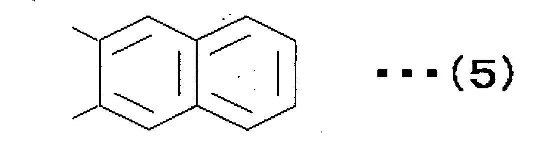 the formula (6) is: 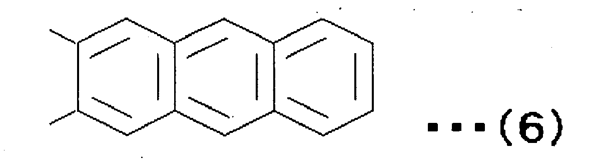 the formula (7) is: 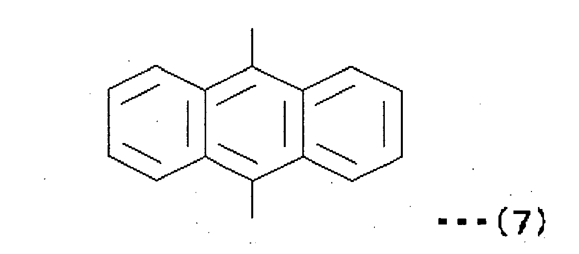 and, the formula (8) is:

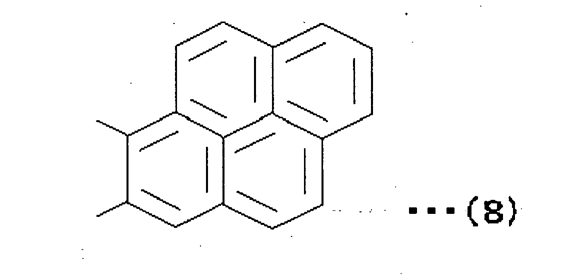

2. A process of producing a white light-emitting compound represented by the formula (1), comprising dehydrating an aromatic amine represented by formula (9) and a diol represented by formula (10) to produce a first compound represented by formula (11) ; dehydrogenating the first compound; reacting the dehydrogenated compound with an alkyl halide, the chemical formula of which is R¹-X wherein R¹ denotes the same as that defined in claim 1, and X is a halogen atom, to produce a second compound represented by formula (12) ; and subj ecting the second compound to a ring-closing reaction, wherein
the formula (9) is:
R³ ―NH₂ (9)
wherein R³ denotes the same as that defined in claim 1,
the formula (10) is: 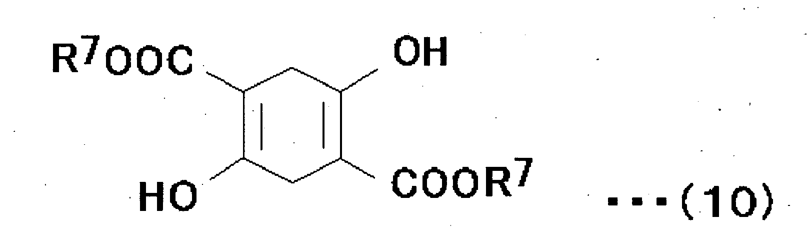 wherein two R⁷s may be the same or different from each other,
the formula (11) is: 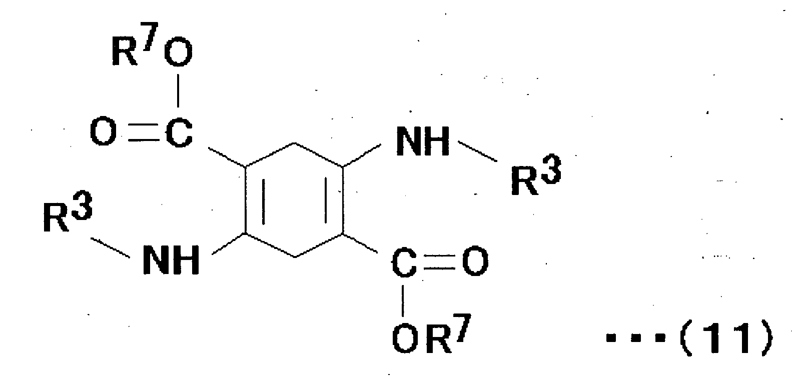 wherein R³ denotes the same as that defined in claim 1 and R⁷ denotes the same as that defined above,
the formula (12) is: 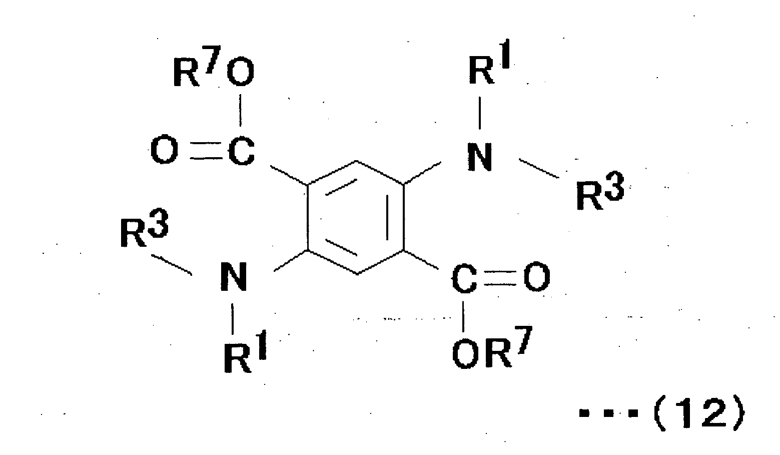 wherein R¹ denotes the same as that defined in claim 1 and there are no cases where both R¹ s are hydrogen atoms, and R³ and R⁷ are the same as those defined above.

3. A white light-emitting element having a pair of electrodes and a light-emitting layer sandwiched between the electrodes, the light-emitting layer including a white light-emitting compound represented by the formula (1) shown in claim 1.
